(19) Europäisches Patentamt
European Patent Office
Office européen des brevets

(11) EP 1 736 546 A1

(12) **EUROPEAN PATENT APPLICATION**
published in accordance with Art. 158(3) EPC

(43) Date of publication:
**27.12.2006 Bulletin 2006/52**

(21) Application number: **05721307.6**

(22) Date of filing: **23.03.2005**

(51) Int Cl.:
***C12N 15/65*** *(2006.01)* ***C12N 15/55*** *(2006.01)*
***A01K 67/027*** *(2006.01)* ***C12N 5/10*** *(2006.01)*
***C12Q 1/42*** *(2006.01)* ***G01N 33/15*** *(2006.01)*
***G01N 33/50*** *(2006.01)* ***G01N 33/68*** *(2006.01)*

(86) International application number:
**PCT/JP2005/005257**

(87) International publication number:
**WO 2005/090580 (29.09.2005 Gazette 2005/39)**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(30) Priority: **23.03.2004 JP 2004084810**

(71) Applicant: **Eisai R&D Management Co., Ltd.**
**Tokyo 112-0002 (JP)**

(72) Inventors:
- **OKAMOTO, Kiyoshi,**
**c/o EISAI CO., LTD.**
**Tsukuba-shi,**
**Ibaraki 3002635 (JP)**
- **KAWAMURA, Takanori,**
**c/o EISAI CO., LTD.**
**Tsukuba-shi,**
**Ibaraki 3002635 (JP)**
- **ASANO, Makoto,**
**c/o EISAI CO., LTD.**
**Tsukuba-shi,**
**Ibaraki 3002635 (JP)**
- **SHITAKUBO, Daiya,**
**c/o EISAI R&D Management Co.,Ltd**
**Tokyo 171-0033 (JP)**
- **SHIRATO, Manabu,**
**c/o EISAI CO., LTD.**
**Tsukuba-shi,**
**Ibaraki 3002635 (JP)**
- **ASADA, Makoto,**
**c/o EISAI CO., LTD.**
**Tsukuba-shi,**
**Ibaraki 3002635 (JP)**

(74) Representative: **HOFFMANN EITLE**
**Patent- und Rechtsanwälte**
**Arabellastrasse 4**
**81925 München (DE)**

(54) **USING NONHUMAN ANIMAL MODEL, METHOD OF MEASURING TRANSCRIPTION ACTIVITY, METHOD OF MEASURING CELL QUANTITY AND METHOD OF MEASURING TUMOR VOLUME**

(57) In a nonhuman animal model that produces a secretory protein, which is obtained by transplanting secretory protein-expression vector-transfected cells into a nonhuman animal, an amount of the secretory protein is measured and, based on the amount of the secretory protein, transcriptional activity, number of the transplanted cells, and tumor volume is measured. Further, screening of a compound that affects a transcriptional activity, number of transplanted cells, or tumor volume is performed by using the nonhuman animal model to which a compound has been administered.

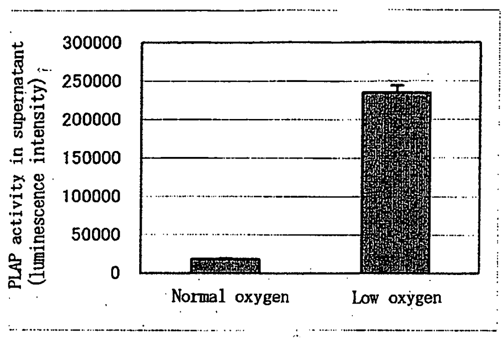


Fig. 3

EP 1 736 546 A1

## Description

TECHNICAL FIELD

The present invention relates to a method of measuring transcriptional activity in transplanted cells, a method of measuring the number of the transplanted cells, and. a method of measuring tumor volume in a nonhuman animal model.

BACKGROUND ART

It has been known that regulating transcription to regulate expression amount of a protein plays an important role in expression of function of the protein. Attempts have been made to develop therapeutic agents for treating various diseases by controlling transcriptional regulation. For developing such drugs, methods of properly evaluating transcriptional activity are indispensable. There have been many reports on methods of measuring transcriptional activity in cultured cells (in vitro). However, few methods that can properly measure transcriptional activity in nonhuman animal models (in vivo) have been known.
Further, it plays an important role, particularly in the development of anti-tumor agents, to measure number of cultured cells transplanted into a nonhuman animal model. However, few methods that enable non-invasive and simple measurement of cell number have been known.
Conventionally, as a method of measuring transcriptional activity in a nonhuman animal model, a method that involves analysis of mRNA expression by Northern blotting (Non-patent Document 1) and a method that involves use of β-Gal gene as a reporter gene (Non-patent Document 2) have been used. However, these methods have a problem that they have an extremely low processing efficiency since they each involve cumbersome operations such as extraction of mRNA, preparation of tissue sections and the like. In addition, since tissue has to be extracted before measuring transcriptional activity, it is difficult to examine time-dependent change.
Further, in recent years, a method that involves use of a luciferase gene as a reporter gene is reported (Non-patent Document 3). In this method, however, in order to measure transcriptional activity, it is necessary to transplant into a mouse cultured cells in which the luciferase gene has been transfected, anesthetize the mouse, intravenously inject to the mouse a substrate for the luciferase, photographing luminescence due to the luciferase activity in a dark room, and analyze the obtained image.
For this reason, this method has problems that it requires a special apparatus, and involves the above-mentioned cumbersome operations, and it is influenced by anesthesia and lacks quantitative accuracy.
On the other hand, as a method of measuring the number of cultured cells that have been transplanted into a nonhuman animal model, a method that involves transplanting cells that have been stained with a dye in advance has been used (Non-patent Document 4). However, this method has a problem that it has an extremely low processing efficiency since it involves a cumbersome operation such as extraction of a tissue. In addition, the necessity of extracting the tissue makes it difficult to examine time-dependent change. Further, since the dye decreases after every cell division of the cultured cells, the method has a problem of sensitivity of measurement.
Further, as a method of measuring the number of cultured cells that have been transplanted into a nonhuman animal model, a method that involves use of a GFP gene as a reporter gene is reported by AntiCancer, Inc. (Non-patent Document 5). According to this method, cell number is measured by introducing a plasmid which has a constitutive transcription regulatory sequence ligated upstream of the GFP gene into tumor cells, transplanting the tumor cells into a mouse, photographing an organ of the mouse by means of a fluorescence microscope, and analyzing the obtained image. However, in this method, it is necessary to subject the mouse to laparotomy or craniotomy before the photographing by a fluorescence microscope in order to measure number of the tumor cells transplanted into the internal organ or brain of the mouse. Therefore, the method is invasive and cumbersome and has problems of influence of anesthesia and of quantitative accuracy.

[Non-Patent Document 1] Molecular Cloning, A Laboratory Manual 3rd ed., Cold Spring Harbor Press (2001) Section 7.42)
[Non-Patent Document 2] Kucharczuk, et. al. (1999) Development 126(9): 1957-1965
[Non-Patent Document 3] Shoemaker. The 7th International Symposium on Cancer Chemotherapy, Tokyo, 2002
[Non-Patent Document 4] Yamagata, et. al. (2000) Journal of experimental & clinical cancer research, 19(2): 211-217
[Non-Patent Document 5] Yang, et. al. (1999) Cancer Research 59(4): 781-786

DISCLOSURE OF THE INVENTION

Under such circumstances, the present invention has been made, and it is an object of the present invention to establish a method of non-invasively, simply, and accurately measuring transcriptional activity through a transcription regulatory sequence in transplanted cells and a method of non-invasively, simply, and accurately screening a compound that affects transcriptional activity of the transcription regulatory sequence in a nonhuman animal model, a method of non-invasively, simply, and accurately measuring the number of transplanted cells, a method of non-invasively, simply, and accurately screening a compound that affects the number of the transplanted cells in a non-human animal model, a method of non-invasively, simply, and accurately measuring tumor volume, and a method of non-invasively, simply, and accurately screening a compound that affects tumor volume in a non-human animal model.

The inventors of the present invention have made extensive studies with a view to achieve the above-mentioned objects. As a result, they have found that transcriptional activity through a transcription regulatory sequence can be measured by preparing a placenta-derived alkaline phosphatase (hereinafter, also referred to as "PLAP") reporter plasmid obtained by inserting the transcription regulatory sequence to upstream of a PLAP gene that has been modified into a secretion type, transplanting cultured cells into which the PLAP reporter plasmid has been transfected into a nonhuman animal, and measuring alkaline phosphatase activity in blood of the non-human animal model.

Furthermore, the inventors of the present invention have found that a compound that affects a transcriptional activity through a transcription regulatory sequence in a non-human animal model can be screened non-invasively, simply, and accurately by administering the compound to a nonhuman animal model that have been transplanted with cultured cells into which the PLAP reporter plasmid has been transfected, and measuring alkaline phosphatase activity in blood of the non-human animal model.

Furthermore, the inventors of the present invention have found that number of transplanted cells and tumor volume in a nonhuman animal model can be measured and that a compound that affects the number of transplanted cells and tumor volume can be screened non-invasively, simply, and accurately by preparing a PLAP reporter plasmid using a constitutive transcription regulatory sequence as a transcription regulatory sequence, transplanting cultured cells that have been transfected with the PLAP reporter plasmid into a non-human animal model, and measuring alkaline phosphatase activity in blood of the obtained non-human animal model.
Thus, the present invention has been accomplished.

That is, the present invention relates to the followings.

(1) A method of measuring transcriptional activity in cells transplanted into a nonhuman animal model, comprising:

measuring an amount of a secretory protein in a nonhuman animal model that produces the secretory protein, the nonhuman animal model being obtained by transplanting cells that have been transfected therein an expression vector comprising a transcription regulatory sequence and a polynucleotide coding for the secretory protein operably linked to the transcription regulatory sequence, into a nonhuman animal; and
measuring transcriptional activity through the transcription regulatory sequence based on the amount of the secretory protein.

(2) The method according to (1), wherein the transcription regulatory sequence comprises a transcription regulatory factor-binding sequence.
(3) The method according to (2), wherein the transcription regulatory factor-binding sequence is at least one sequence selected from the group consisting of SEQ ID No: 1, SEQ ID No: 2, SEQ ID No: 3, SEQ ID No: 4, SEQ ID No: 5, SEQ ID No: 6, SEQ ID No: 7, and SEQ ID No: 8.
(4) The method according to any one of (1) to (3), wherein the secretory protein is a secretory enzyme.
(5) The method according to (4), wherein the secretory enzyme is a secretory alkaline phosphatase.
(6) The method according to (5), wherein the secretory alkaline phosphatase is a heat-resistant secretory alkaline phosphatase.
(7) The method according to (5), wherein the secretory alkaline phosphatase is a secretory placenta-derived alkaline phosphatase.
(8) The method according to (7), wherein the secretory placenta-derived alkaline phosphatase is a protein consisting of an amino sequence of SEQ ID No: 11.
(9) The method according to any one of (1) to (8), wherein an amount of the secretory protein in blood is measured.
(10) The method according to (9), wherein the amount of the secretory protein in blood is measured by measuring an enzymatic activity.
(11) The method according to (10), wherein the enzymatic activity is alkaline phosphatase activity.
(12) The method according to any one of (1) to (11), wherein the cells are tumor cells or immortalized cells.
(13) A method of screening a compound that affects transcriptional activity, comprising the steps of:

(a) administering a compound to a nonhuman animal model that produces a secretory protein, the nonhuman animal model being obtained by transplanting cells that have been transfected therein an expression vector comprising a polynucleotide coding for the secretory protein; into a nonhuman animal; and
(b) measuring transcriptional activity in the transplanted cells in the nonhuman animal model administered with the compound, by the method according to any one of (1) to (12).

(14) A method of screening a compound that affects transcriptional activity, comprising the steps of:

(a) transplanting cells that have been transfected therein an expression vector comprising a polynucleotide coding for a secretory protein, into a nonhuman animal administered with a compound; and
(b) measuring transcriptional activity in the transplanted cells in the nonhuman animal model, by the method according to any one of (1) to (12).

(15) A method of measuring the number of transplanted cells in a nonhuman animal model, comprising:

measuring an amount of a secretory protein in a nonhuman animal model that produces the secretory protein, the nonhuman animal model being obtained by transplanting cells that have been transfected therein an expression vector comprising a transcription regulatory sequence and a polynucleotide coding for a secretory protein operably linked to the transcription regulatory sequence, into a nonhuman animal; and
measuring the number of the transplanted cells based on the amount of the secretory protein.

(16) The method according to (15), wherein the secretory protein is a secretory enzyme.
(17) The method according to (16), wherein the secretory enzyme is a secretory alkaline phosphatase.
(18) The method according to (17), wherein the secretory alkaline phosphatase is a heat-resistant secretory alkaline phosphatase.
(19) The method according to (17), wherein the secretory alkaline phosphatase is a secretory placenta-derived alkaline phosphatase.
(20) The method according to (19), wherein the secretory placenta-derived alkaline phosphatase is a protein consisting of an amino sequence represented by SEQ ID No: 11.
(21) The method according to any one of (15) to (20), wherein an amount of the secretory protein in blood is measured.
(22) The method according to (21), wherein the amount of the secretory protein in blood is measured by measuring an enzymatic activity.
(23) The method according to (22), wherein the enzymatic activity is alkaline phosphatase activity.
(24) The method according to any one of (15) to (23), wherein the cells are tumor cells or immortalized cells.
(25) The method according to any one of (15) to (24), wherein the transcription regulatory sequence comprises a constitutive transcription regulatory sequence.
(26) The method according to (25), wherein the constitutive transcription regulatory sequence is at least one sequence selected from the group consisting of SV40 promoter, CMV promoter, thymidine kinase promoter, ubiquitin C promoter, elongation factor 1 alpha (EF1a) promoter, β-actin promoter, glyceraldehyde-3-phosphate dehydrogenase promoter, phosphoglycerokinase promoter, β2-microglobulin promoter, and β-glucuronidase promoter.
(27) The method according to (25), wherein the constitutive transcription regulatory sequence is SV40 promoter.
(28) The method according to (25), wherein the constitutive transcription regulatory sequence is a sequence represented by SEQ ID No: 9.
(29) A method of screening a compound that affects transcriptional activity, comprising the steps of:

(a) administering a compound to a nonhuman animal model that produces a secretory protein, the nonhuman animal model being obtained by transplanting cells that have been transfected therein an expression vector comprising a polynucleotide coding for the secretory protein, into a nonhuman animal; and
(b) measuring the number of the transplanted cells in the nonhuman animal model administered with the compound, by the method according to any one of (15) to (28).

(30) A method of screening a compound that affects the number of transplanted cells, comprising the steps of:

(a) transplanting cells that have been transfected therein an expression vector comprising a polynucleotide coding for a secretory protein, into a nonhuman animal administered with a compound; and
(b) measuring the number of the transplanted cells in the nonhuman animal by the method according to any one of (15) to (28).

(31) A method of measuring tumor volume in a nonhuman animal model, comprising:

measuring an amount of a secretory protein in a nonhuman animal model that develops a tumor and produces the secretory protein in the tumor, the nonhuman animal model being obtained by transplanting cells that have been transfected therein an expression vector comprising a transcription regulatory sequence and a polynucleotide coding for a secretory protein operably linked to the transcription regulatory sequence, into a nonhuman animal; and
measuring tumor volume based on the amount of the secretory protein.

(32) The method according to (31), wherein the secretory protein is a secretory enzyme.
(33) The method according to (32), wherein the secretory enzyme is a secretory alkaline phosphatase.
(34) The method according to (33), wherein the secretory alkaline phosphatase is a heat-resistant secretory alkaline phosphatase.
(35) The method according to (33), wherein the secretory alkaline phosphatase is a secretory placenta-derived alkaline phosphatase.
(36) The method according to (35), wherein the secretory placenta-derived alkaline phosphatase is a protein consisting of an amino sequence represented by SEQ ID No: 11.
(37) The method according to any one of (31) to (36), wherein an amount of the secretory protein in blood is measured.
(38) The method according to (37), wherein the amount of the secretory protein in blood is measured by measuring an enzymatic activity.
(39) The method according to (38), wherein the enzymatic activity is alkaline phosphatase activity.
(40) The method according to any one of (31) to (39), wherein the cell is a tumor cell or an immortalized cell.
(41) The method according to any one of (31) to (40), wherein the transcription regulatory sequence comprises a constitutive transcription regulatory sequence.
(42) The method according to (41), wherein the constitutive transcription regulatory sequence is at least one sequence selected from the group consisting of SV40 promoter, CMV promoter, thymidine kinase promoter, ubiquitin C promoter, elongation factor 1 alpha (EF1a) promoter, β-actin promoter, glyceraldehyde-3-phosphate dehydrogenase promoter, phosphoglycerokinase promoter, β2-microglobulin promoter, and β-glucuronidase promoter.
(43) The method according to (41), wherein the constitutive transcription regulatory sequence is an SV40 promoter.
(44) The method according to (41), wherein the constitutive transcription regulatory sequence is a sequence represented by SEQ ID No: 9.
(45) A method of screening a compound that affects tumor volume, comprising the steps of:

(a) administering a compound to a nonhuman animal model that develops a tumor and produces a secretory protein in the tumor, the nonhuman animal model being obtained by transplanting cells that have been transfected therein an expression vector comprising a polynucleotide coding for the secretory protein, into a nonhuman animal; and
(b) measuring tumor volume in the nonhuman animal model administered with the compound, by the method according to any one of (31) to (44).

(46) An expression vector comprising a polynucleotide coding for a secretory protein, for use in the method according to any one of (1) to (45).
(47) A cell that has been transfected therein an expression vector comprising a polynucleotide coding for a secretory protein, for use in the method according to any one of (1) to (45).
(48) A nonhuman animal that produces a secretory protein, obtained by transplanting cells that have been transfected therein an expression vector that comprises a polynucleotide coding for a secretory protein, into a nonhuman animal, for use in the method according to any one of (1) to (45).
(49) A measuring kit, comprising an expression vector that comprises a polynucleotide coding for a secretory protein, for use in the method according to any one of (1) to (45).
(50) A measuring kit, comprising cells that have been transfected therein an expression vector that comprises a polynucleotide coding for a secretory protein, for use in the method according to any one of (1) to (45).
(51) A measuring kit, comprising a nonhuman animal that produces a secretory protein, obtained by transplanting cells that have been transfected therein an expression vector that contains a polynucleotide coding for the secretory protein, into a nonhuman animal, for use in the method according to any one of (1) to (45).

BRIEF DESCRIPTION OF THE DRAWINGS

Fig. 1 shows the structure of PLAP basic vector plasmid.

Fig. 2 shows the structure of HRE-PLAP reporter plasmid.

Fig. 3 shows results of analysis on PLAP activity of the cells that have been transfected with HRE-PLAP reporter plasmid.

Fig. 4 shows results of experiments where cells that had been transfected with HRE-PLAP reporter plasmid were subcutaneously transplanted into a nude mouse. Filled circles represent PLAP activity in blood and white triangles represent tumor volume. The horizontal axis indicates days after transplantation.

Fig. 5 shows the structure of dsRNA expression vector plasmid.

Fig. 6 shows the structure of HIF-1α dsRNA expression vector plasmid.

Fig. 7 shows results of analysis on PLAP activity of cells in which HIF-1α dsRNA expression vector plasmid was stably transfected. Ratio of induction of PLAP production by low oxygen was calculated by dividing the PLAP activity value in a medium with low oxygen concentration (2%) by the PLAP activity value in a medium with normal oxygen concentration (21%).

Fig. 8 shows results of analysis on the HIF-1α mRNA amount in cells in which HIF-1α dsRNA expression vector plasmid was stably transfected.

Fig. 9 shows results of experiments where the cell clone 2D4 in which HIF-1α dsRNA expression vector plasmid had been stably transfected and the control clone MD2 were subcutaneously transplanted into a nude mouse. Circles show PLAP activity in blood, triangles show tumor volume, and black symbols represent the clone MD2 and white symbols represent the clone 2D4. The horizontal axis indicates days after the transplantation.

Fig. 10 shows results of experiments where an anti-VEGF antibody was administered to a nude mouse into which HRE-PLAP reporter plasmid-transfected cells had been subcutaneously transplanted. Circles show PLAP activity in blood and triangles show tumor volume, and black symbols represent an anti-VEGF antibody-administered group and white symbols represent a control group. The horizontal axis indicates days after initiation of the therapy.

Fig. 11 shows results of experiments where HRE-PLAP reporter plasmid-transfected cells were intraperitoneally transplanted into a nude mouse. Each series shows PLAP activity in blood of each individual. The horizontal axis indicates days after the transplantation.

Fig. 12 shows the structure of VEGF-PLAP reporter plasmid.

Fig. 13 shows results of experiments where VEGF-PLAP reporter plasmid-transfected cells were intracranially transplanted into a nude mouse. Each series shows PLAP activity in blood of each individual. The horizontal axis indicates days after the transplantation.

Fig. 14 shows the structure of pCREBP2x2-tkPLAP vector.

Fig. 15 shows the structure of pCRBP2x2-TK promoter-PLAP basic vector.

Fig. 16 shows the structure of STAT6-PLAP reporter plasmid.

Fig. 17 shows the structure of STAT6 expression vector plasmid.

Fig. 18 shows results of analysis of PLAP activity of cells which stably express STAT6-PLAP reporter plasmid and STAT6 expression vector plasmid, without human IL4-stimulation or 24 hours after human IL4-stimulation.

Fig. 19 shows results of analysis of PLAP activity in blood of a mouse into which cells stably expressing STAT6-PLAP reporter plasmid and STAT6 expression vector plasmid had been transplanted into air sacs in the dorsal region in the cases of non-stimulation with human IL4 and 24 hours after stimulation with human IL4.

Fig. 20 shows results of analysis of PLAP activity in blood of a mouse into which cells stably expressing STAT6-PLAP reporter plasmid and STAT6 expression vector plasmid had been transplanted into air sacs in the dorsal region in a test compound-administered group and a control group 24 hours after stimulation with human IL4.

Fig. 21 shows the structure of SV40-PLAP reporter plasmid.

Fig. 22 shows results of experiments where SV40-PLAP reporter plasmid-transfected cells were subcutaneously transplanted into a nude mouse. The filled circles show PLAP activity in blood and white triangles show a tumor volume. The horizontal axis indicates days after the transplantation.

DESCRIPTION OF THE PREFERRED EMBODIMENTS

Hereinafter, embodiments of the present invention are explained. The following embodiments are for exemplary purpose for explaining the present invention, and the present invention should not be limited thereto. The present invention may be practiced in various embodiments as long as they do not depart from the gist of the present invention. The literatures, published patent applications, published patents, and other patent documents as mentioned herein are incorporated herein by reference.

1. Secretory protein

In the present invention, secretory protein refers to a protein which can be secreted out of cells and it is not particularly limited so long as it is a protein secreted out of cells. Examples of a secretory protein include peptidic hormones, cytokinin, albumin (for example, serum albumin), globulin (for example, immunoglobulin), enzymes, and other secretory proteins.

Examples of a peptide hormone include natriuretic peptide, hypothalamic hormone (for example, opioid peptide, thyroid stimulating hormone-releasing hormone, luteinizing hormone-releasing hormone, gonadotropic hormone, somatostatin, adrenocorticotropic hormone-releasing hormone, growth hormone-releasing factor, chromatophorotropic hormone-releasing factor, chromatophorotropic hormone release-inhibitory factor, prolactin-releasing factor, prolactin release-inhibitory factor, and head activator, etc.), pituitary gland hormone (for example, adrenocorticotropic hormone-stimulating hormone, growth hormone, prolactin, thyroid-stimulating hormone, luteinizing hormone, follicle-stimulating hormone, chromatophorotropic hormone, oxytocin, and vasopressin, etc.), thyroid hormones (for example, carcitonin), parathyroid hormone, pancreatic hormone (for example, insulin, glucagon, and pancreatic polypeptide, etc.), gastrointestinal hormone (secretin, vasoactive intestinal polypeptide, gastric inhibitory polypeptide, gastrin, gastrin-releasing peptide, cholecystokinin, motilin, cerulein, urogastrone, etc.), salivary gland hormone, placental hormone (for example, chorionic gonadotrophic hormone and placental lactogen, etc.), ovarian hormone, and growth factor (for example, epithelial cell growth factor, fibroblast growth factor, platelet-derived growth factor, erythropoietin, vascular endothelial cell growth factor, insulin-like growth factor, nerve growth factor, osteogenesis-promoting factor, tumor growth factor, hepatocyte growth factor, and transforming growth factor, etc.).

Examples of a cytokinin include macrophage-activating factor, tuftsin, macrophage migrating factor, macrophage-migrating inhibitory factor, thymus gland hormone (for example, thymosin, killer T cell-activating factor, suppressor T cell-inducing factor, and T cell DNA synthesis-inhibitory substance, etc.), cyclosporin, interleukins (for example, interleukin-1, interleukin-2, interleukin-3, interleukin-4, interleukin-5, and interleukin-6, etc.), colony-stimulating factor, muramyl dipeptide, interferons (for example, interferon-$\alpha$, interferon-$\beta$, and interferon-$\gamma$, etc.), tumor necrosis factor, and lymphotoxin.

Examples of an enzyme include proteolytic enzymes (for example, serine protease (for example, thrombin, kallikrein, urokinase, and plasmin, etc.), thiol protease, carboxy protease, and metal protease, etc.), blood coagulation factors (for example, blood coagulation factor I, blood coagulation factor II, blood coagulation factor III, blood coagulation factor VII, blood coagulation factor VIII, blood coagulation factor IX, blood coagulation factor X, blood coagulation factor XI, blood coagulation factor XII, and blood coagulation factor XIII, etc.), plasminogen-activating factor, oxidoreductase (for example, superoxide dismutase, etc.), transferase, lyase, isomerase, ligase, kinase (for example, tyrosine kinase and serine threonine kinase, etc.), phosphatase (for example, liver-derived alkaline phosphatase, bone-derived alkaline phosphatase, and placenta-derived alkaline phosphatase, etc.), decarboxylase and so on.

Examples of other secretory proteins include prostate-specific antigen and apolipoprotein A-I.

Further, examples of a secretory protein also include fusion proteins having addition of other peptides. A peptide to be added to secretory proteins can be selected from peptides that contain a sequence facilitating identification of a protein or a sequence imparting stability upon expression of a protein, for example, peptides containing a full-length or a part of proteins or peptides such as influenza hemagglutinin (HA), glutathione S transferase (GST), substance P, polyhistidine tag (6xHis, 10×His, etc.), protein C fragment, maltose-binding protein (MBP), immunoglobulin constant region fragment, $\alpha$-tubulin fragment, $\beta$-galactosidase, B-tag, c-myc fragment, E-tag (epitope on a monoclonal phage), FLAG (Hopp et al. (1988) Bio/Technol. 6: 1204-10), lck tag, p18 HIV fragment, HSV-tag (human simplex herpes virus glycoprotein), SV40T antigen fragment, T7-tag (T7 gene 10 protein), and VSV-GP fragment (Vesicular stomatitis virus glycoprotein).

Furthermore, secretory proteins also include artificially modified ones so that they can be secreted. Modification of proteins so that they can be secreted can be performed by a method that involves deletion of a membrane-binding domain from a peptide, a method that involves linking a peptide (for example, signal peptide) containing a sequence that is known as a secretion signal, and so on.

A particularly preferable example of such an artificially modified protein so as to be secreted include a secretory placenta-derived alkaline phosphatase described below.

It is preferable that a secretory protein transcribed and translated from the secretory protein-expression vector as described below (hereinafter, also referred to as "secretory proteins of the present invention") is one that is distinguishable from endogenous secretory proteins of a nonhuman animal as described below. Examples of the secretory protein of the present invention that is distinguishable from endogenous secretory proteins include secretory proteins each having a sequence derived from an animal species that is different from the nonhuman animal described below, secretory proteins having a mutated amino acid sequence, and fusion proteins having addition of other peptides.

The secretory proteins of the present invention can be distinguished from endogenous proteins by using an antibody that recognizes the secretory protein of the present invention but does not recognize the endogenous proteins. Further, the secretory protein of the present invention can be distinguished from endogenous proteins by a method that involves treating proteins under a condition under which the secretory protein is not decomposed and/or inactivated but endogenous proteins are decomposed and/or inactivated. Examples of such a condition under which endogenous proteins are decomposed and/or inactivated include heat treatment, enzymatic treatment, and so on.

2. Secretory protein-expression vector

In the present invention, the secretory protein-expression vector refers to a vector that can express a secretory protein and it is not particularly limited as long as it is a vector that can express a secretory protein.
Preferable examples of a secretory protein-expression vector include vectors that have been ligated thereto a polynucleotide containing a transcription regulatory sequence and a polynucleotide coding for a secretory protein. More preferable examples of a secretory protein-expression vector include expression vectors that have been ligated thereto a polynucleotide that codes for a secretory protein located downstream of a transcription regulatory sequence, that is, expression vectors that contain a transcription regulatory sequence and a polynucleotide coding for a secretory protein operably linked to the transcription regulatory sequence.
The type of the vector used for preparing a secretory protein-expression vector is not particularly limited as long as it is a vector that can be designed so as to express a secretory protein. Examples of a vector used for preparing a secretory protein-expression vector include plasmid vectors, cosmid vectors, virus vectors (for example, vectors derived from adenovirus, adeno-associated virus, retrovirus, and so on), and bacteriophage vectors. Also, examples include various vectors such as cloning vectors and expression vectors (Molecular Cloning, A Laboratory Manual 2nd ed., Cold Spring Harbor Press (1989); Current Protocols in Molecular Biology, John Wiley & Sons (1987)).
In the present invention, the transcription regulatory sequence refers to a nucleotide sequence having a transcription initiation activity. Examples of a transcription regulatory sequence include promoters having no transcription regulatory factor-binding sequence, promoters having a transcription regulatory factor-binding sequence, and so on.
Examples of a promoter which can be used include an adenovirus late promoter (Kaufman et al. (1989) Mol. Cell. Biol. 9: 946), CAG promoter (Niwa et al. (1991) Gene 108: 193-200), CMV immediate early promoter (Seed and Aruffo (1987) Proc. Natl. Acad. Sci. USA 84: 3365-9), EF1$\alpha$ promoter (Mizushima et al. (1990) Nucleic Acids Res. 18: 5322; Kim et al. (1990) Gene 91: 217-23), HSV TK promoter, SR$\alpha$ promoter (Takebe et al. (1988) Mol. Cell. Biol. 8: 466), SV40 promoter (Mulligan et al. (1979) Nature 277: 108), SV40 early promoter (Genetic Engineering Vol. 3, Williamson ed., Academic Press (1982) pp.83-141), SV40 late promoter (Gheysen and Fiers (1982) J. Mol. Appl. Genet. 1: 385-94), RSV (Rous sarcoma virus)-LTR promoter (Cullen (1987) Methods Enzymol. 152: 684-704), and MMLV-LTR promoter. In the case that a nucleotide sequence of a promoter is known, those skilled in the art can easily obtain a polynucleotide composed of the same nucleic acid sequence.
Here, "polynucleotide" refers to a polymer composed of a plurality of nucleotides or nucleotide pairs such as a deoxyribonucleic acid (DNA) or ribonucleic acid (RNA), which includes DNA, cDNA, genomic DNA, chemically synthesized DNA, and RNA. In addition, the term encompasses a polynucleotide that comprises a nucleotide other than a natural one as required.
A transcription regulatory factor-binding sequence means a sequence to which a transcription regulatory factor binds. Examples of a transcription regulatory factor-binding sequence include an enhancer, a suppressor, an upstream regulatory sequence and so on.
Examples of a transcription regulatory factor-binding sequence which can be used include hypoxia response element (hereinafter, also referred to as "HRE", Kimura, et. al. (2001) The Journal of Biological Chemistry, 276: 2292-2298), IL4 responsible element (hereinafter, also referred to as "IL4RE", Richard Moriggl, et. al. (1997) Molecular and Cellular Biology, vol. 17: 3663-3678), E2F-binding nucleotide sequence (Ginsberg, et. al. (1994) Genes & development, 8 (22): 2665-2679), estrogen receptor-binding sequence (Fawell, et. al. (1990) Cell, 60 (6): 953-962), GATA-1-binding nucleotide sequence (Orkin (1990) Cell, 63 (4): 665-72), AP1-binding nucleotide sequence (Wasylyk, et. al. (1989) Molecular and Cellular Biology, 9 (5): 2247-2250), p53-binding nucleotide sequence (Levine, et. al. (1991) Nature, 351 (6326): 453-6), and other transcription regulatory factor-binding sequence (Steffen, et. al. (1992) Nucleic Acids Research, 20 (1): 3-26).
Examples of HRE include a sequence represented by SEQ ID NO: 1, a sequence represented by SEQ ID NO: 2, a sequence represented by SEQ ID NO: 3, and a sequence represented by SEQ ID NO: 4, and preferably include the sequence represented by SEQ ID NO: 1.
Examples of IL4RE include a sequence represented by SEQ ID NO: 5, a sequence represented by SEQ ID NO: 6, a sequence represented by SEQ ID NO: 7, and a sequence represented by SEQ ID NO: 8 (Martin Seidel, et al. (1995) Proc. Natl. Acad. Sci, USA, vol. 92: 3041-3045), and preferably include the sequence represented by SEQ ID NO: 8.
Polynucleotide comprising the transcription regulatory factor-binding sequence can be obtained with ease on the basis of a nucleic acid sequence of the transcription regulatory factor-binding sequence. As a more specific example, a double-strand polynucleotide containing a desired transcription regulatory factor-binding sequence can be obtained by chemically synthesizing single strand polynucleotides each having the same sequence as the transcription regulatory factor-binding sequence or a sequence complementary thereto, mixing the polynucleotides, then heating them at 95°C for 2 to 10 minutes, and then cooling them at 37°C for 1 hour and at room temperature for 1 hour.
On the other hand, a polynucleotide coding for a secretory protein can be obtained from cDNA library or genomic library of animals such as human, mouse, rat, rabbit, hamster, chicken, pig, bovine, goat, and sheep, preferably human, by designing primers on the basis of the nucleotide sequence coding for the secretory protein and by using a gene amplification technique (e.g., PCR) (Current Protocols in Molecular Biology, John Wiley & Sons (1987) Section 6.1-6.4).

For the method of preparing cDNA library, reference can be made to "Molecular Cloning, A Laboratory Manual, 2nd ed." (Cold Spring Harbor Press (1989)). Commercially available cDNA library or genomic library can also be used.

More specifically, in preparation of cDNA library, first, total RNA is prepared from cells, organ, tissue, and so on (for example, placenta) that express a polynucleotide coding for a secretory protein by a guanidine ultracentrifugation method (Chirwin et al. (1979) Biochemistry 18: 5294-9), AGPC method (Chomczynski and Sacchi (1987) Anal. Biochem. 162: 156-9), or the like, and then mRNA is purified by using, for example, mRNA purification Kit (Pharmacia). A kit for directly preparing mRNA, such as Quick Prep mRNA Purification Kit (Pharmacia) may also be used. Then, cDNA is synthesized from the obtained mRNA by using a reverse transcriptase. Also, a kit for cDNA synthesis such as AMV Reverse Transcriptase First-strand cDNA Synthesis Kit (Seikagaku Corporation) is commercially available. As another method, cDNA can be synthesized and amplified by a 5'-RACE method which utilizes PCR (Frohman et al. (1988) Proc. Natl. Acad. Sci. USA 85: 8998-9002; Belyavsky et al. (1989) Nucleic Acids Res. 17: 2919-32). Further, to prepare cDNA library having high full-length ratio, a known method such as an oligo cap method (Maruyama and Sugano (1994) Gene 138: 171-4; Suzuki (1997) Gene 200: 149-56) can be adopted. The cDNA obtained as mentioned above can be inserted into an appropriate vector.

Confirmation of the nucleic acid sequence of the polynucleotide coding for the secretory protein can be performed by sequencing with a conventional method. For example, it can be performed by a dideoxynucleotide chain termination method (Sanger et al. (1977) Proc. Natl. Acad. Sci. USA 74: 5463) or the like. Also, it is possible to analyze a sequence by using an appropriate DNA sequencer.

Insertion of a polynucleotide containing a transcription regulatory sequence, a polynucleotide coding for a secretory protein and so on into a vector can be performed by a ligase reaction. In this case, a restriction enzyme site can also be utilized (Current Protocols in Molecular Biology, John Wiley & Sons (1987) Section 11.4-11.11; Molecular Cloning, A Laboratory Manual 2nd ed., Cold Spring Harbor Press (1989) Section 5.61-5.63).

On the other hand, a secretory protein-expression vector may contain a marker gene which enables selection of host cells into which the secretory protein-expression vector has been transfected. Examples of a marker gene that can be selected include drug-resistant genes (e.g., neomycin-resistant gene, hygromycin-resistant gene, puromycin-resistant gene, etc.) and polypeptides coding for fluorescent proteins (e.g., GFP, EGFP, etc.).

Further, a secretory protein-expression vector can preferably have an ori for replication of the vector in *Escherichia coli,* and a marker gene for selecting transformed hosts. It is preferable to use drug-resistant genes that enable selection of hosts by means of the drugs such as ampicillin, tetracyclin, kanamycin, and chloramphenicol.

An example of the secretory protein-expression vector preferably includes a PLAP vector of the present invention as described below.

3. Cells that have been transfected with a secretory protein-expression vector

Preparation of cells that have been transfected with a secretory protein-expression vector (which may also be referred to herein as "secretory protein-expression vector -transfected cells") can be performed by transfection of cells with the above-mentioned secretory protein-expression vector according to a conventional method.
Hereinafter, a method of preparing secretory protein-expression vector-transfected cells is explained in detail.

Cells into which a secretory protein-expression vector is transfected may be any cells that can be transplanted into an animal, and the type thereof is not particularly limited. Preferable examples of such cells include eukaryotic cells derived from mammals (for example, retina cells, liver cells, spleen cells, nerve cells, glia cells, pancreatic β cells, bone marrow cells, mesangium cells, Langerhans cells, epidermal cells, epithelial cells, endothelial cells, fibroblasts, fiber cells, muscle cells, adipose cells, immune cells (for example, macrophages, T cells, B cells, natural killer cells, mast cells, neutrophil leucocytes, basophil leucocytes, acidophil leucocytes, and monocytes), megakaryocytes, synoviocytes, cartilage cells, bone cells, osteoblasts, osteoclasts, mammary cells, liver cells, or interstitial cells, or precursor cells thereof, stem cells, and immortalized cells or tumor cells), and immortalized cells and tumor cells are particularly preferable.

Introduction of the secretory protein-expression vector into host cells can be performed by an electroporation method (Chu et al. (1987) Nucleic Acids Res. 15: 1311-26), a cationic liposome method, a pulse electroporation method (Current Protocols in Molecular Biology, John Wiley & Sons (1987) Section 9.1-9.9), a direct injection method using a micro glass tube, a microinjection method, lipofection (Derijard (1994) Cell 7: 1025-37; Lamb (1993) Nature Genetics 5: 22-30; Rabindran et al. (1993) Science 259: 230-4), a lipofectamine method (GIBCO-BRL), a calcium phosphate method (Chen and Okayama (1987) Mol. Cell. Biol. 7: 2745-52), a DEAE dextran method (Lopata et al. (1984) Nucleic Acids Res. 12: 5707-17; Sussman and Milman (1985) Mol. Cell. Biol. 4: 1642-3), FuGene6 reagent (Boehringer-Mannheim), or the like.

Culture of host cells can be performed by a known method that is suitable for the selected cells. For example, media such as DMEM, MEM, RPMI 1640, IMDM, and F12 can be used and serum such as fetal calf serum (FCS), amino acids, glucose, penicillin, and streptomycin may be added thereto as necessary and culture may be performed at pH of about 6 to about 8 at 30 to 40°C for about 15 to about 200 hours. The media may be exchanged, and aeration and agitation may be performed during culture as necessary.

While the secretory protein-expression vector-transfected cells can be used as they are, cloning can be preformed to avoid deviation of properties during culture, and/or to make it possible to obtain cells that express more secretory protein for stable evaluation. The cloning of cells can be performed by a conventional method (for example, an ultra-dilution method, cell sorting by flow cytometry, or the like). After cloning, most suitable cell line transfected with the secretory protein-expression vector of the present invention can be selected by measuring an amount of the secretory protein secreted out of the cell (hereinafter, also referred to as "secretory protein amount") or analyzing a copy number of mRNA of the secretory protein by a molecular biological technique (for example, a quantitative RT-PCR method, Northern blotting, etc.).

An example of secretory protein-expression vector-transfected cell preferably includes PLAP vector-transfected cell of the present invention as described below.

4. Method of measuring an amount of a secretory protein

An amount of a secretory protein contained in a sample containing the secretory protein can be measured by a known method. Although a sample containing a secretory protein may be any sample that contains a secretory protein without particular limitations, biological fluids such as blood, plasma, serum, urine, cerebrospinal fluid, and so on can be used, and blood, plasma, and serum can be preferably used, and plasma can be particularly preferably used as described below.

The method of measuring an amount of a secretory protein is not particularly limited, and measurement can be performed by means of immunological techniques (for example, ELISA, RIA, EIA, flow cytometry, and Western blotting), chromatography, mass spectrometry, enzymatic activity, or the like. For example, when the amount is measured by ELISA method, an antibody to the secretory protein (primary antibody) is immobilized on a carrier such as a plate and then a sample containing the secretory protein is added, followed by incubation. Subsequently, a secondary antibody that recognizes the secretory protein-recognizing antibody is added and the plate is incubated. After that, the plate is washed and a label conjugated to the secondary antibody is detected. In this manner, the amount of the secretory protein can be determined. The primary antibody and secondary antibody which are used for the measurement may be commercially available ones or may be prepared by a known method. Further, when a peptide comprising a full-length or a part of a peptide consisting of a sequence that facilitates identification of proteins such as influenza hemagglutinin (HA), glutathione S transferase (GST), substance P, poly-histidine tag (6xHis, 10xHis, etc.), protein C fragment, maltose-binding protein (MBP), immunoglobulin constant region fragments, α-tubulin fragment, β-galactosidase, B-tag, c-myc fragment, E-tag (epitope on a monoclonal phage), FLAG (Hopp et al. (1988) Bio/Tehcnol. 6: 1204-10), 1ck tag, p18 HIV fragment, HSV-tag (human simplex herpes virus glycoprotein), SV40T antigen fragment, T7-tag (T7 gene10 protein), and VSV-GP fragment (Vesicular stomatitis virus glycoprotein) is attached to the secretory protein in advance, an antibody that recognizes these peptides can be used as a primary antibody.

Further, when the secretory protein is an enzyme, the amount of the secretory protein can be calculated by measuring enzymatic activity. One skilled in the art can easily understand that the amount of the secretory protein can be quantitatively measured by measuring enzymatic activity. In the present invention, the measurement of the amount of the secretory protein includes measuring enzymatic activity.

Measurement of enzymatic activity can be performed, for example, as described hereinafter. A sample containing a secretory protein and a substrate solution are mixed and the mixture is incubated. Subsequently, the amount of the product produced by the enzymatic reaction or the amount of the added substrate is measured. In this manner, the enzymatic activity can be measured. Further, the amount of the secretory protein can be measured on the basis of the enzymatic activity.

The amounts of the substrate and of the product can be measured based on absorbance, intensity of fluorescence, intensity of chemiluminescence, or the like. Here, the absorbance, intensity of fluorescence, intensity of chemiluminescence, or the like may be either that derived from the substrate or product or that derived from a label attached to the antibody that recognizes the substrate or product.

It is preferable that the amount of the secretory protein in the sample containing the secretory protein is measured while distinguishing the secretory protein from the endogenous secretory proteins in the nonhuman animal described below. Examples of the method of distinguishing the secretory protein of the present invention from endogenous secretory proteins include a method using a secretory protein having a sequence derived from an animal species different from the nonhuman animal described below, a method using a secretory protein having a mutated amino acid sequence, and a method using a fusion protein having addition of another peptide.

In these methods, the secretory protein of the present invention can be measured while distinguishing it from endogenous proteins by an immunological technique using an antibody that recognizes the secretory protein of the present invention but does not recognize endogenous proteins. Also, the secretory protein of the present invention can be measured while distinguishing it from endogenous proteins by treating them under a condition under which the secretory protein of the present invention is not decomposed and/or inactivated but endogenous proteins are decomposed and/or inactivated. Examples of the conditions under which endogenous proteins are decomposed and/or inactivated include heat treatment, enzymatic treatment, and so on.

5. Method of measuring transcriptional activity in transplanted cells in a nonhuman animal model

In a nonhuman animal model that produces a secretory protein obtained by transplanting secretory protein-expression vector-transfected cells into a nonhuman animal, an amount of the secretory protein in a biological fluid is measured and based on the amount of the secretory protein, transcriptional activity through a transcription regulatory sequence in the transplanted cells in the nonhuman animal model can be determined.

Hereinafter, more detailed description is made.

The secretory protein-expression vector-transfected cells can be obtained and cultured as described above. Also, the cells to be transplanted may be those cultured in vivo (Asano et al, Jpn J Cancer Res. 1999 Jan; 90(1):93-100).
The secretory protein-expression vector-transfected cells are transplanted into a nonhuman animal. The nonhuman animals to be transplanted are not particularly limited, and examples thereof include mouse, rat, guinea pig, hamster, rabbit, dog, monkey, chicken, pig, sheep, bovine, and cat. The examples preferably include mice and rats, and particularly preferably include mice. Meanwhile, the transplantation sites in a nonhuman animal are not particularly limited. Examples thereof include subcutaneous transplantation, intraperitoneal transplantation, transplantation in blood, and transplantation in organs (for example, brain, each site of brain (for example, retina, olfactory bulb, amygdaloid nucleus, basal ganglion, hippocampus, thalamus, hypothalamus, brain cortex, medullary, cerebellum, etc.), spinal cord, pituitary, stomach, pancreas, kidney, liver, genital glands, thyroid, gall bladder, bone marrow, adrenal, skin, muscle, lung, digestive tracts (for example, large intestine and small intestine), blood vessels, heart, thymus gland, spleen, mandibular gland, peripheral blood, prostate gland, testis, ovary, placenta, uterus, bone, joints, skeletal muscle etc.).
Number of cells to be transplanted is not particularly limited, and is, for example, $10^2$ to $10^9$ cells/head, preferably $10^4$ to $10^8$ cells/head, more preferably $10^5$ to $10^7$ cells/head, and particularly preferably $5 \times 10^5$ to $5 \times 10^6$ cells/head.
Upon transplantation, the cells may be transplanted as they are, or the cells may be transplanted along with a pharmaceutically acceptable carrier. Examples of the carrier include physiological saline, phosphate buffer, culture medium, serum, a biological fluid, and carboxymethylcellulose solution. Further, the cells may be transplanted in combination with solids that provide an anchorage for the cells (for example, cytodex3 (Amersham Bioscience, 17-0485-01), etc.), extracellular matrix components (for example, collagen, fibronectin, vitronectin, laminin, heparan sulfate, proteoglycan, glycosaminoglycan, chondroitin sulfate, hyaluron, or elastin or a combination of two or more of them) or gel-like supports.
Further, upon transplantation, the cells that contain angiogenesis factors (for example, vessel endothelial cell growth factor (VEGF), basic fibroblast growth factor (bFGF), acidic fibroblast growth factor (aFGF), platelet-derived growth factor (PDGF), transforming growth factor-β (TGF-β), angiopoietin, hepatocyte growth factor (HGF), etc.) may be transplanted.
After transplantation, the animal is raised for a suitable period of time (for example, 1 hour to 1,095 days, preferably 2 hours to 365 days, more preferably 24 hours to 180 days, etc.), and biological fluids are collected. A kind of the biological fluid is not particularly limited as long as it is derived from the nonhuman animal, and examples of the biological fluid include blood, plasma, serum, urine, and cerebrospinal fluid, and blood, plasma and serum are preferable, and plasma and serum are more preferable, and plasma is particularly preferable.
The collected biological fluid may be fractionated into a fraction containing the secretory protein and a fraction containing no secretory protein as necessary. In the case of blood, it is preferable to separate plasma by centrifugation and use it as a biological fluid.
The separated biological fluid can be stored at a suitable temperature, preferably 4°C or lower, and particularly preferably -20°C or lower until the amount of the secretory protein is measured.
The amount of the secretory protein in the biological fluid can be measured by the above-mentioned method of measuring the amount of the secretory protein.
Here, the secretory protein is a protein that is transcribed from the secretory protein-expression vector, translated into the secretory protein, and secreted into the biological fluid. Therefore, the amount of the secretory protein obtained by the above-mentioned method changes depending on the transcriptional activity through a transcription regulatory sequence in the secretory protein-expression vector, so the amount of the secretory protein is measured, and based on the amount of the secretory protein, transcriptional activity through the transcription regulatory sequence inserted into the secretory protein-expression vector can be determined.

6. Method of screening a compound that affects transcriptional activity in a nonhuman animal model

A compound that affects transcriptional activity can be screened in a nonhuman animal model by administering a test compound to a nonhuman animal model that produces a secretory protein, which is obtained by transplanting secretory protein-expression vector transfected cells into a nonhuman animal, measuring an amount of the secretory protein in a biological fluid, and selecting a compound that changes the amount of the secretory protein.
Further, a compound that affects transcriptional activity in a nonhuman animal model can be screened by measuring an amount of a secretory protein in a biological fluid of the nonhuman animal model that produces the secretory protein which is obtained by transplanting secretory protein-expression vector-transfected cells into a nonhuman animal that have been administered with a test compound, and selecting a compound that changes the amount of the secretory protein.

Hereinafter, detailed description is made.

In the method of screening a compound that affects transcriptional activity in a nonhuman animal model, it is preferable that the secretory protein-expression vector contains a polynucleotide having a transcription regulatory factor-binding sequence. The transcription regulatory factor-binding sequence is not particularly limited and examples of the transcription regulatory factor-binding sequence that can be used include HRE, IL4RE, E2F-binding nucleotide sequence, estrogen receptor-binding nucleotide sequence, GATA-1-binding nucleotide sequence, AP1-binding nucleotide sequence, p53-binding nucleotide sequence, and other transcription factor-binding sequences, and among them, HRE or IL4RE are preferable.
Examples of HRE include a sequence represented by SEQ ID No: 1, a sequence represented by SEQ ID No: 2, a sequence represented by SEQ ID No: 3, and a sequence represented by SEQ ID No: 4, and among them, the sequence represented by SEQ ID No: 1 is preferable.
Examples of IL4RE include a sequence represented by SEQ ID No: 5, a sequence represented by SEQ ID No: 6, a sequence represented by SEQ ID No: 7, and a sequence represented by SEQ ID No: 8 (Martin Seidel, et al. (1995) Proc. Natl. Acad. Sci. USA, vol92:3041-3045), and among them, the sequence represented by SEQ ID No: 8 is preferable.
A nonhuman animal model can be prepared by transplanting secretory protein-expression vector-transfected cells into a nonhuman animal model by the above-mentioned method.
The methods of administering a compound to a nonhuman animal model are not particularly limited and examples thereof include oral administration, intravenous administration, subcutaneous administration, intraperitoneal administration, intramuscular administration, and intracranial administration. On the other hand, a non-administered group, a vehicle-administered group and so on can be used as a control group.
The amount of a compound to be administered to the nonhuman animal model is not particularly limited and is, for example, 1 ng/kg to 1 g/kg, preferably 100 ng/kg to 1 g/kg, more preferably 1 mg/kg to 1 g/kg, and particularly preferably 10 mg/kg to 300 mg/kg.
The timing of administering a compound to the nonhuman animal model is not particularly limited and is, for example, before transplanting the cells, at the time of transplanting the cells, after transplanting the cells, and so on.
The measurement of the amount of the secretory protein in a biological fluid can be performed by the above-mentioned method.
A compound that affects transcriptional activity can be screened by comparing an amount of the secretory protein in a biological fluid of the nonhuman animal model to which a compound has been administered, with an amount of the secretory protein in a biological fluid of the control group.
In the screening method, for example, when the amount of the secretory protein in the biological fluid of the nonhuman animal model to which a compound has been administered increases or decreases 1.2 times or more, preferably 1.5 times or more, more preferably 2.0 times or more, and particularly preferably 3.0 times or more, as compared with the amount of the secretory protein in the biological fluid of the control group, it is judged that the transcriptional activity is affected.
In the present invention, examples of test compounds include peptides, proteins, antibodies, nonpeptidic compounds, synthetic compounds, polynucleotides, fermented products, cell extracts, plant extracts, animal tissue extracts, and plasma. Examples of the polynucleotides include antisense polynucleotides, ribozyme nucleotides, and double strand RNA. Here, a double strand RNA refers to a double strand RNA that causes RNA interference (Fire, et. al. (1998) Nature, 391:806-811).

7. Method of measuring the number of transplanted cells and/or tumor volume based on the amount of the secretory protein in a nonhuman animal model

In the nonhuman animal model that produces a secretory protein, which is obtained by transplanting secretory protein-expression vector-transfected cells into the nonhuman animal model, an amount of the secretory protein in a biological fluid is measured and based on the amount of the secretory protein, number of the transplanted cells can be determined.
Further, by transplanting the secretory protein-expression vector-transfected cells into a nonhuman animal model, a tumor develops in the nonhuman animal and the secretory protein can be produced in the tumor. In this case, the amount of the secretory protein in a biological fluid is measured and based on the amount of the secretory protein, tumor volume in the nonhuman animal model can be determined.

Hereinafter, detailed description is made.

In the method of measuring the number of the transplanted cells and/or tumor volume based on the amount of the secretory protein in a nonhuman animal model, it is preferable that the secretory protein-expression vector comprises a polynucleotide consisting of a constitutive transcription regulatory sequence. Here, the constitutive transcription regulatory sequence refers to a sequence that has a constitutive transcription initiation activity. The constitutive transcription regulatory sequence is not particularly limited and, for example, SV40 promoter, CMV promoter, thymidine kinase promoter, Ubiquitin C promoter, Elongation factor 1 alpha (EF1a) promoter, β-actin promoter, Glyceraldehyde-3-phosphate dehydrogenase promoter, Phosphoglycerokinase promoter, β2-Microglobulin promoter, β-Glucuronidase promoter, and so on can be used. An example of the SV40 promoter includes a sequence represented by SEQ ID No: 9. By the above-mentioned method, the secretory protein-expression vector-transfected cells can be transplanted into a nonhuman animal and the amount of the secretory protein in a biological fluid can be measured.
Here, transcriptional activity by a constitutive transcription regulatory sequence is considered to show always a certain value, so the secretory protein is secreted into the biological fluid in an amount depending on the number of the transplanted cells. Therefore, the amountof the secretory protein obtained by the above-mentioned method changes depending on the number of the transplanted cells in the nonhuman animal model, so the amount of the secretory protein is measured and based on the amount of the secretory protein, the number of the transplanted cells can be determined. Further, in the present invention, "number of the transplanted cells" refers to the number of cells derived from the cells that have been artificially transplanted into a nonhuman animal, and includes the number of cells after an increase or decrease of the transplanted cells in the nonhuman animal.
Therefore, comparison between the amount of the secretory protein in the biological fluid before and after raising of the animal for a suitable period of time enables measurement of an increase or decrease in number of transplanted cells in a nonhuman animal model. Further, in solid tumors, the cell number is considered to correlate with the tumor volume and hence the increase or decrease in the tumor volume can be measured in the case where solid tumor is caused to develop in the nonhuman animal.
8. Method of screening a compound that affects the number of transplanted cells and/or tumor volume based on the amount of the secretory protein in a nonhuman animal model
A compound that affects the number of transplanted cells can be screened in a nonhuman animal model by administering a nonhuman animal model that produces a secretory protein, which is obtained by transplanting secretory protein-expression vector-transfected cells into a nonhuman animal, measuring an amount of the secretory protein in a biological fluid, and selecting a compound that changes the amount of the secretory protein.
Also, a compound that affects the number of the transplanted cells can be screened in a nonhuman animal model by measuring an amount of the secretory protein in a biological fluid of a nonhuman animal model that produces the secretory protein, which is obtained by transplanting the secretory protein-expression vector-transfected cells into a nonhuman animal which has been administered with a compound; and selecting a compound that changes the amount of the secretory protein.
Further, transplanting the secretory protein-expression vector-transfected cells into a nonhuman animal generates tumor in the nonhuman animal and the secretory protein is produced in the tumor. In this case, a compound that affects tumor volume in the nonhuman animal model can be screened by administering a compound to the nonhuman animal model, measuring an amount of the secretory protein in a biological fluid, and selecting a compound that changes the amount of the secretory protein.

Hereinafter, detailed description is made.

In the method of screening a compound that affects the number of the transplanted cells and/or tumor volume based on the amount of the secretory protein in a nonhuman animal model, it is preferable that the secretory protein-expression vector comprises a polynucleotide consisting of a constitutive transcription regulatory sequence. Here, the constitutive transcription regulatory sequence refers to a sequence that has a constitutive transcription initiation activity. The constitutive transcription regulatory sequence is not particularly limited and, for example, SV40 promoter, CMV promoter, thymidine kinase promoter, Ubiquitin C promoter, Elongation factor 1 alpha (EF1a) promoter, β-actin promoter, Glyceraldehyde-3-phosphate dehydrogenase promoter, Phosphoglycerokinase promoter, β2-Microglobulin promoter, β-Glucuronidase promoter, and so on can be used. An example of the SV40 promoter includes a sequence represented by SEQ ID No: 9.

By the above-mentioned method, the secretory protein-expression vector-transfected cells can be transplanted to prepare a nonhuman animal model.

The method of administering a compound to a nonhuman animal model is not particularly limited and examples thereof include oral administration, intravenous administration, subcutaneous administration, intraperitoneal administration, intramuscular administration, intracranial administration and so on. A non-administered group, a vehicle-administered group, and so on can be used as a control group.

The amount of a compound to be administered to the nonhuman animal model is not particularly limited and is, for example, 1 ng/kg to 1 g/kg, preferably 100 ng/kg to 1 g/kg, more preferably 1 mg/kg to 1 g/kg, and particularly preferably 10 mg/kg to 300 mg/kg.

The timing of administering a compound to the nonhuman animal model is not particularly limited and is, for example, before transplanting the cells, at the time of transplanting the cells, after transplanting the cells, and so on.

The measurement of the amount of the secretory protein in a biological fluid can be performed by the above-mentioned method.

The compound that affects the number of transplanted cells can be screened by comparing the amount of the secretory protein in a biological fluid of the nonhuman animal model to which a compound has been administered, with the amount of the secretory protein in a biological fluid of the control group. In solid tumors, cell number is considered to correlate with tumor volume, so the compound that affects the tumor volume can also be screened. In the screening method, for example, when the amount of the secretory protein in a biological fluid of the nonhuman animal model to which a compound has been administered increases or decreases 1.2 times or more, preferably 1.5 times or more, more preferably 2.0 times or more, and particularly preferably 3.0 times or more, as compared with the amount of the secretory protein in a biological fluid of the control group, it is judged that the number of the transplanted cells and/or the tumor volume is affected.

9. Measuring kit

A measuring kit that contains a secretory protein-expression vector is used in (1) a method of measuring transcriptional activity in transplanted cells in a nonhuman animal model (the above-mentioned "5. Method of measuring transcriptional activity in transplanted cells in a nonhuman animal"), (2) a method of screening a compound that affects transcriptional activity in a nonhuman animal model (the above-mentioned "6. Method of screening a compound that affects transcriptional activity in a nonhuman animal model"), (3) a method of measuring the number of transplanted cells and/or tumor volume based on the amount of the secretory protein in a nonhuman animal model (the above-mentioned "7. Method of measuring the number of transplanted cells and/or tumor volume based on the amount of the secretory protein in a nonhuman animal model"), and (4) a method of screening a compound that affects the number of transplanted cells and/or tumor volume based on the amount of the secretory protein in a nonhuman animal model (the above-mentioned "8. Method of screening a compound that affects the number of transplanted cells and/or tumor volume based on the amount of the secretory protein in a nonhuman animal model"). The measuring kit has only to contain a secretory protein-expression vector, and other components are not limited.

The measuring kit may contain, for example, a control vector, a transfection reagent, cell culture solution, an antibody to the secretory protein for measurement, and buffer solution for measurement.

A measuring kit that contains secretory protein-expression vector-transfected cells is used in (1) a method of measuring transcriptional activity in transplanted cells in a nonhuman animal model (the above-mentioned "5. Method of measuring transcriptional activity in transplanted cells in a nonhuman animal"), (2) a method of screening a compound that affects transcriptional activity in a nonhuman animal model (the above-mentioned "6. Method of screening a compound that affects transcriptional activity in a nonhuman animal model"), (3) a method of measuring the number of transplanted cells and/or tumor volume based on the amount of the secretory protein in a nonhuman animal model (the above-mentioned "7. Method of measuring the number of transplanted cells and/or tumor volume based on the amount of the secretory protein in a nonhuman animal model"), and (4) a method of screening a compound that affects the number of transplanted cells and/or tumor volume based on the amount of the secretory protein in a nonhuman animal model (the above-mentioned "8. Method of screening a compound that affects the number of transplanted cells and/or tumor volume based on the amount of the secretory protein in a nonhuman animal model"). The measuring kit has only to contain secretory protein-expression vector-transfected cells, and other components are not limited.

The measuring kit may contain, for example, control cells, cell culture solution, an antibody to the secretory protein for measurement, and buffer solution for measurement.

A measuring kit that contains a nonhuman animal into which secretory protein-expression vector-transfected cells have been transplanted is used in (1) a method of measuring transcriptional activity in transplanted cells in a nonhuman animal model (the above-mentioned "5. Method of measuring transcriptional activity in transplanted cells in a nonhuman animal"), (2) a method of screening a compound that affects transcriptional activity in a nonhuman animal model (the above-mentioned "6. Method of screening a compound that affects transcriptional activity in a nonhuman animal model"), (3) a method of measuring the number of transplanted cells and/or tumor volume based on the amount of the secretory protein in a nonhuman animal model (the above-mentioned "7. Method of measuring the number of transplanted cells and/or tumor volume based on the amount of the secretory protein in a nonhuman animal model"), and (4) a method of screening a compound that affects number of transplanted cells and/or tumor volume based on the amount of the secretory protein in a nonhuman animal model (the above-mentioned section "8. Method of screening a compound that affects number of transplanted cells and/or tumor volume based on the amount of the secretory protein in a nonhuman animal model"). The measuring kit has only to contain a nonhuman animal into which the secretory protein-expression vector-transfected cells have been transplanted, and other components are not limited.
The measuring kit may contain, for example, a control nonhuman animal, an antibody to the secretory protein for measurement, and buffer solution for measurement.

10. PLAP of the present invention

In the present invention, a secretory protein is preferably a secretory enzyme, more preferably a secretory alkaline phosphatase, and particularly preferably a secretory placenta-derived alkaline phosphatase. The secretory alkaline phosphatase is more preferably a heat-resistant, secretory alkaline phosphatase. Here, the term "heat-resistant" means that enzymatic activity of the enzyme is not lost by heat treatment, at not less than 40°C, preferably not less than 45°C, more preferably not less than 50°C, further more preferably not less than 55°C, much more preferably not less than 60°C, and particularly preferably not less than 65°C, for not less than 5 minutes, preferably for not less than 10 minutes, and more preferably for not less than 20 minutes.
Hereinafter, a case in which a secretory placenta-derived alkaline phosphatase is used as a secretory protein is explained in detail.
In the present invention, a placenta-derived alkaline phosphatase (hereinafter, also referred to as "PLAP") refers to a polypeptide that is derived from placenta and has enzymatic activity as alkaline phosphatase, including a polypeptide that comprises, for example, an amino acid sequence represented by GenBank Accession No. M13077, preferably a polypeptide consisting of the amino acid sequence represented by GenBank Accession No. M13077. A method of measuring an enzymatic activity of alkaline phosphatase is not particularly limited and the enzymatic activity of the alkaline phosphatase can be measured by, for example, the method of measuring PLAP activity described below. Further, the secretory placenta-derived alkaline phosphatase (hereinafter, also referred to as "PLAP of the present invention") refers to a polypeptide that has an enzymatic activity of alkaline phosphatase and is modified so that it can be secreted out of the cell. A polypeptide comprising an amino acid sequence represented by SEQ ID No: 11 is preferable and a polypeptide consisting of an amino acid sequence represented by SEQ ID No: 11 is more preferable. Further, a polypeptide comprising an amino acid sequence that is substantially the same as the amino acid sequence represented by SEQ ID No: 11, preferably a polypeptide consisting of an amino acid sequence that is substantially the same as the amino acid sequence represented by SEQ ID No: 11 can be used.

Hereinafter, the PLAP of the present invention is explained in detail.

Examples of an amino acid sequence that is substantially the same as the amino acid sequence represented by SEQ ID No: 11 include an amino acid sequence represented by SEQ ID No: 13 and an amino acid sequence represented by SEQ ID No: 15.
Further, examples of an amino acid sequence that is substantially the same as the amino acid sequence represented by SEQ ID No: 11 include an amino acid sequence having a homology of about 90% or more, preferably about 95% or more, and more preferably about 98% or more to the amino acid sequence represented by SEQ ID No: 11. In particular, examples of an amino acid sequence that is substantially the same as the amino acid sequence represented by SEQ ID No: 11 include, besides the above-mentioned amino acid sequence, an amino acid sequence represented by SEQ ID No: 11 in which mutation such as deletion, substitution, or addition has occurred in one or plural (for example one or several) amino acids thereof and the amino acid sequence of a polypeptide having an enzymatic activity of alkaline phosphatase.
Examples thereof include (i) an amino acid sequence represented by SEQ ID No: 11, in which 1 to 5 amino acids, preferably 1 to 3 amino acids, more preferably 1 to 2 amino acids, and still more preferably one amino acid has been deleted, (ii) an amino acid sequence represented by SEQ ID No: 11, in which 1 to 5 amino acids, preferably 1 to 3 amino acids, more preferably 1 to 2 amino acids, and still more preferably one amino acid has been added, (iii) an amino acid sequence represented by SEQ ID No: 11, in which 1 to 5 amino acids, preferably 1 to 3 amino acids, more preferably 1 to 2 amino acids, and still more preferably one amino acid has been inserted, (iv) an amino acid sequence represented by SEQ ID No: 11, in which 1 to 5 amino acids, preferably 1 to 3 amino acids, more preferably 1 to 2 amino acids, and still more preferably one amino acid has been substituted by other amino acid(s), and (v) amino acid sequence resulting from the combinations of the above-mentioned (i) to (iv).

Those polypeptides having amino acid sequence with deletion, insertion, substitution, or addition of 1 or plural amino acids and having the same biological activity as that of the original polypeptide are encompassed by the scope of the present invention (Mark et al. (1984) Proc. Natl. Acad. Sci. USA 81: 5662-6; Zoller and Smith (1982) Nucleic Acids Res. 10: 6487-500; Wang et al. (1984) Science 224: 1431-3; Dalbadie-McFarland et al. (1982) Proc. Natl. Acad. Sci. USA 79: 6409-13).

Here, substitution of amino acids refers to a mutation in which one or more amino acid residues in a sequence are replaced by amino acid residues of different kinds. When modifying the amino acid sequence of the PLAP of the present invention by such substitution, it is preferable that conservative substitution is performed in order to maintain the function of the protein. The conservative substitution refers to changing a sequence so that it codes an amino acid that has a similar property as the amino acid before the substitution. Amino acids can be grouped according to the properties thereof into, for example, nonpolar amino acids (e.g., Ala, Ile, Leu, Met, Phe, Pro, Trp, Val), uncharged amino acids (e.g., Asn, Cys, Gln, Gly, Ser, Thr, Tyr), acidic amino acids (e.g., Asp, Glu), basic amino acids (e.g., Arg, His, Lys), neutral amino acids (e.g., Ala, Asn, Cys, Gln, Gly, Ile, Leu, Met, Phe, Pro, Ser, Thr, Trp, Tyr, Val), aliphatic amino acids (e.g., Ala, Gly), branched amino acids (e.g., Ile, Leu, Val), hydroxyamino acids (e.g., Ser, Thr), amido-type amino acids (e.g., Gln, Asn), sulfur-containing amino acids (e.g., Cys, Met), aromatic amino acids (e.g., His, Phe, Trp, Tyr), heterocyclic amino acids (e.g., His, Trp), and imino acids (e.g., Pro, 4Hyp).
Therefore, it is preferable that substitution is performed between nonpolar amino acids, or between uncharged amino acids. Among them, substitutions between Ala, Val, Leu, and Ile, between Ser and Thr, between Asp and Glu, between Asn and Gln, between Lys and Arg, and between Phe and Tyr are preferable as substitutions that maintain the properties of the protein. The number and sites of amino acids to be modified are not particularly limited.

The PLAP of the present invention includes a fusion protein having addition of other peptide. A peptide to be attached to the PLAP of the present invention can be selected from peptides containing a sequence that facilitates identification of proteins or a sequence that imparts stability upon expression of a protein, the peptides comprising a full-length or a part of the proteins or peptides such as influenza hemagglutinin (HA), glutathione S transferase (GST), substance P, poly-histidine tag (6xHis, 10xHis, etc.), protein C fragment, maltose-binding protein (MBP), immunoglobulin constant region fragment, α-tubulin fragment, β-galactosidase, B-tag, c-myc fragment, E-tag (epitope on a monoclonal phage), FLAG (Hopp et al. (1988) Bio/Tehcnol. 6: 1204-10), lck tag, p18 HIV fragment, HSV-tag (human simplex herpes virus glycoprotein), SV40T antigen fragment, T7-tag (T7 gene10 protein), and VSV-GP fragment (Vesicular stomatitis virus glycoprotein).

The PLAP of the present invention includes the polypeptides as mentioned above and, for example, a polypeptide comprising an amino acid sequence that is substantially the same as the amino acid sequence represented by SEQ ID No: 11 and having PLAP activity that is of substantially the same quality as that of the polypeptide consisting of the amino aid sequence represented by SEQ ID No: 11, and being secreted out of the cells is preferable. Here, the term "PLAP activity" refers to the enzymatic activity of alkaline phosphatase of PLAP. The activity that is of substantially the same quality means that the activity by its property (for example, physiologically and chemically, or pharmacologically) is of same quality. For example, when the PLAP activity per unit amount of a protein is, for example, 1% or more, preferably 3% or more, more preferably 10% or more, and still more preferably 30% or more as compared with the PLAP activity of the polypeptide consisting of the amino acid sequence represented by SEQ ID No: 11, it can be said that the polypeptide has an activity of substantially the same quality. A specific method of measuring the PLAP activity will be described below.

## 11. PLAP vector of the present invention

In the present invention, an expression vector for a secretory placenta-derived alkaline phosphatase (hereinafter, also referred to as "PLAP vector of the present invention") refers to a vector that can express the PLAP of the present invention, and is not particularly limited as long as it is a vector that can express the PLAP of the present invention.

The PLAP vector of the present invention is preferably one to which a polynucleotide comprising a transcription regulatory sequence and a polynucleotide coding for the PLAP of the present invention are ligated. In a more preferable aspect, an example of the PLAP vector includes an expression vector to which a polynucleotide coding for the PLAP of the present invention is linked downstream of the transcription regulatory sequence, that is, expression vector that comprises a transcription regulatory sequence and a polynucleotide coding for the PLAP of the present invention operably linked to the transcription regulatory sequence.

A polynucleotide coding for the PLAP of the present invention includes a polynucleotide comprising a nucleotide sequence that is the same or substantially the same as the nucleotide sequence represented by SEQ ID No: 10. The polynucleotide comprising substantially the same nucleotide sequence is not particularly limited as long as it comprises a nucleotide sequence coding for the PLAP of the present invention. For example, in addition to the polynucleotide coding for the amino acid sequence represented by SEQ ID No: 11 (that encompasses, besides the nucleotide sequence represented by SEQ ID No: 10, a nucleotide sequence other than the nucleotide sequence represented by SEQ ID No: 10 owing to degeneration of genetic code), polynucleotide coding for a mutant polypeptide consisting of an amino acid sequence represented by SEQ ID No: 11 in which one or plural amino acids have been deleted, inserted, substituted, or added and having a PLAP activity and being secreted out of the cell can be used in the present invention.

Examples of a polynucleotide comprising substantially the same nucleotide sequence as the nucleotide sequence represented by SEQ ID No: 10 include SEQ ID No: 12 and SEQ ID No: 14. Those polynucleotides are polynucleotides consisting of partial sequences of pSEAP and pSEAP2 (manufactured by Clontech Laboratories, Inc.) and can be purchased from Clontech Laboratories, Inc.

On the other hand, a polynucleotide coding for the PLAP of the present invention can be obtained from cDNA library or genomic library of animals such as human, mouse, rat, rabbit, hamster, chicken, pig, bovine, goat, and sheep, and preferably from cDNA library or genomic library of human by designing primers based on the nucleotide sequence of SEQ ID NO: 10 and using gene amplification technique (PCR) (Current Protocols in Molecular Biology, John Wiley & Sons (1987) Section 6.1-6.4).

Hereinafter, an example of a method of obtaining a polynucleotide coding for the PLAP of the present invention is described.

Vector plasmid M13tg131 (Kiney, et. al. (1983) 26(1): 91-99) can be cleaved with PvuII to obtain MCS sequence fragment. The MCS sequence fragment can be inserted into the PvuII site in a vector plasmid pUC18 (TOYOBO) by ligase reaction (TAKARA, Cat. 6022) to obtain a vector plasmid pUG131.

273-bp PLAP cDNA fragment 1 can be obtained by performing PCR using human placenta cDNA library as a template and oligo DNAs represented by SEQ ID No: 16 and SEQ ID No: 17 as primers. Then, 1028-bp PLAP cDNA fragment 2 can be obtained by an additional PCR using human placenta cDNA library as a template and oligo DNAs represented by SEQ ID No: 18 and SEQ ID No: 19 as primers. The nucleotide sequences of the primers are as follows.

Primer: CCAGAATTCCTGCCTCGCCACTGTCC (SEQ ID NO: 16)
Primer: TTAGGATCCTGGCAGCTGTCAC (SEQ ID NO: 17)
Primer: GTGACAGCTGCCAGGATCCTAA (SEQ ID NO: 18)
Primer: AGGACCGTGTAGGCCTCCCTGT (SEQ ID NO: 19)

After the PLAP cDNA fragments 1 and 2 are cleaved with Bam HI, those can be ligated by a ligase reaction (TAKARA, Cat. 6022) to obtain PLAP cDNA fragment 3. Then, the PLAP cDNA fragment 3 is cleaved with EcoRI and SmaI and inserted into pBlueScript KS (Stratagene), which has been cleaved with EcoRI and SmaI, by a ligase reaction (TAKARA, Cat. 6022). The obtained plasmid can be cleaved with HindIII and XmaI to obtain PLAP cDNA fragment 4.

To synthesize PLAP cDNA 3'-side fragment, the oligo DNAs represented by SEQ ID No: 20 and SEQ ID No: 21 can be annealed under an appropriate condition and then converted into a double strand DNA fragment using a DNA polymerase and inserted into the HincII site of pUG131 by a ligase reaction (TAKARA, Cat. 6022). Then, the plasmid can be cleaved with XmaI and AatII to obtain PLAP cDNA fragment 5. The nucleotide sequences of the oligo DNAs are as follows.

Oligo DNA:
AAGCCCGGGATCGTAAGGCCTACACAGTGCTACTGTATGGCAATGGCCCAGGGTAT
GTCCTAAAGGATGGAGCTAGACCAGATGTCACAGAGTCAGAG (SEQ ID NO: 20)

Oligo DNA:
AAAGACAGCGACGTCTTCCCCTGCGTGAGTCTCTTCATCTAACGGTACGGCCGATT
GCTGACGGTACTCTGGAGATCCAGACTCTGACTCTGTGACAT (SEQ ID NO: 21)

Similarly, after the oligo DNAs represented by SEQ ID No: 22 and SEQ ID No: 23 are annealed under an appropriate condition, those can be converted into a double strand DNA fragment using a DNA polymerase and inserted into the HincII site of pUG131 by a ligase reaction (TAKARA, Cat. 6022). Then, the plasmid can be cleaved with AatII and BglII to obtain PLAP cDNA fragment 6. The nucleotide sequences of the oligo DNAs are as follows.

Oligo DNA:

GGAAGACGTCGCTGTCTTTGCAAGAGGTCCCCAGGCACATCTCGTGCATGGCGTA CAGGAACAGACTTTCATCGCTCATGTAATGGCATTCGCAGCAT (SEQ ID NO: 22)

Oligo DNA:

TCCAGATCTGGGTTAACCTGGATGGGCAGCGTCTGTCGTACCTGCTGGTGGAGCTA AATCGCAAGCGGTATATGGCTCCAAACATGCTGCGAATGCCATT (SEQ ID NO: 23)

The PLAP cDNA fragments 5 and 6 can be inserted by a ligase reaction (TAKARA, Cat. 6022) into apUG131 that has been cleaved with XmaI and BglII in advance. The plasmid can be cleaved with XmaI and BglII to obtain PLAP cDNA fragment 7.

Subsequently, oligo DNAs represented by SEQ ID No: 24 and SEQ ID No: 25 can be synthesized and annealed to obtain an adapter DNA 1. The nucleotide sequences of the oligo DNAs are as follows.

Oligo DNA: AATTCAAGCTTACCATG (SEQ ID NO: 24)

Oligo DNA: GTAAGCTTG (SEQ ID NO: 25)

On the other hand, PLAP cDNA fragment 4 and PLAP cDNA fragment 7 can be inserted into the HindIII/BglII sites of pUG131 by a ligase reaction (TAKARA, Cat. 6022). The plasmid can be cleaved with EcoRI and SphI and the adapter DNA 1 can be inserted into the plasmid by a ligase reaction (TAKARA, Cat. 6022). By these operations, a polynucleotide coding for the PLAP of the present invention (SEQ ID No: 10) can be obtained.

The polynucleotide coding for an amino acid sequence represented by SEQ ID No: 11 in which one or plural amino acids have been deleted, inserted, substituted or added, which is encompassed in the polynucleotide coding for the PLAP of the present invention, can be prepared according to a method such as a site-directed mutagenesis method as described, for example, in "Molecular Cloning, A Laboratory Manual 2nd ed." (Cold Spring Harbor Press (1989)), "Current Protocols in Molecular Biology" (John Wiley & Sons (1987-1997); in particular, Section 8.1-8.5), Hashimoto-Goto et al. (1995) Gene 152: 271-5, Kunkel (1985) Proc. Natl. Acad. Sci. USA 82: 488-92, Kramer and Fritz (1987) Method. Enzymol. 154: 350-67, and Kunkel (1988) Method. Enzymol. 85: 2763-6.

Mutations can be introduced into a polynucleotide by means of a known technique such as the Kunkel method or the Gapped duplex method, using a kit for introducing mutation utilizing a site-directed mutagenesis method, for example, QuikChange™ Site-Directed Mutagenesis Kit (manufactured by Stratagene), GeneTailor™ Site-Directed Mutagenesis System (manufactured by Invitrogen), or TaKaRa Site-Directed Mutagenesis System (Mutan-K, Mutan-Super Express Km, etc.: manufactured by TaKaRa Bio).

Confirmation of the nucleic acid sequence of the polynucleotide coding for the PLAP of the present invention can be performed by sequencing by a conventional method. For example, the confirmation can be performed according to a dideoxynucleotide chain termination method (Sanger et al. (1977) Proc. Natl. Acad. Sci. USA 74: 5463) or the like. Also, it is possible to analyze the sequence by using an appropriate DNA sequencer.

The PLAP vector of the present invention can also be obtained by inserting a transcription regulatory sequence into pSEAP or pSEAP2 (manufactured by Clontech). The insertion of the transcription regulatory sequence can be performed according to a conventional method.

12. Cells into which the PLAP vector of the present invention has been transfected

Preparation of cells into which the PLAP vector of the present invention has been transfected (also referred to as "PLAP vector-transfected cells of the present invention" in the present description) can be performed by transfecting cells with the above-mentioned PLAP vector of the present invention according to a known method.

Hereinafter, a method of preparing the PLAP vector-transfected cells of the present invention is explained in detail.

The cells into which the PLAP vector of the present invention is transfected may be any cells as long as they can be transplanted into an animal, and the type thereof is not limited. Preferable examples of such cells include eukaryotic cells derived from mammals (for example, retina cells, liver cells, spleen cells, nerve cells, glia cells, pancreatic β cells, bone marrow cells, mesangium cells, Langerhans cells, epidermal cells, epithelial cells, endothelial cells, fibroblasts, fiber cells, muscle cells, adipose cells, immune cells (for example, macrophages, T cells, B cells, natural killer cells, mast cells, neutrophil leucocytes, basophil leucocytes, acidophil leucocytes, and monocytes), megakaryocytes, synoviocytes, cartilage cells, bone cells, osteoblasts, osteoclasts, mammary cells, liver cells or interstitial cells, or precursor cells thereof, stem cells, and immortalized cells or tumor cells), and particularly preferably immortalized cells or tumor cells.
Introduction of the PLAP vector of the present invention into host cells can be performed by an electroporation method (Chu et al. (1987) Nucleic Acids Res. 15: 1311-26), a cationic liposome method, a pulse electroporation method (Current Protocols in Molecular Biology, John Wiley & Sons (1987) Section 9.1-9.9), a direct injection method using a micro glass tube, a microinjection method, lipofection (Derijard (1994) Cell 7: 1025-37; Lamb (1993) Nature Genetics 5: 22-30; Rabindran et al. (1993) Science 259: 230-4), a lipofectamine method (GIBCO-BRL), a calcium phosphate method (Chen and Okayama (1987) Mol. Cell. Biol. 7: 2745-52), a DEAE dextran method (Lopata et al. (1984) Nucleic Acids Res. 12: 5707-17; Sussman and Milman (1985) Mol. Cell. Biol. 4: 1642-3), a FuGene6 reagent (Boehringer-Mannheim), or the like.
Culture of host cell can be performed by a known method suitable for the selected cell. For example, medium such as DMEM, MEM, RPMI1640, IMDM, and F12 can be used and serum such as fetal calf serum (FCS), amino acids, glucose, penicillin or streptomycin may be added thereto as necessary and culture can be performed at pH of about 6 to about 8 at 30 to 40°C for about 15 to about 200 hours. Medium may be exchanged, or aeration and agitation may be performed during culture as necessary.
Although the PLAP vector-transfected cells of the present invention can be used as they are, cloning can be preformed to avoid the deviation of the properties of the cells during culture, and/or enable to obtain a cell that expresses more PLAP of the present invention for a stable evaluation. The cloning of the cells can be performed by a conventional method (for example, a limiting dilution method, cell sorting by flow cytometry, or the like). The most suitable PLAP vector-transfected cell line of the present invention can be selected by measuring the PLAP activity and analysis of a copy number of PLAP mRNA by a molecular biological technique (for example, a quantitative RT-PCR method, Northern blotting, etc.).

13. Method of measuring an amount of PLAP of the present invention

The amount of the PLAP of the present invention contained in a sample containing the PLAP of the present invention can be measured by a known method. Here, although a sample containing the PLAP of the present invention may be any sample that contains the PLAP of the present invention without particular limitations, biological fluids, for example, blood, plasma, serum, urine, cerebrospinal fluid and so on, preferably blood, plasma and serum, particularly preferably plasma as described below can be used.
The method of measuring the amount of the PLAP of the present invention is not particularly limited and the measurement can be performed by means of immunological techniques (for example, ELISA, RIA, EIA, flow cytometry, and Western blotting), chromatography, mass spectrometry, enzymatic activity, or the like. Since it is preferable that the amount of the PLAP of the present invention is measured by means of enzymatic activity, the method of measuring the PLAP activity is described hereinafter. (1) Method of measuring the activity of PLAP of the present invention in vitro
The PLAP activity can be measured by cultured the PLAP vector-transfected cells of the present invention for a suitable period of time, mixing the culture supernatant with a substrate solution, incubating for a suitable period of time, and then measuring the intensity of chemiluminescence or using colorimetry, preferably by measuring the intensity of chemiluminescence.

Hereinafter, a more detailed description is made.

The PLAP vector-transfected cells of the present invention can be obtained and cultured as described above. When FCS is added to the culture medium, it is desirable to carry out heat treatment in order to inactivate the PLAP activity in the FCS. The heat treatment can be performed by heating at 50°C to 80°C, preferably 60°C to 70°C, and particularly preferably 64°C to 66°C, for 5 to 120 minutes, and preferably 20 minutes to 60 minutes.
Upon measuring the PLAP activity, it is desirable that the PLAP vector-transfected cells of the present invention which have been cultured in advance are seeded on a cell culture plate, and the plate is further incubated.
After cultured for a suitable period of time (for example, 2 to 96 hours), the culture supernatant is recovered. To inactivate the PLAP activity derived from the serum contained in the recovered culture supernatant, the culture supernatant can be heated at 50°C to 80°C, preferably 60°C to 70°C, and particularly preferably 64°C to 66°C, for 5 minutes to 120 minutes, preferably 20 minutes to 60 minutes.
Then, the culture supernatant is mixed with a substrate solution. A kind of the substrate solution is not limited as long as it contains a substance that serves as a substrate for PLAP and generates chemiluminescence by being reacted with PLAP. An example of the substrates that can be used includes 4-methoxy-4-(3-phosphatephenyl)spiro[1,2-dioxetane-3,2'-adamantane], disodium salt (for example, Lumi-Phos 530 (Lumigen, Inc.)). On the other hand, various buffer solution can be used as a solution that dissolves the substrate. For example, an aqueous solution containing 0.28M $Na_2CO_3$-$NaHCO_3$, 8mM $MgSO_4$, pH 10.0 can be used. The culture supernatant can be used after appropriate dilution and it is desirable that the culture supernatant is added to be about 10% (v/v) of the substrate solution.

The culture supernatant is mixed with the substrate solution, and incubated for a suitable period of time. The incubation can be performed at 5°C to38°C, preferably 15°C to 30°C, and particularly preferably 20°C to 25°C, for 10 minutes to 24 hours, and preferably 30 minutes to 4 hours. It is preferable to shield light during the incubation.
After the incubation, the intensity of chemiluminescence is measured. The intensity of chemiluminescence can be measured using a commercially available plate reader (for example, Perkin Elmer, ARVO). The value obtained as a result of the measurement can be defined as PLAP activity.

(2) Method of measuring the activity of PLAP of the present invention in vivo

The PLAP activity can be measured, in a nonhuman animal model that produces the PLAP of the present invention, obtained by transplanting the PLAP vector-transfected cells of the present invention into a nonhuman animal, by mixing a biological fluid with a substrate solution, incubating the mixture for a suitable period of time, and then measuring the intensity of chemiluminescence or using colorimetry, preferably by measuring the intensity of chemiluminescence.

Hereinafter, a more detailed description is made.

By the above-mentioned method, the PLAP vector-transfected cells of the present invention are transplanted into a nonhuman animal.
After the transplantation, the nonhuman animal is raised for a suitable period of time (for example, 1 hour to 1,095 days, preferably 2 hours to 365 days, and more preferably 24 hours to 180 days), and biological fluids are collected. The kind of the biological fluid is not limited as long as it is derived from the nonhuman animal and examples of the biological fluid include blood, plasma, serum, urine, and cerebrospinal fluid, preferably blood, plasma or serum, more preferably plasma or serum, and particularly preferably plasma.
The collected biological fluid may be fractionated into a fraction containing PLAP and a fraction containing no PLAP as necessary. In the case of blood, it is desirable that plasma is separated by centrifugation and used as a biological fluid. The separated biological fluid can be stored at a suitable temperature, preferably 4°C or lower, and particularly preferably -20°C or lower until the PLAP activity is measured.
It is desirable that the PLAP activity is measured while distinguishing it from the activity of alkaline phosphatase derived from the endogenous alkaline phosphatase in the nonhuman animal described below. Examples of a method of distinguishing the PLAP activity derived from the PLAP of the present invention from the alkaline phosphatase activity derived from the endogenous alkaline phosphatase include a method that involves treating under a condition under which the PLAP of the present invention is not decomposed and/or inactivated but the endogenous alkaline phosphatase is decomposed and/or inactivated, so the PLAP activity derived from the PLAP of the present invention can be measured while being distinguished from the alkaline phosphatase activity derived form the endogenous alkaline phosphatase. A condition under which the endogenous alkaline phosphatase is decomposed and/or inactivated includes heat treatment and so on.
For example, in order to inactivate the alkaline phosphatase activity derived from the nonhuman animal contained in the biological fluid, the biological fluid can be heated at 50°C to 80°C, preferably 60°C to 70°C, and particularly preferably 64°C to 66°C, for 5 to 120 minutes, and preferably 20 minutes to 60 minutes.
Then, the biological fluid is mixed with a substrate solution. The kind of the substrate solution is not limited as long as it contains a substance that serves as a substrate for PLAP and generates chemiluminescence by being reacted with the PLAP. An example of the substrates that can be used includes 4-methoxy-4-(3-phosphatephenyl)spiro[1,2-dioxetane-3,2'-adamantane], disodium salt (for example, Lumi-Phos 530 (Lumigen, Inc.)). On the other hand, various buffer solution can be used as a solution that dissolves the substrate. For example, an aqueous solution containing 0.28M $Na_2CO_3$-$NaHCO_3$, 8mM $MgSO_4$, pH 10.0 can be used. The biological fluid can be used after appropriate dilution and it is preferable that the culture supernatant is added to be about 1% to about 10% (v/v) of the substrate solution.
The biological fluid is mixed with a substrate solution, and is incubated for a suitable period of time. The incubation can be performed at 5°C to 38°C, preferably 15°C to 30°C, and particularly preferably 20°C to 25°C, for 10 minutes to 24 hours, and preferably 30 minutes to 4 hours. It is preferable to shield light during incubation.
After incubation, the intensity of chemiluminescence is measured. The intensity of chemiluminescence can be measured using a commercially available plate reader (for example, Perkin Elmer, ARVO). The value obtained as a result of the measurement can be defined as PLAP activity.

14. Method of measuring transcriptional activity in transplanted cells in a nonhuman animal model

In a nonhuman animal model that produces the PLAP of the present invention obtained by transplanting the PLAP vector-transfected cells of the present invention into a nonhuman animal, the PLAP activity in the biological fluid is measured and based on the PLAP activity, the transcriptional activity through a transcription regulatory sequence in the transplanted cells can be measured in the nonhuman animal model.

Hereinafter, a more detailed description is made.

The PLAP vector-transfected cells of the present invention can be obtained and cultured as described above. Also, the cells to be transplanted may be those cultured in vivo (Asano et al, Jpn J Cancer Res. 1999 Jan; 90(1):93-100).
The PLAP vector-transfected cells of the present invention are transplanted into a nonhuman animal. The nonhuman animal to be transplanted is not particularly limited and examples thereof include mouse, rat, guinea pig, hamster, rabbit, dog, monkey, chicken, pig, sheep, bovine, and cat. Preferable examples include mouse and rat, and particularly preferable example is mouse. Meanwhile, the transplantation site in nonhuman animal is not particularly limited. Examples thereof include subcutaneous, intraperitoneal, blood, and organs (for example, brain, each site of brain (for example, retina, olfactory bulb, amygdaloid nucleus, basal ganglion, hippocampus, thalamus, hypothalamus, brain cortex, medullary, and cerebellum, etc.), spinal cord, pituitary, stomach, pancreas, kidney, liver, genital glands, thyroid, gall bladder, bone marrow, adrenal, skin, muscle, lung, digestive tracts (for example, large intestine and small intestine), blood vessels, heart, thymus gland, spleen, mandibular gland, peripheral blood, prostate gland, testis, ovary, placenta, uterus, bone, joints, and skeletal muscle, etc.).
The number of cells to be transplanted is not particularly limited and is, for example, $10^2$ to $10^9$ cells/head, preferably $10^4$ to $10^8$ cells/head, more preferably $10^5$ to $10^7$ cells/head, and particularly preferably $5 \times 10^5$ to $5 \times 10^6$ cells/head.
Upon transplantation, the cells may be transplanted as they are or the cells may be transplanted along with a pharmaceutically acceptable carrier. Examples of the carrier include physiological saline, phosphate buffer, culture medium, serum, a biological fluid, and carboxymethylcellulose solution. Further, the cells may be transplanted in combination with solids that provide a anchorage for the cells (for example, cytodex3 (Amersham Bioscience, 17-0485-01), etc.), extracellular matrix components (for example, collagen, fibronectin, vitronectin, laminin, heparan sulfate, proteoglycan, glycosaminoglycan, chondroitin sulfate, hyaluron, or elastin or a combination of two or more of them) or gel-like supports.
Further, upon transplantation, the cells that contain angiogenesis factors (for example, vessel endothelial cell growth factor (VEGF), basic fibroblast growth factor (bFGF), acidic fibroblast growth factor (aFGF), platelet-derived growth factor (PDGF), transforming growth factor-β (TGF-β), angiopoietin, hepatocyte growth factor (HGF), etc.) may be transplanted.
After transplantation, the animal is raised for a suitable period of time (for example, 1 hour to 1,095 days), and biological fluids are collected. The kind of the biological fluid is not particularly limited as long as it is derived from the nonhuman animal and examples of the biological fluid include blood, plasma, serum, urine, and cerebrospinal fluid, and blood, plasma and serum are preferable, plasma and serum are preferable, and plasma is particularly preferable.
The collected biological fluid may be fractionated into a fraction containing the PLAP of the present invention and a fraction containing no PLAP of the present invention as necessary. In the case of blood, it is preferable that plasma is separated by centrifugation and used as a biological fluid. The separated biological fluid can be stored at a suitable temperature, preferably 4°C or lower, and particularly preferably -20°C or lower until the amount of the secretory protein is measured.
The PLAP activity in the biological fluid can be measured by the above-mentioned method of measuring the PLAP activity.
Here, the PLAP of the present invention is transcribed from the PLAP vector of the present invention, translated into the PLAP of the present invention, and secreted into the biological fluid. Therefore, the PLAP activity obtained by the above-mentioned method changes depending on the transcriptional activity of the PLAP vector of the present invention, so the PLAP activity is measured and based on the amount of the secretory protein, the transcriptional activity through the transcription regulatory sequence inserted into the PLAP vector of the present invention can be measured.

15. Method of screening a compound that affects transcriptional activity in a nonhuman animal model

A compound that affects transcriptional activity can be screened in a nonhuman animal model by administering a test compound to a nonhuman animal model that produces the PLAP of the present invention obtained by transplanting the PLAP vector-transfected cells of the present invention into a nonhuman animal, measuring the PLAP activity in the biological fluid, and selecting the compound that changes the PLAP activity.
Further, a compound that affects transcriptional activity in a nonhuman animal model can be screened by measuring the PLAP activity in the biological fluid in the nonhuman animal model that produces the PLAP of the present invention obtained by transplanting the PLAP vector-transfected cells of the present invention into the nonhuman animal that has been administered with the test compound and selecting the compound that changes the PLAP activity.

Hereinafter, a more detailed description is made.

In the method of screening a compound that affects the transcriptional activity in a nonhuman animal model, it is preferable that the PLAP vector of the present invention contains a polynucleotide comprising a transcription regulatory factor-binding sequence. The transcription regulatory factor-binding sequence is not particularly limited, and examples of the transcription regulatory factor-binding sequence that can be used include HRE, IL4RE, E2F-binding nucleotide sequence, estrogen receptor-binding nucleotide sequence, GATA-1-binding nucleotide sequence, AP1-binding nucleotide sequence, p53-binding nucleotide sequence and other transcription factor-binding sequences, and HRE and IL4RE are preferable.
Examples of HRE include a sequence represented by SEQ ID No: 1, a sequence represented by SEQ ID No: 2, a sequence represented by SEQ ID No: 3, a sequence represented by SEQ ID No: 4, and so on, and the sequence represented by SEQ ID No: 1 is preferable.
Examples of IL4RE include a sequence represented by SEQ ID No: 5, a sequence represented by SEQ ID No: 6, a sequence represented by SEQ ID No: 7, and a sequence represented by SEQ ID No: 8 (Martin Seidel, et al. (1995) Proc. Natl. Acad. Sci. USA, vol92:3041-3045), and the sequence represented by SEQ ID No: 8 is preferable.
A nonhuman animal model can be prepared by transplanting the PLAP vector-transfected cells of the present invention into a nonhuman animal model by the above-mentioned method.
A method of administering a compound to a nonhuman animal model is not particularly limited, and examples thereof include oral administration, intravenous administration, subcutaneous administration, intraperitoneal administration, intramuscular administration, and intracranial administration. On the other hand, a non-administered group, a vehicle-administered group, and so on can be used as a control group.
The amount of a compound to be administered to the nonhuman animal model is not particularly limited and is, for example, 1 ng/kg to 1 g/kg, preferably 100 ng/kg to 1 g/kg, more preferably 1 mg/kg to 1 g/kg, and particularly preferably 10 mg/kg to 300 mg/kg.
The timing of administering a compound to the nonhuman animal model is not particularly limited and is, for example, before transplanting the cells, at the time of transplanting the cells, after transplanting the cells, and so on.
The PLAP activity in the biological fluid can be measured by the above-mentioned method.
A compound that affects the transcriptional activity can be screened by comparing the PLAP activity in a biological fluid of the nonhuman animal model to which a compound has been administered, with the PLAP activity in the biological fluid of a control group. In the screening method, for example, when the PLAP activity in the biological fluid of the nonhuman animal model is increased or decreased 1.2 times or more, preferably 1.5 times or more, more preferably 2.0 times or more, and particularly preferably 3.0 times or more, as compared with the PLAP activity in the biological fluid of the control group, it is judged that the transcriptional activity has been affected.
In the present invention, examples of a test compound include peptides, proteins, antibodies, nonpeptidic compounds, synthetic compounds, polynucleotides, fermented products, cell extracts, plant extracts, animal tissue extracts, and plasma. Examples of polynucleotides include anti-sense polynucleotides, ribozyme nucleotides, and double strand RNAs. Here, a double strand RNA refers to a double strand RNA that causes RNA interference (Fire, et. al. (1998) Nature, 391:806-811).

16. Method of measuring the number of transplanted cells and/or tumor volume based on the amount of the PLAP activity in a nonhuman animal model

In the nonhuman animal model which produces the PLAP, obtained by transplanting the PLAP vector-transfected cells of the present invention into a nonhuman animal, the PLAP activity in the biological fluid is measured and based on the PLAP activity, the number of transplanted cells can be measured.
Further, by transplanting the PLAP vector-transfected cells of the present invention into a nonhuman animal model, a tumor is caused to develop in the nonhuman animal and the PLAP of the present invention can be produced in the tumor. In this case, the PLAP activity in the biological fluid is measured and based on the PLAP activity, the tumor volume in the nonhuman animal model can be measured.

Hereinafter, a more detailed description is made.

In the method of measuring the number of transplanted cells and/or tumor volume based on the PLAP activity in a nonhuman animal model, it is preferable that the PLAP vector of the present invention contains a polynucleotide consisting of a constitutive transcription regulatory sequence. Here, the constitutive transcription regulatory sequence refers to a sequence having a constitutive transcription initiation activity. The constitutive transcription regulatory sequence is not particularly limited as long as it is a sequence having a constitutive transcription initiation activity, and for example, SV40 promoter, CMV promoter, thymidine kinase promoter, Ubiquitin C promoter, Elongation factor 1 alpha (EF1a) promoter, β-actin promoter, Glyceraldehyde-3-phosphate dehydrogenase promoter, Phosphoglycerokinase promoter, β2-Microglobulin promoter, β-Glucuronidase promoter and so on can be used. An example of SV40 promoter includes a sequence represented by SEQ ID No: 9.
By the above-mentioned method, the PLAP vector-transfected cells of the present invention can be transplanted into a nonhuman animal model and the PLAP activity in the biological fluid can be measured.
Here, transcriptional activity by a constitutive transcription regulatory sequence is considered to show always a certain value, so the PLAP of the present invention is secreted into the biological fluid, in an amount depending on the number of the transplanted cells. Therefore, the PLAP activity obtained by the above-mentioned method changes depending on the number of the transplanted cells in the nonhuman animal model, so the PLAP activity is measured and based on the PLAP activity, the number of the transplanted cells can be measured.
Further, in the present invention, the term "number of transplanted cells" refers to the number of cells derived from the cells that are artificially transplanted into a nonhuman animal, and encompasses the number of cells after an increase or decrease of the transplanted cells in the nonhuman animal.
Therefore, comparison between the PLAP activity in the biological fluid before raising the animal and the PLAP activity in the biological fluid after raising the animal for a suitable period of time enables determination of an increase or decrease in the number of the transplanted cells in the nonhuman animal model. Further, in solid tumors, the cell number is considered to correlate with the tumor volume and hence the increase or decrease in the tumor volume can be determined when solid tumor is caused to develop in the nonhuman animal.

17. Method of screening a compound that affects number of transplanted cells and/or tumor volume based on PLAP activity as in a nonhuman animal model

A compound that affects the number of transplanted cells can be screened in a nonhuman animal model by administering a compound to a nonhuman animal model that produces the PLAP of the present invention obtained by transplanting the PLAP vector-transfected cells of the present invention into the nonhuman animal, measuring the PLAP activity in the biological fluid, and selecting the compound that changes the PLAP activity.
Also, a compound that affects the number of transplanted cells can be screened in a nonhuman animal model by measuring the PLAP activity in a biological fluid in a nonhuman animal model that produces the PLAP of the present invention obtained by transplanting the PLAP vector-transfected cells of the present invention into a nonhuman animal that has been administered with a compound, and selecting a compound that affects the number of the transplanted cells.
Further, transplanting the PLAP vector-transfected cells of the present invention into a nonhuman animal enables a tumor to develop in the nonhuman animal and the PLAP of the present invention to be produced in the tumor. In this case, a compound that affects the tumor volume in a nonhuman animal can be screened by administering a compound to the nonhuman animal model, measuring the PLAP activity in the biological fluid, and selecting a compound that changes the amount of the secretory protein.

Hereinafter, a more detailed description is made.

In the method of screening a compound that affects the number of transplanted cells and/or tumor volume based on the PLAP activity in a nonhuman animal model, it is preferable that the PLAP vector of the present invention contains a polynucleotide consisting of a constitutive transcription regulatory sequence. Here, the constitutive transcription regulatory sequence refers to a sequence having a constitutive transcription initiation activity. The constitutive transcription regulatory sequence is not particularly limited as long as it is a sequence having a constitutive transcription initiation activity and for example, SV40 promoter, CMV promoter, thymidine kinase promoter, Ubiquitin C promoter, Elongation factor 1 alpha (EF 1a) promoter, β-actin promoter, Glyceraldehyde-3-phosphate dehydrogenase promoter, Phosphoglycerokinase promoter, β2-Microglobulin promoter, β-Glucuronidase promoter, and so on can be used. An example of SV40 promoter includes a sequence represented by SEQ ID No: 9.
By the above-mentioned method, the PLAP vector-transfected cells of the present invention can be transplanted to prepare a nonhuman animal model.
The method of administering a compound to a nonhuman animal model is not particularly limited, and examples thereof include oral administration, intravenous administration, subcutaneous administration, intraperitoneal administration, intramuscular administration, and intracranial administration. A non-administered group, a vehicle-administered group, and so on can be used as a control group.
The amount of the compound to be administered to the nonhuman animal model is not particularly limited and is, for example, 1 ng/kg to 1 g/kg, preferably 100 ng/kg to 1 g/kg, more preferably 1 mg/kg to 1 g/kg, and particularly preferably 10 mg/kg to 300 mg/kg.
The timing of administering a compound to the nonhuman animal model is not particularly limited and is, for example, before transplanting the cells, at the time of transplanting the cells, after transplanting the cells, and so on.

The PLAP activity in the biological fluid can be measured by the above-mentioned method.

A compound that affects the number of the transplanted cells can be screened by comparing the PLAP activity in the biological fluid of the nonhuman animal model to which a compound has been administered, with the PLAP activity in the biological fluid of a control group. On the other hand, in solid tumors, the cell number is considered to correlate with the tumor volume, so the compound that affects the tumor volume can also be screened. In the screening method, for example, when the PLAP activity in the biological fluid of the nonhuman animal model to which the compound has been administered increases or decreases, for example, 1.2 times or more, preferably 1.5 times or more, more preferably 2.0 times or more, and particularly preferably 3.0 times or more, as compared with the PLAP activity in the biological fluid of the control group, it can be judged that an the number of transplanted cells and/or tumor volume has been affected.

18. Measuring kit

A measuring kit that contains a secretory placenta-derived alkaline phosphatase-expression vector is used in (1) a method of measuring transcriptional activity in transplanted cells in a nonhuman animal model (the above-mentioned "14. Method of measuring transcriptional activity in transplanted cells in a nonhuman animal model"), (2) a method of screening a compound that affects transcriptional activity in a nonhuman animal model (the above-mentioned "15. Method of screening a compound that affects transcriptional activity in a nonhuman animal model"), (3) a method of screening a compound that affects the number of transplanted cells and/or tumor volume based on the PLAP activity in a nonhuman animal model (the above-mentioned "16. Method of screening a compound that affects the number of transplanted cells and/or tumor volume based on the PLAP activity in a nonhuman animal model"), or (4) a method of screening a compound that affects the number of transplanted cells and/or tumor volume based on the PLAP activity in a nonhuman animal model (the above-mentioned "17. Method of screening a compound that affects number of transplanted cells and/or tumor volume based on PLAP activity in a nonhuman animal model"). The measuring kit has only to contain the PLAP vector of the present invention, and other components are not limited.
The measuring kit may contain, for example, a control vector, transfection reagent, cell culture broth, PLAP substrate solution, and buffer solution for measurement of the PLAP activity.

A measuring kit that contains secretory placenta-derived alkaline phosphatase-expression vector-transfected cells is used in (1) a method of measuring transcriptional activity in transplanted cells in a nonhuman animal model (the above-mentioned "14. Method of measuring transcriptional activity in transplanted cells in a nonhuman animal model"), (2) a method of screening a compound that affects transcriptional activity in a nonhuman animal model (the above-mentioned "15. Method of screening a compound that affects transcriptional activity in a nonhuman animal model"), (3) a method of screening a compound that affects the number of transplanted cells and/or tumor volume based on the PLAP activity in a nonhuman animal model (the above-mentioned "16. Method of screening a compound that affects the number of transplanted cells and/or tumor volume based on the PLAP activity in a nonhuman animal model"), or (4) a method of screening a compound that affects the number of transplanted cells and/or tumor volume based on the PLAP activity in a nonhuman animal model (the above-mentioned "17. Method of screening a compound that affects the number of transplanted cells and/or tumor volume based on PLAP activity in a nonhuman animal model"). The measuring kit has only to contain the PLAP vector-transfected cells of the present invention, and other components are not limited.
The measuring kit may contain, for example, control cells, cell culture broth, PLAP substrate solution, and buffer solution for measurement of the PLAP activity.

A measuring kit that contains a nonhuman animal into which placenta-derived alkaline phosphatase-expression vector-transfected cells are transplanted is used in (1) a method of measuring transcriptional activity in transplanted cells in a nonhuman animal model (the above-mentioned "14. Method of measuring transcriptional activity in transplanted cells in a nonhuman animal model"), (2) a method of screening a compound that affects transcriptional activity in a nonhuman animal model (the above-mentioned "15. Method of screening a compound that affects transcriptional activity in a nonhuman animal model"), (3) a method of screening a compound that affects the number of transplanted cells and/or tumor volume based on the PLAP activity in a nonhuman animal model (the above-mentioned "16. Method of screening a compound that affects the number of transplanted cells and/or tumor volume based on the PLAP activity in a nonhuman animal model"), or (4) a method of screening a compound that affects the number of transplanted cells and/or tumor volume based on the PLAP activity in a nonhuman animal model (the above-mentioned "17. Method of screening a compound that affects number of transplanted cells and/or tumor volume based on the PLAP activity in a nonhuman animal model"). The measuring kit has only to contain a nonhuman animal into which the PLAP vector-transfected cells of the present invention are transplanted, and other components are not limited.
The measuring kit may contain, for example, a control nonhuman animal, PLAP substrate solution, and buffer solution for measurement of the PLAP activity.

In the description and drawings, when nucleotides and amino acids are indicated by abbreviations, such abbreviations are those according to IUPAC-IUB Commission on Biochemical Nomenclature or commonly used abbreviations in this art. Examples thereof are described below. In the case where amino acids may have optical isomers, they are L-forms unless otherwise indicated explicitly.
DNA: deoxyribonucleic acid
cDNA: complementary deoxyribonucleic acid
A: adenine
T: thymine
G: guanine
C: cytosine
U: uracil
N: adenine (A), guanine (G), cytosine (C), or thymine (T)
RNA: ribonucleic acid
mRNA: messenger ribonucleic acid
dATP: deoxyadenosine triphosphate dTTP: deoxythymidine triphosphate
dGTP: deoxyguanosine triphosphate
dCTP: deoxycytidine triphosphate
Gly or G: glycine
Ala or A: alanine
Val or V: valine
Leu or L: leucine
Ile or I: isoleucine
Ser or S: serine
Thr or T: threonine
Cys or C: cysteine
Met or M: methionine
Glu or E: glutamic acid
Asp or D: asparaginic acid
Lys or K: lysine
Arg or R: arginine
His or H: histidine
Phe or F: phenylalanine
Tyr or Y: tyrosine
Trp or W: tryptophane
Pro or P: proline
Asn or N: asparagine
Gln or Q: glutamine

The SEQ ID NOS in the Sequence Listing described herein each represent the following sequences.

SEQ ID NO: 1 represents a nucleotide sequence of HRE.
SEQ ID NO: 2 represents a nucleotide sequence of HRE.
SEQ ID NO: 3 represents a nucleotide sequence of HRE.
SEQ ID NO: 4 represents a nucleotide sequence of HRE.
SEQ ID NO:5 represents a consensus nucleotide sequence of IL4RE.
SEQ ID NO: 6 represents a consensus nucleotide sequence of IL4RE.
SEQ ID NO: 7 represents a consensus nucleotide sequence of IL4RE.
SEQ ID NO: 8 represents a nucleotide sequence of IL4RE.
SEQ ID NO: 9 represents a nucleotide sequence of SV40.
SEQ ID NO: 10 represents a nucleotide sequence of a secretory placenta-derived alkaline phosphatase.
SEQ ID NO: 11 represents an amino acid sequence of a secretory placenta-derived alkaline phosphatase.
SEQ ID NO: 12 represents a nucleotide sequence of a secretory placenta-derived alkaline phosphatase.
SEQ ID NO: 13 represents an amino acid sequence of a secretory placenta-derived alkaline phosphatase.
SEQ ID NO: 14 represents a nucleotide sequence of a secretory placenta-derived alkaline phosphatase.
SEQ ID NO: 15 represents an amino acid sequence of a secretory placenta-derived alkaline phosphatase.
SEQ ID NO: 16 represents a nucleotide sequence of a primer for obtaining a polynucleotide of a secretory placenta-derived alkaline phosphatase.
SEQ ID NO: 17 represents a nucleotide sequence of a primer for obtaining a polynucleotide of a secretory placenta-derived alkaline phosphatase.
SEQ ID NO: 18 represents a nucleotide sequence of a primer for obtaining a polynucleotide of a secretory placenta-derived alkaline phosphatase.

SEQ ID NO: 19 represents an oligo DNA sequence for obtaining a polynucleotide of a secretory placenta-derived alkaline phosphatase.
SEQ ID NO: 20 represents an oligo DNA sequence for obtaining a polynucleotide of a secretory placenta-derived alkaline phosphatase.
SEQ ID NO: 21 represents an oligo DNA sequence for obtaining a nucleic acid of a secretory placenta-derived alkaline phosphatase.
SEQ ID NO: 22 represents an oligo DNA sequence for obtaining a polynucleotide of a secretory placenta-derived alkaline phosphatase.
SEQ ID NO: 23 represents an oligo DNA sequence for obtaining a polynucleotide of a secretory placenta-derived alkaline phosphatase.
SEQ ID NO: 24 represents an oligo DNA sequence for obtaining a polynucleotide of a secretory placenta-derived alkaline phosphatase.
SEQ ID NO: 25 represents an oligo DNA sequence for obtaining a polynucleotide of a secretory placenta-derived alkaline phosphatase.
SEQ ID NO: 26 represents a nucleotide sequence of a multi-cloning site (MCS).
SEQ ID NO: 27 represents a nucleotide sequence of a multi-cloning site (MCS).
SEQ ID NO: 28 represents a nucleotide sequence for producing HIF-Response element (HRE).
SEQ ID NO: 29 represents a nucleotide sequence for producing HIF-Response element (HRE).
SEQ ID NO: 30 represents a nucleotide sequence of a primer for obtaining a CMV promoter.
SEQ ID NO: 31 represents a nucleotide sequence of a primer for obtaining a CMV promoter.
SEQ ID NO: 32 represents a nucleotide sequence of a primer for obtaining a human H1 promoter.
SEQ ID NO: 33 represents a nucleotide sequence of a primer for obtaining a human H1 promoter.
SEQ ID NO: 34 represents a nucleotide sequence of a linker DNA.
SEQ ID NO: 35 represents a nucleotide sequence of a linker DNA.
SEQ ID NO: 36 represents a partial nucleotide sequence of HIF-1α mRNA.
SEQ ID NO: 37 represents a nucleotide sequence of an oligo DNA for producing HIF-1α dsRNA.
SEQ ID NO: 38 represents a nucleotide sequence of an oligo DNA for producing HIF-1α dsRNA.
SEQ ID NO: 39 represents a nucleotide sequence for producing RXR-binding sequence in the promoter region of CRBP2.
SEQ ID NO: 40 represents a nucleotide sequence for producing RXR-binding sequence in the promoter region of CRBP2.
SEQ ID NO: 41 represents a nucleotide sequence for producing IL4RE.
SEQ ID NO: 42 represents a nucleotide sequence for producing IL4RE.
SEQ ID NO: 43 represents the nucleotide sequence of a primer for obtaining a nucleic acid of STAT6.
SEQ ID NO: 44 represents the nucleotide sequence of a primer for obtaining a nucleic acid of STAT6.

EXAMPLES

Hereinafter, the present invention is shown by specific examples. However, the present invention is not limited thereto.

Example 1: Preparation of PLAP basic vector plasmid

TNF-α-promoter region was removed from TNF-α-PLAP vector plasmid (Goto, et. al. (1996) Molecular Pharmacology, 49:860-873) and instead a polynucleotide consisting of a multi-cloning site (MCS) was inserted thereto to obtain a PLAP basic vector plasmid. More specifically, oligo DNAs represented by SEQ ID No: 26 and SEQ ID No: 27 were prepared (entrusted to Japan Bio Service Co., Ltd.). Each of them was dissolved in TE buffer (10 mM Tris-HCL, pH 8.0, 1 mM EDTA) to be a concentration of 100 μM. 25 μl each of the 100 μM oligo DNA solution was mixed, and heated at 95°C for 10 minutes and then cooled at 37°C for 1 hour and at room temperature for 1 hour to anneal the oligo DNAs to obtain MCS oligo DNA. The oligo DNAs have the following nucleotide sequences.
Oligo DNA: CGAGCTCTTACGCGTGCTAGCCCGGGCTCGAGA (SEQ ID NO: 26)
Oligo DNA: AGCTTCTCGAGCCCGGGCTAGCACGCGTAAGAGCTCGGTAC (SEQ ID NO: 27)
Then, the oligo DNA was inserted by a ligase reaction (TAKARA BIO, Cat. 6022) into the TNF-α-PLAP vector plasmid which had been cleaved in advance with KpnI and HindIII. The resultant plasmid was transfected into E. *coli* by a conventional method to transform the E. *coli*. Plasmid was recovered from several E. *coli* transformants and the sequence was confirmed by ABI prism DNA sequencing kit (Applied Biosystems), thereby PLAP basic vector plasmid was obtained (Fig. 1).

Example 2: Preparation of HRE-FLAP reporter plasmid

It has been known that transcription regulatory factor-binding sequence HRE confers transcription promoting activity by low oxygen (Kimura, et. al. (2001) The Journal of Biological Chemistry, 276: 2292-2298). Three tandem repeats of the polynucleotide consisting of HRE were inserted to the KpnI site of the PLAP basic vector plasmid to obtain HREx3-PLAP. More specifically, by referring to the HRE sequence of the promoter sequence of a VEGF gene (Forsythe, et. al. (1996) Molecular and Cellular Biology, 16(9): 4604-4613), oligo DNAs each represented by SEQ ID No: 28 and SEQ ID No: 29 were prepared (entrusted to Japan Bio Service Co., Ltd.) and each of them was dissolved in TE buffer (10 mM Tris-HCl pH 8.0, 1 mM EDTA) to be a concentration of 100 μM. 25 μl each of the 100 μM oligo DNA solution was mixed, and heated at 95°C for 10 minutes and then cooled at 37°C for 1 hour and then at room temperature for 1 hour to anneal the oligo DNAs to obtain HRE oligo DNA. The oligo DNAs have the following nucleotide sequences.
Oligo DNA: CACAGTGCATACGTGGGCTCCAACAGGTCCTCTTCGTAC (SEQ ID NO: 28)
Oligo DNA: GAAGAGGACCTGTTGGAGCCCACGTATGCACTGTGGTAC (SEQ ID NO: 29)
Then, the PLAP basic vector was cleaved with KpnI, and HRE oligo DNA was inserted thereto by a ligase reaction (TAKARA, Cat. 6022). The obtained plasmid was transfected into *E. coli* by a conventional method to transform the *E. coli.* Plasmid was recovered from several *E. coli* transformants. A clone in which the 3'-side of SEQ ID No: 28 faces the HindIII site side of the PLAP basic vector plasmid was selected and named HREx1-PLAP vector plasmid. Further, the HREx1-PLAP vector plasmid was cleaved with KpnI, and HRE oligo DNA was inserted thereto by a ligase reaction, and in the same manner as described above, HREx2-PLAP vector plasmid was prepared. Further, the HREx2-PLAP vector plasmid was cleaved with KpnI, and HRE oligo DNA was inserted thereto by a ligase reaction, and in the same manner as described above, HREx3-PLAP vector plasmid was prepared. The sequence of the HREx3-PLAP vector plasmid was confirmed by ABI prism DNA sequencing kit (Applied Biosystems). As a result, it was observed that one HRE region out of the three HREs inserted in tandem lacked one nucleotide. That is, T, which is 6th bp from the 5'-side of SEQ ID No: 28, was deleted. Since this T is not an essential nucleotide for the activation of HRE (Kimura, et. al. (2001) The Journal of Biological Chemistry, 276: 2292-2298), the HREx3-PLAP vector plasmid was used in the following experiments.
Then, CMV promoter region of pCDNA3.1/Hygro (+) (Invitrogen) was amplified by PCR, and inserted to MluI/HindIII site of the HREx3-PLAP vector plasmid to obtain HRE-PLAP reporter plasmid. More specifically, by using pCDNA3.1/Hygro (+) as a template and oligo DNAs each represented by SEQ ID No: 30 and SEQ ID No: 31 (prepared by entrusting to Invitrogen) as primers, PCR was performed by using Expand High Fidelity PCR System (Roche Dioagnostic), by repeating 15 times a cycle of 94°C for 30 seconds, 60°C for 30 seconds, and 72°C for 60 seconds. The primers had the following nucleotide sequences.
Primer: GGCGGTACGCGTGTACGGTGGGAGGTC (SEQ ID NO: 30)
Primer: TACCAAGCTTAAGTTTAAACGC (SEQ ID NO: 31)
The DNA fragment obtained by the PCR was cleaved with MluI and HindIII and inserted by a ligase reaction (TAKARA, Cat. 6022) into the HREx3-PLAP vector plasmid which had been cleaved with MluI and HindIII. The obtained plasmid was transfected into *E. coli* by a conventional method to transform the E. *coli.* Plasmid was recovered from several E. *coli* transformants and the sequence was confirmed by ABI prism DNA sequencing kit (Applied Biosystems), thereby HRE-PLAP reporter plasmid was obtained (Fig. 2).

Example 3: Preparation of a cell into which HRE-PLAP reporter plasmid has been stably transfected

The HRE-PLAP reporter plasmid obtained in Example 2 was transfected into a human ovary cancer cell line SK-OV-3 and subjected to cloning to obtain a clone having a high induction ratio of PLAP expression by low oxygen.

Hereinafter, detailed description is made.

1. Introduction of HRE-PLAP reporter plasmid into cell

SK-OV-3 cells (American Type Culture Collection, HTB-77) subcultured in RPMI medium were recovered and suspended in RPMI medium to be a concentration of 25 x $10^4$ cells/ml. Here, the RPMI medium refers to a medium consisting of 500 ml of RPMI (SIGMA, R8758) which has been added with 5 ml of Penicillin/Streptomycin (Invitrogen, 15140-122), 5 ml of 100 mM Pyruvate (Invitrogen, 11360-070), 500 μl of 2-Mercaptoethanol (Invitrogen, 21985-023), and 50 ml of calf fetal serum (Sanko Jyunyaku Co., Ltd., No. 3308-502) which had been inactivated by heat treatment at 56°C for 20 minutes (hereinafter, the same holds true). Then, the cell suspension was inoculated at a volume of 2 ml/well on a 6-well cell culture plate (Becton Dickinson Labware, 35-3046) and cultured in a $CO_2$ incubator. On the next day, 100 μl of OPTI-MEM I (Invitrogen, 31985-062), to which 4 μl of Fugene 6 Transfection Reagent (Roche Diagnostics Corporation) and 2 μg of the HRE-PLAP reporter plasmid had been added, was incubated at room temperature for 15 minutes. Then, the suspension was added to the culture supernatant of the cells and cultured in a $CO_2$ incubator. On

the next day, the cells were removed from the wall of the vessel with trypsin-EDTA treatment and suspended in 5 ml of RPMI medium. Into a Petri dish for cell culture having a diameter of 10 cm (Becton Dickinson Labware, 35-3003), 11.2 ml of the RPMI medium and 0.8 ml of the cell suspension were added and the culture was continued in the $CO_2$ incubator. On the next day, G-418 (Geneticin : Invitrogen) was added to the culture supernatant to be a final concentration of 500 μg/ml and the culture was further continued in the $CO_2$ incubator. The RPMI medium containing 500 μg/ml G-418 was exchanged every three days. On day 11 after addition of G-418 and culture, colonies formed on the plate were removed from the wall of the vessel with trypsin-EDTA treatment and transferred to a 24-well cell culture plate (Becton Dickinson Labware, 35-3047) to perform cloning.

2. Confirmation of stable transfection of HRE-PLAP reporter plasmid

Culture in the $CO_2$ incubator was continued until the cloned cell became confluent. Then, the cells were removed from the wall of the vessel with trypsin-EDTA treatment and suspended in 500 μl of the RPMI medium for PLAP measurement. Here, the RPMI medium for PLAP measurement refers to a medium consisting of 500 ml of RPMI (SIGMA, R8758) which has been added with 5 ml of Penicillin Streptomycin (Invitrogen, 15140-122), 5 ml of 100 mM Pyruvate (Invitrogen, 11360-070), 500 μl of 2-Mercaptoethanol (Invitrogen, 21985-023), and 50 ml of calf fetal serum (Sanko Jyunyaku Co., Ltd., No. 3308-502) which had been inactivated by heat treatment at 65°C for 20 minutes (hereinafter, the same holds true). Then, the cell suspension was inoculated at a volume of 50 μl/well on two 384-well cell culture plates (Greiner, 781182) and cultured in a $CO_2$ incubator at a normal oxygen concentration (21 %). On the next day, one of the plates was transferred into a low oxygen incubator (TABAI ESPEC, BNP110M) in which the oxygen concentration was set to 2%, and cultured. The other plate was cultured in a $CO_2$ incubator at a normal oxygen concentration. On the next day, the PLAP activity in the culture supernatant was measured and used as an index for transcriptional activity. More specifically, equal amounts of Lumi-Phos 530 (Lumigen, Inc.) and PLAP buffer (0.28 M $Na_2CO_3$-$NaHCO_3$ pH 10.0, 8 mM $MgSO_4$) were mixed to form a substrate solution. Then, 100 μl of the substrate solution was added to a White Plate for ELISA (Sumitomo Bakelite Co., Ltd. No. MS-8496W) and 10 μl of the culture supernatant was added thereto, and mixed. Then, while shielding light, the mixture was incubated at room temperature for 1 hour, followed by measurement of intensity of chemiluminescence using a plate reader (Perkin Elmer, ARVO). This procedure was repeated for each clone, and clone A3 (Fig. 3) in which a higher PLAP activity is induced under a condition of a low oxygen concentration (2%) than a condition of a normal oxygen concentration (21 %) was obtained. Hereinafter, the clone A3 is described as SK-OV-3/HRE-PLAP(A3).

Example 4: Measurement of in vivo transcriptional activity of HRE-PLAP reporter plasmid-transfected cells (subcutaneous transplantation)

1. Transplantation of HRE-PLAP reporter plasmid-transfected cells in mouse and collection of blood

SK-OV-3/HRE-PLAP (A3) (Example 3) subcultured in RPMI medium containing 500 μg/ml G-418 was subjected to trypsin-EDTA treatment to be removed from the wall of the vessel and suspended in RPMI medium to be a concentration of $5 \times 10^7$ cells/ml. 100 μl of the cell suspension ($5\times10^6$ cells/mouse) was subcutaneously transplanted to a BALB/c nude mouse (female, 7 weeks, purchased from CLEA Japan, Inc.) by using a 1-ml syringe (with a 26G needle) for tuberculin. Orbital blood drawing was conducted using a heparin-coated hematocrit tube (manufactured by Drummond) on consecutive days including the day of transplantation. The hematocrit tube was closed on one end thereof with a putty for its exclusive use (manufactured by Terumo Corporation). The obtained blood was centrifuged at 10,000 rpm for 2 minutes using a centrifuge for hematocrit tubes (Tomy SEIKO Co., Ltd., RC-24BN) to separate a plasma fraction. Plasma was diluted 10-fold with physiological saline to prepare 10-fold diluted plasma, which was stored under refrigeration at -20°C until assay of its PLAP activity.
On the other hand, to examine the relationship between the size of tumor of the transplanted SK-OV 3/HRE-PLAP(A3) and PLAP activity in blood, the minor axis (mm) and major axis (mm) of the tumor was measured with an electronic digital micrometer caliper, and the tumor volume was obtained by the following equation.

$$\text{Tumor volume (mm}^3) = \text{Major axis (mm)} \times \text{minor axis (mm)} \times \text{minor axis (mm)}/2$$

2. Assay of PLAP activity in blood

After the 10-fold diluted plasma had been thawed at room temperature, it was treated under heating at 65°C for 1 hour in a dry heat sterilizer (Tokyo Rikakikai Co., Ltd., WF0-600SD) to inactivate the endogenous alkaline phosphatase. Then, the 10-fold diluted plasma after the heat treatment was diluted with PLAP buffer (0.28 M $Na_2CO_3$-$NaHCO_3$ pH 10.0, 8 mM $MgSO_4$) to prepare 100-fold diluted plasma.
On the other hand, equal amounts of Lumi-Phos 530 (Lumigen, Inc.) and the PLAP buffer (0.28 M $Na_2CO_3$-$NaHCO_3$ pH 10.0, 8 mM $MgSO_4$) were mixed to prepare a substrate solution. Then, 100 μl of the substrate solution was added to a White Plate for ELISA (Sumitomo Bakelite Co., Ltd. No. MS-8496W) and 10 μl of the 100-fold diluted plasma was added thereto and mixed. Subsequently, while shielding light, the mixture was incubated at room temperature for 1 hour, followed by measurement of intensity of chemiluminescence using a plate reader (Perkin Elmer, ARVO), and the result was used as an index for blood PLAP activity.
As a result, the PLAP activity in blood was 1,000 units or less before the transplantation of SK-OV-3/HRE-PLAP(A3), but it increased on the next day of the transplantation, once decreased, and increased again along with the proliferation of the tumor composed of SK-OV-3/HRE-PLAP(A3), thus showing a two-phase change (Fig. 4). It is considered that immediately after the transplantation, the tumor was at a low oxygen condition since no blood vessel was present in the tumor, so that the transcriptional activity through HRE was induced and the transcriptional activity of the HRE-PLAP reporter plasmid was enhanced, which resulted in an increase in blood PLAP activity. Further, it is considered that angiogenesis occurred and the oxygen concentration increased in the tumor, which inhibited the transcriptional activity through HRE to decrease the blood PLAP activity, and thereafter, the blood PLAP activity increased with an increasing number of tumor cells.
The above-mentioned results revealed that in a nonhuman animal model that produces secretory protein obtained by transplanting secretory protein-expression vector-transfected cells into a nonhuman animal, transcriptional activity through a transcription regulatory sequence in the transplanted cells in the nonhuman animal model can be determined by measuring the amount of the secretory protein and using the obtained amount of the secretory protein as an index.

Example 5: Preparation of dsRNA-expression vector plasmid

In transcription control through HRE, activation of transcription is known to be caused by binding of the transcription factor HIF-1 to HRE (Harris (2002) Nature Reviews Cancer, 2:38-47). The transcription factor HIF-1 is a heterodimer of HIF-1α and HIF-1β (Harris (2002) Nature Reviews Cancer, 2: 38-47). To confirm whether the blood PLAP activity observed in Example 4 was physiological one which is dependent on HIF-1, an HIF-1α-dsRNA-expression vector plasmid was prepared.
Here, dsRNA refers to a double strand RNA, which causes RNA interference (Fire, et. al. (1998) Nature, 391:806-811, hereinafter also referred to as RNAi).
Hereinafter, detailed explanation is made.

1. Cloning of human H1 promoter from a human genomic DNA

Using a human genomic DNA (Roche Diagnostics Corporation) as a template, and oligo DNAs each represented by SEQ ID No: 32 and SEQ ID No: 33 (prepared by entrusted to Invitrogen) as primers, PCR was performed by using Pfu polymerase (Promega) by repeating 35 times a cycle of 95°C for 30 seconds, 60°C for 30 seconds, and 72°C for 3 minutes to amplify the DNA fragment consisting of a human H1 promoter.
Primer: ACAGAATTCGAACGCTGACGTCATCA (SEQ ID NO: 32)
Primer: GAAAGCTTGGTAGATCTGTGGTCTCATACAGAACTTATAAGAT (SEQ ID NO: 33)
The DNA fragment was cleaved with EcoRI and HindIII and inserted by a ligase reaction into pUG18 (Toyobo Corporation) that had been cleaved with EcoRI and HindIII in advance. The obtained plasmid was transfected into *E. coli* by a conventional method to transform the E. *coli.* Plasmid was recovered from several *E. coli* transformants and the sequence was confirmed by ABI prism DNA sequencing kit (Applied Biosystems), thereby pUC18/H1 promoter was obtained.

2. Preparation of dsRNA-expression vector plasmid

Oligo DNAs each represented by SEQ ID No: 34 and SEQ ID No: 35 were prepared (entrusted to Invitrogen) and each of them was dissolved in TE buffer (10 mM Tris-HCl pH 8.0, 1mM EDTA) to be a concentration of 100 μM. 25 μl each of the 100 μM oligo DNA solution was mixed and heated at 90°C for 2 minutes, and cooled at 37°C for 1 hour and then at room temperature for 1 hour to anneal the oligo DNAs to obtain a linker DNA fragment having XbaI and EcoRI sites on respective terminals. The oligo DNAs have the following nucleotide sequences.
Oligo DNA: CTAGAGGTACCAGCTGCTAGCG (SEQ ID NO: 34)
Oligo DNA: AATTCGCTAGCAGCTGGTACCT (SEQ ID NO: 35)
The pUC18/H1 promoter was cleaved with EcoRI and HindIII to obtain an H1 promoter fragment. The H1 promoter fragment and the linker DNA fragment were inserted by a ligase reaction into pREP7 (Invitrogen) that had been cleaved with XbaI and HindIII in advance. The obtained plasmid was transfected into *E. coli* by a conventional method to transform the *E. coli.* Plasmid was recovered from several *E. coli* transformants and the sequence was confirmed by ABI prism DNA sequencing kit (Applied Biosystems), thereby pREP/H1 promoter was obtained (Fig. 5).

Example 6: Preparation of HIF-1α dsRNA-expression vector plasmid

Oligo DNAs each represented by SEQ ID No: 37 and SEQ ID No: 38 were prepared on the basis of the partial sequence of HIF-1α mRNA represented by SEQ ID No: 36 (entrusted to Invitrogen), and each of them was dissolved in TE buffer (10 mM Tris-HCl pH 8.0, 1mM EDTA) to be a concentration of 100 μM. 25 μl each of the 100 μM oligo DNA solution was mixed and heated at 90°C for 2 minutes, and cooled at 37°C for 1 hour and then at room temperature for 1 hour to anneal the oligo DNAs, thereby a DNA fragment for HIF-1α dsRNA expression was obtained. The RNA and oligo DNAs have the following nucleotide sequences.
mRNA sequence: GAUAAGUUCUGAACGUCGA (SEQ ID NO: 36)

Oligo DNA:

GATCCCCGATAAGTTCTGAACGTCGATTCAAGAGATCGACGTTCAGAACTTATCTT

TTTGGAAA (SEQ ID NO: 37)

Oligo DNA:

AGCTTTTCCAAAAAGATAAGTTCTGAACGTCGATCTCTTGAATCGACGTTCAGAAC

TTATCGGG (SEQ ID NO: 38)

The pREP-H 1 obtained in Example 5 was cleaved with Bg1 II and HindIII, and the DNA fragment for HIF-1α dsRNA expression was inserted by a ligase reaction (TAKARA, Cat. 6022). The obtained plasmid was transfected into E. *coli* by a conventional method to transform the *E. coli.* Plasmid was recovered from several *E. coli* transformants and the sequence was confirmed by ABI prism DNA sequencing kit (Applied Biosystems), thereby an HIF-1α dsRNA-expression vector plasmid pREP-H1-HIF1RNAi was obtained (Fig. 6).

Example 7: Preparation of cells into which HIF-1α dsRNA-expression vector plasmid has been stably transfected

The HRE-PLAP reporter plasmid-stably transfected cells SK-OV-3/HRE-PLAP(A3) obtained in Example 3 were transfected with the HIF-1α dsRNA-expression vector plasmid pREP-H1-HIF1RNAi obtained in Example 6, and stably-transfected cells were cloned.
Hereinafter, detailed description is made.

1. Introduction of pREP-H1-HIF1RNAi into SK-OV-3/HRE-PLAP (A3)

SK-OV-3/HRE-PLAP (A3) subcultured in RPMI medium containing 500 μg/ml G-418 was recovered and suspended in the RPMI medium to be a concentration of $2.5 \times 10^5$ cells/ml. Then, each of the cell suspension was inoculated at a volume of 2 ml/well on a 6-well cell culture plate (Becton Dickinson Labware, 35-3046) and cultured in a $CO_2$ incubator. On the next day (Day 2), 100 μl of OPTI-MEM I (Invitrogen, 31985-062), to which 4 μl of Fugene 6 Transfection Reagent (Roche Diagnostics Corporation) and 2 μg of pREP-H1-HIF1RNAi had been added, was incubated at room temperature for 15 minutes, and added to the culture supernatant of the cells and the mixture was cultured in a $CO_2$ incubator. On Day 4, the transfected cells were subjected to trypsin-EDTA treatment and the recovered cells were suspended in 5 ml of the RPMI medium. In a Petri dish for cell culture having a diameter of 10 cm (Becton Dickinson Labware, 35-3003), 11.5 ml of the RPMI medium and 0.5 ml of the cell suspension were added and the cultivation was continued in the $CO_2$ incubator. On Day 7, Hygromycin B (Invitrogen) was added to the RPMI culture supernatant to be a final concentration of 200 μg/ml and the cultivation was further continued in the $CO_2$ incubator. The RPMI medium containing 200 μg/ml Hygromycin B was exchanged every three days. On Day 21 and subsequently, colonies formed on the plate were removed from the wall of the vessel with trypsin-EDTA treatment and transferred to a 24-well cell culture plate (Becton Dickinson Labware, 35-3047) to perform cloning.

2. Confirmation of stable introduction of pREP-H1-HIF1RNAi

Cultivation in the $CO_2$ incubator was continued until each of the isolated clones grew sufficiently in amount and became confluent, and then a portion of the cells was inoculated again on a 96-well cell culture plate (Coming, 3628) and cultured at low oxygen of 2% for one night, and the PLAP activity in the culture supernatant was measured. For each clone, this procedure was repeated to obtain clone 2D4 that had low PLAP activity at low oxygen of 2% (Fig. 7). On the other hand, in the same manner as described above, SK-OV-3/HRE-PLAP(A3) was transfected with pREP-H1 and subjected to cloning to obtain clone MD2 (Fig. 7).

To examine whether HIF-1α mRNA was decreased due to the RNAi effect in each of the clones obtained in the section described above, total RNA was prepared from respective clones by using an RNA extraction kit RNeasy Mini Kit (Qiagen), and real-time PCR analysis was performed to quantitate the amounts of HIF-1α mRNA. More specifically, 450 ng of RNA was reverse-transcribed into cDNA with Random Hexamers primer in 50 μl of a reaction solution by using TaqMan Reverse Transcription Reagents (Applied Biosystems, N808-0234). After completion of the reaction, the reaction solution was diluted 3-fold with distilled water to prepare a cDNA solution. 12.5 μl of TaqMan Universal PCR Master Mix (Applied Biosystems, 4304437), 1.25 μl of 20X Assay-on-Demand™ Gene Expression Assay Mix for HIF-1α (Applied Biosystems, 4331182, Hs00153153_ml) or Assay Mix for β-actin (Applied Biosystems, 4310881E), and 1.25 μl of distilled water were added to 10 μl of the cDNA solution, and real-time PCR was performed by using ABI PRISM 7900HT Sequence Detection System (Applied Biosystems). The results were analyzed by a Comparative Ct method (Applied Biosystems Prism 7700 Users Bulletin No.2) to quantitate the amounts of HIF-1α mRNA and β-actin mRNA. The obtained amount of HIF-1α mRNA was divided by the amount of β-actin mRNA to make a correction.
As a result, it was confirmed that in the clone 2D4 transfected with pREP-H1-HIF1RNAi, 95% or more of HIF-1α mRNA expression was suppressed as compared with that of the clone MD2 which was transfected with pREP-H1, a vector containing no RNAi-causing sequence (Fig. 8). Accordingly, the clone 2D4 was used in subsequent experiments.

Example 8: Influence of suppression of HIF-1α by RNAi on in vivo transcriptional activity in HRE-PLAP reporter plasmid-transfected cells

Each of the clones 2D4 and MD2 (Example 7) subcultured in RPMI medium containing 500 μg/ml G-418 and 200 μg/ml Hygromycin B was subjected to trypsin-EDTA treatment to be removed from the wall of the vessel and then suspended in the RPMI medium to be a concentration of $5 \times 10^7$ cells/ml. 200 μl each of the cell suspension ($2 \times 10^6$ cells/mouse) was subcutaneously transplanted to a BALB/c nude mouse CD-1 nude mouse (female, 9 weeks, purchased from Charles River Japan Inc.) by using a 1-ml syringe (with a 26G needle) for tuberculin. Orbital blood drawing was conducted using a heparin-coated hematocrit tube (manufactured by Drummond) on consecutive days including the day of transplantation. The hematocrit tube was closed on one end thereof with a putty for its exclusive use (manufactured by Terumo Corporation). The obtained blood was centrifuged at 10,000 rpm for 2 minutes using a centrifuge for hematocrit tubes to separate a plasma fraction. Plasma was diluted 10-fold with physiological saline to prepare 10-fold diluted plasma, which was stored under refrigeration at -20°C until assay of its PLAP activity. Measurement of tumor volume of clone 2D4 and of clone MD2 and assay of PLAP activity in blood were performed by the method described in Example 4.
As a result, the control clone MD2 showed an increase in blood PLAP activity immediately after the transplantation, while the clone 2D4 in which expression of HIF-1α was suppressed by RNAi showed a suppressed increase in blood PLAP activity immediately after the transplantation (Fig. 9). Therefore, it revealed that the increase in blood PLAP activity due to HRE-PLAP-reporter plasmid observed in Example 4 was dependent on HIF-1. Accordingly, it is evident that the PLAP activity reflects the transcriptional activity through the transcription regulatory sequence and thus the transcriptional activity of the transcription regulatory sequence can be determined by measuring the PLAP activity.

Example 9: Influence of administration of anti-VEGF antibody on in vivo transcriptional activity in HRE-PLAP reporter plasmid-transfected cells

To confirm that the transcriptional activity in transplanted cells can be determined by measuring blood PLAP activity in a nonhuman animal model, the following experiments were performed.
It was reported that, under oxygen concentration of 0.5% or more, the amount of HIF-1 increases as the oxygen concentration decreases (Jiang, et. al. (1996) Am. J. Physiol, 271:C1172-C1180). On the other hand, it is considered that administration of an anti-VEGF antibody could inhibit angiogenesis, leading to lower oxygen concentration in the tumor (Blagosklonny (2004) Cancer Cell, 5:13-17). Then, to examine whether induction of HRE-PLAP transcriptional activity due to lowering of oxygen concentration in the tumor can be determined based on the blood PLAP activity, an anti-VEGF antibody was administered to a nude mouse to which HRE-PLAP reporter plasmid-transfected cells had been subcutaneously transplanted and time-dependent change of blood PLAP activity was measured.

More specifically, SK-OV-3/HRE-PLAP (A3) subcultured in RPMI medium containing 500 μg/ml G-418 (Ex-

ample 3) was subjected to trypsin-EDTA treatment to be removed from the wall of the vessel and suspended in the RPMI medium to be a concentration of $5\times10^7$ cells/ml. Then, 100 μl of the cell suspension ($5\times10^6$ cells/mouse) was subcutaneously transplanted to a BALB/c nude mouse (female, 7 weeks, purchased from CLEA Japan, Inc.) by using a 1-ml syringe (with a 26G needle) for tuberculin. 100 μg/head of an anti-VEGF antibody (R&D, MAb293) was intravenously administered to the mouse every 4 days after 10 days from the transplantation. From the day of the administration, orbital blood drawing was conducted using a heparin-coated hematocrit tube (manufactured by Drummond). The hematocrit tube was closed on one end thereof with a putty for its exclusive use (manufactured by Terumo Corporation). The obtained blood was centrifuged at 10,000 rpm for 2 minutes using a centrifuge for hematocrit tubes to separate a plasma fraction. Plasma was diluted 10-fold with physiological saline to prepare 10-fold diluted plasma, which was stored under refrigeration at -20°C until assay of its PLAP activity. Measurement of the volume of the tumor composed of SK-OV-3/HRE-PLAP(A3) and assay of the plasma PLAP antibody were performed by the methods described in Example 4.

As a result, while there was no substantial difference in the tumor volume between the anti-VEGF antibody-administered group (5 animals) and the control group (5 animals), the blood PLAP activity of the anti-VEGF antibody-administered group was higher than that of the control group (Fig. 10). It was considered that administration of the anti-VEGF antibody inhibits angiogenesis to decrease the oxygen concentration in the tumor, and an increase in the HRE-PLAP activity in response to the decrease of the oxygen concentration in the tumor increases the blood PLAP activity. Therefore, it is considered that in the nonhuman animal model, the oxygen concentration in the cells can be monitored by monitoring the blood PLAP activity.

Also, the above-mentioned results revealed that in a nonhuman animal model that produces secretory protein obtained by transplanting secretory protein-expression vector-transfected cells into a nonhuman animal, transcriptional activity through a transcription regulatory sequence in transplanted cells in the nonhuman animal model can be determined by measuring the amount of the secretory protein in a biological fluid and using the obtained amount of the secretory protein as an index.

Example 10: In vivo measurement of transcriptional activity in HRE-PLAP reporter plasmid-transfected cells (intraperitoneal transplantation)

SK-OV-3/HRE-PLAP (A3) subcultured in RPMI medium containing 500 μg/ml G-418 (Example 3) was subjected to trypsin-EDTA treatment to be removed from the wall of the vessel and suspended in the RPMI medium to be $1\times10^7$ cells/ml. 200 μl of the cell suspension ($2\times10^6$ cells/mouse) was intraperitoneally transplanted into a BALB/c nude mouse (female, 7 weeks, purchased from Charles River Japan Inc.) by using a 1-ml syringe (with a 26G needle) for tuberculin. Orbital blood drawing was conducted using a heparin-coated hematocrit tube (manufactured by Drummond) on consecutive days including the day of transplantation. The hematocrit tube was closed on one end thereof with a putty for its exclusive use (manufactured by Terumo Corporation). The obtained blood was centrifuged at 10,000 rpm for 2 minutes using a centrifuge for hematocrit tubes to separate a plasma fraction. Plasma was diluted 10-fold with physiological saline to prepare 10-fold diluted plasma, which was stored under refrigeration at -20°C until assay of its PLAP activity. Measurement of the volume of the tumor composed of SK-OV-3/HRE-PLAP (A3) and assay of the blood PLAP activity were performed by the method described in Example 4.

As a result, the blood PLAP activity was 1,000 units or less before the transplantation as in the subcutaneous transplantation experiment (Example 4), but it increased on the next day of the transplantation, once decreased, and increased again, thus showing a two-phase change (Fig. 11). Two animals out of the five animals showed a delayed increase in PLAP, which is considered to be due to the survival rate of the tumor.

Also, the above-mentioned results revealed that in a nonhuman animal model that produces a secretory protein obtained by transplanting secretory protein-expression vector -transfected cells into a nonhuman animal, transcriptional activity through a transcription regulatory sequence in the transplanted cells in the nonhuman animal model can be determined by measuring the amount of the secretory protein and using the obtained amount of the secretory protein as an index. Further, the results indicate that the site of the transplantation may not be only subcutaneous but also intraperitoneal.

Example 11: In vivo measurement of transcriptional activity in HRE-PLAP reporter plasmid-transfected cells (intracranial transplantation)

U251/VEGF-PLAP (Mizui, et. al. (2004) The Journal of antibiotics, 57: 188-196) obtained by stably introducing a VEGF-PLAP reporter plasmid (Fig. 12), which had been obtained by inserting a polynucleotide consisting of a promoter sequence of a VEGF gene into the KpnI/Nhe I site of a PLAP basic vector plasmid, into a human brain cancer line U-251 (Riken Cell Bank, RCB0461) was transplanted into the cranium of a mouse, and blood PLAP activity was measured. More specifically, U251NEGF-PLAP subcultured in RPMI medium containing 500 μg/ml G-418 was subjected to trypsin-EDTA treatment to be removed from the wall of the vessel and suspended in 2% methylcellulose to be a concentration of $1\times10^7$ cells/ml. 10 μl of the cell suspension ($1\times10^5$ cells/mouse) was subcranially transplanted to a BALB/c nude mouse (female, 12 weeks, purchased from Charles River Japan Inc., anesthetized by intraperitoneal administration of 0.1 ml (70 μl/head) of a solution of 10 mg/ml ketamine, 13.4 mg/ml xylazine, and 6.4 μg/ml acepromadine) by using a 25-μl Hamilton syringe. Orbital blood drawing was conducted using a heparin-coated hematocrit tube (manufactured by Drummond) on consecutive days including the day of transplantation. The hematocrit tube was closed on one end thereof with a putty for its exclusive use (manufactured by Terumo Corporation). The obtained blood was centrifuged at 10,000 rpm for 2 minutes using a centrifuge for hematocrit tubes to separate a plasma fraction. Plasma was diluted 10-fold with physiological saline to prepare 10-fold diluted plasma, which was stored under refrigeration at -20°C until assay of its PLAP activity. Measurement of the tumor volumes of the tumor composed of U251/VEGF-PLAP and assay of the blood PLAP activity were performed by the method described in Example 4.
As a result, the blood PLAP activity, as in the case where HRE-PLAP reporter plasmid-transfected SK-OV-3 was subcutaneously transplanted (Example 4), was 1,000 units or less before the transplantation, but it increased on the next day of the transplantation, once decreased, and increased again, thus showing a two-phase change (Fig. 13).

Also, the above-mentioned results revealed that, in a nonhuman animal model that produces a secretory protein obtained by transplanting secretory protein-expression vector-transfected cells into a nonhuman animal, transcriptional activity through a transcription regulatory sequence in the transplanted cells in the nonhuman animal model can be determined by measuring the amount of the secretory protein in a biological fluid and using the obtained amount of the secretory protein as an index.
Further, the results indicate that the site of the transplantation may not be only subcutaneous but also intracranial.
Still further, the results indicate that the transcription regulatory sequence is not limited to HRE.

Next, to confirm that the transcriptional activity in transplanted cells can be measured also in a promoter having a transcription regulatory factor-binding sequence different from HRE, the following experiments were performed.
IL-4 is known to activate STAT6 through the cell surface IL-4R to cause formation of a STAT6 homodimer (Jinzhao Hou, et. al. (1994), Science, vol. 265 (16):1701-1706). Further, STAT6 is known to bind to an enhancer IL4RE after formation of the homodimer (Richard Moriggl, et al. (1997) Molecular and Cellular Biology, vol. 17:3663-3678) and to regulate the transcriptional activity (Helen Kotanides, et al. (1996), The Journal of Biological Chemistry, vol. 271(41):25555-25561). Then, to confirm that the transcriptional activity in the transplanted cells can be determined by measuring blood PLAP activity in a nonhuman animal model, the following experiments were performed.

Example 12: Preparation of STAT6-PLAP reporter plasmid

HIV-1 kB-PLAP vector plasmid (MOLECULAR PHARMACOLOGY, 49:860-873 (1996)) was digested with restriction enzymes SpeI and XbaI. Then, the obtained fragment was dephosphorylated with alkaline phosphatase (TaKaRa, 2120B), and then electrophoresed on agarose gel. After the electrophoresis, about 5.5 kbp band was excised from the agarose gel and the DNA fragment was extracted using "SUPREC-01 " (TaKaRa, 9040). This was named TK-PLAP vector fragment.

Then, a DNA region to which RXR binds in the promoter region of Cellular Retinoid Binding Protein 2 (hereinafter, also referred to as CRBP2) was inserted into the above-mentioned TK-PLAP vector fragment. More specifically, after oligo DNAs each represented by SEQ ID No: 39 and SEQ ID No: 40 (entrusted to Japan Bio Service Co., Ltd.) were treated with T4 polynucleotide kinase (TOYOBO, PNK-103), NaCl was added thereto to be 0.1M NaCl and left to stand at 65°C for 10 minutes. After that, the mixture was cooled to room temperature to allow annealing. Then, the oligo DNA was inserted into the TK-PLAP vector fragment by a ligase reaction (TAKARA BIO, Cat. 6022). The plasmid was transfected into *E. coli* by a conventional method to transform the *E. coli.* Plasmid was recovered from several *E. coli* transformants and the sequence was confirmed by ABI prism DNA sequencing kit (Applied Biosystems), thereby pCRBP2x2-tkPLAP vector was obtained (Fig. 14).
Oligo DNA: CTAGTCAGGTCACAGGTCACAGGTCACAGTTCAAT (SEQ ID NO: 39)
Oligo DNA: CTAGATTGAACTGTGACCTGTGACCTGTGACCTGA (SEQ ID NO: 40)
Then, the CRBP2x2-TK promoter region was excised from the pCRBP2x2-tkPLAP vector. More specifically, the pCRBP2x2-TtkPLAP vector was digested with restriction enzymes SpeI and HindIII and electrophoresed on agarose gel. After the electrophoresis, an about 200 bp band was excised from the agarose gel and the DNA fragment was extracted using "SUPREC-01" (TaKaRa, 9040). This was named CRBP2x2-TK promoter fragment. Then, the CRBP2x2-TK promoter fragment was inserted into the PLAP basic vector which had been digested with restriction enzymes Nhe I and HindIII, by a ligase reaction (TAKARA BIO, Cat. 6022). The plasmid was transfected into *E. coli* by a conventional method to transform the *E. coli.* Plasmid was recovered from several *E. coli* transformants and the sequence was confirmed by ABI prism DNA sequencing kit (Applied Biosystems), thereby a pCRBP2x2-TK promoter-PLAP basic vector was obtained (Fig. 15).

Subsequently, the pCRBP2x2 sequence was removed from the pCRBP2x2-TK promoter-PLAP basic vector and instead the oligo DNA of IL4RE was inserted thereto to obtain a STAT6-PLAP reporter plasmid. More specifically, oligo DNAs each represented by SEQ ID No: 41 and SEQ ID No: 42 were prepared (entrusted to Pharmacia biotech) and each of them was dissolved in TE buffer (10 mM Tris-HCl pH 8.0, 1 mM EDTA) to be a concentration of 100 μM. 25 μl each of the 100 μM oligo DNA solution was mixed, and heated at 95°C for 10 minutes and cooled at 37°C for 1 hour and then at room temperature for 1 hour to anneal the oligo DNAs, and thereby IL4RE oligo DNA was obtained. The oligo DNAs have the following nucleotide sequences.

Oligo DNA:

AGCGGTACCTCGACTTCCCAAGAACAGAATCGACTTCCCAAGAACAGAATCGACT TCCCAAGAACAGAATCTAGAGCT (SEQ ID NO: 41)

Oligo DNA:

AGCTCTAGATTCTGTTCTTGGGAAGTCGATTCTGTTCTTGGGAAGTCGATTCTGTTC TTGGGAAGTCGAGGTACCGCT (SEQ ID NO: 42)

The IL4RE oligo DNA was digested with restriction enzymes KpnI and XbaI.
Then, the pCRBP2x2-TK promoter-PLAP basic vector was digested with restriction enzymes KpnI and XbaI and electrophoresed on an agarose gel. After the electrophoresis, an about 6.9 kbp band was excised from the agarose gel and the DNA fragment was extracted using "SUPREC-01" (TaKaRa, 9040). This was named TK promoter-PLAP basic vector fragment. Then, the IL4RE oligo DNA digested with restriction enzymes KpnI and XbaI was inserted into the TK promoter-PLAP basic vector fragment by a ligase reaction (TAKARA BIO, Cat. 6022). The plasmid was transfected into *E. coli* by a conventional method to transform the *E. coli.* Plasmid was recovered from several *E. coli* transformants and the sequence was confirmed by ABI prism DNA sequencing kit (Applied Biosystems), thereby STAT6-PLAP reporter plasmid (Fig. 16) was obtained.

Example 13: Preparation of STAT6-expression vector plasmid

Human peripheral blood monocytes were prepared from normal human blood and RNA was extracted therefrom by using RNeasy kit (QIAGEN). Then, by using TAKARA RNA LA PCR kit (TAKARA), reverse transcription reaction (42°C for 45 minutes and 99°C for 5 minutes) was performed according to the manual of the kit, thereby cDNA was prepared.
Then, for cloning a human STAT6 gene, PCR was performed by using the cDNA obtained by the above-mentioned procedure as a template and by using the oligo DNAs each represented by SEQ ID No: 43 and SEQ ID No: 44 (purchased from Japan Bio Service). The PCR was performed under the condition of 94°C for 5 minutes, followed by repeating 30 cycles of reaction of 98°C for 1 minute, 60°C for 1 minute, and 73°C for 3 minutes, and then 73°C for 10 minutes to completely perform elongation reaction. The primers have the following nucleotide sequences, respectively.
Primer: CGGAATTCATGTCTCTGTGGGGTCTGGTCTCCA (SEQ ID NO: 43)
Primer: GCTCTAGATCACCAACTGGGGTTGGCCCTTAGG (SEQ ID NO: 44)
Subsequently, the PCR product was digested with restriction enzymes EcoRI and XbaI and inserted by a ligase reaction (TAKARA BIO, Cat. 6022) into a pBluescript SK(+) plasmid (TOYOBO) which had been digested with restriction enzymes EcoRI and XbaI. The plasmid was transfected into *E. coli* by a conventional method to transform the *E. coli.* Plasmid was recovered from several *E. coli* transformants and the sequence was confirmed by ABI prism DNA sequencing kit (Applied Biosystems), thereby pBluescript SK(+)/STAT6 plasmid was obtained.
Then, the pBlescript SK(+)/STAT6 plasmid was digested with restriction enzymes EcoRI and XbaI by a conventional method to excise the STAT6 gene, which was then inserted by a ligase reaction (TAKARA BIO, Cat. 6022) into pcDNA3.1 (+) plasmid (Invitrogen) which had been digested with restriction enzymes EcoRI and XbaI. The plasmid was transfected into E. *coli* by a conventional method to transform the E. *coli.* Plasmid was recovered from several E. *coli* transformants and the sequence was confirmed by ABI prism DNA sequencing kit (Applied Biosystems), thereby STAT6-expression vector plasmid was obtained (Fig. 17).

Example 14: Preparation of cells into which STAT6-PLAP reporter plasmid and STAT6-expression vector plasmid had been stably transfected

The STAT6-PLAP reporter plasmid and STAT6-expression vector plasmid each obtained in Examples 12 and 13 were transfected into a human embryonic kidney cell line HEK293 (ATCC1573) and subjected to cloning to obtain a clone having a high PLAP expression induction ratio by IL4. Hereinafter, detailed description is made.
HEK293 cells subcloned in DMEM were recovered and suspended in DMEM to be $1.0\times10^5$ cells/ml. Here, DMEM refers to a medium consisting of 500 ml of DMEM (SIGMA, D6429), 5 ml of Penicillin Streptomycin (Invitrogen, 15140-122), and 50 ml of calf fetal serum which had been inactivated by heat treatment at 56°C for 20 minutes (hereinafter, the same holds true). Then, the cell suspension was inoculated at a volume of 3 ml/well on a 6-well cell culture plate (Becton Dickinson Labware, 35-3046) and cultured in a $CO_2$ incubator. On the next day, the cells were washed with OPTI-MEM I (Invitrogen, 31985-062) solution and 1.5 ml of OPTI-MEM I solution was added to the cells. 3 μl each of 500 μg/ml STAT6-PLAP reporter plasmid and 500 μg/ml STAT6-expression vector plasmid were added to 274 μl of the OPTI-MEM I solution. After that, 20 μl of a Lipofectamine (Invitrogen, 18324-012) solution was added thereto, and the mixture was incubated at room temperature for 20 minutes. Then, 1.2 ml of the OPTI-MEM I solution was added to make a solution have a volume of 1.5 ml, which was then added to the culture supernatant of the cells, and the cells were cultured in a $CO_2$ incubator for 2 hours. 1.5 ml of DMEM to which FCS had been added at a final concentration of 20% was added to the culture medium and further cultured in the $CO_2$ incubator. On the next day, the cells were removed from the wall of the vessel with trypsin-EDTA treatment and suspended to be a concentration of 5 cells/ml in DMEM which contains G-418 (Geneticin: Invitrogen) at a final concentration of 1 mg/ml. 200 μl each of the cells was inoculated on a 96-well cell culture plate (Becton Dickinson Labware, 35-3072) to be 1 cell/well, and a cell clone into which STAT6-PLAP reporter plasmid and STAT6-expression vector plasmid had been stably transfected was selected.

Then, whether the cloned cell shows increased PLAP activity upon stimulation of IL4 was studied. More specifically, the cloned cell was subjected to trypsin-EDTA treatment to be removed from the wall of the vessel and suspended in DMEM and cells were inoculated on a 96-well cell culture plate to be $1.0 \times 10^4$ cells/190 μl/well. On the next day, 10 μl of 20 ng/ml human IL4 (Calbiochem, 407635) was added to the culture medium at a final concentration of 1 ng/ml. On the next day, the culture supernatant was recovered and the PLAP activity in the culture supernatant was measured. More specifically, 100 μl of PLAP buffer (0.28 M $Na_2CO_3$-$NaHCO_3$ pH 10.0, 8 mM $MgSO_4$) was added to a black plate for ELISA (Sumitomo Bakelite Co., Ltd., No. MS-8496K), and 10 μl of the culture supernatant which had been treated in a hot water bath at 65°C for 10 minutes was added thereto. Then, 50 μl of Lumi-Phos 530 (Lumigen, Inc.) was added, followed by mixing. Subsequently, while shielding light, the mixture was incubated at room temperature for 1 hour, followed by measurement of intensity of chemiluminescence using a plate reader (Perkin Elmer, ARVO). As a result, a clone in which higher PLAP activity was induced by IL4 stimulation as compared to a non-stimulating condition was obtained as shown in Fig. 18. Hereinafter, this clone is named E9 cell.

Example 15: Preparation of mouse air pouch model by introduction of E9 cells

5% cytodex3 (Amersham Bioscience, 17-0485-01) solution and 2% carboxymethylcellulose (hereinafter, also referred to as CMC) solution (Daiichi Kogyo Seiyaku Co., Ltd.) were prepared. More specifically, 5 g of cytodex3 was suspended in 100 ml of PBS (SIGMA, R8537) and sterilized in an autoclave. On the other hand, the 2% CMC solution was prepared by adding 2 g of CMC in 100 ml of PBS while stirring with a stirrer bar, followed by sterilization in the autoclave.
Then, the E9 cells were subjected to trypsin-EDTA treatment to be removed from the wall of the vessel and the cells were suspended in DMEM to be $1.0 \times 10^6$ cells/ml. 0.2 g of cytodex3 was mixed with $1.0 \times 10^7$ cells/ml E9 cells. More specifically, 4 ml of 5% cytodex3 solution was added to 10 ml of $1.0 \times 10^6$ cells/ml cell suspension. Then, the cell/cytodex3 suspension was inoculated on a non-tissue culture plate (IWAKI, SH90-15) and cultured in a $CO_2$ incubator. On the next day, the cells were recovered and centrifuged to remove the supernatant. DMEM was added to the cells to be $2.0 \times 10^6$ cells/ml, and further the equal amount of 2% CMC solution was added so as to adjust the cell concentration to be $1.0 \times 10^6$ cells/ml and the final concentration of CMC to be 1%.

Under anesthesia with diethyl ether (WAKO, O55-01155), 3 ml of air was introduced under the skin on the back of a CDF1 mouse (Charles River Japan) using a 5-ml syringe with a 27G needle to make an air pouch. Then, 2 ml of the cell suspension was injected into the air pouch using a syringe with a 22G needle. More specifically, $2.0 \times 10^6$ cells/ml of cells per mouse was injected. Then, 1 ml of human IL4 (Calbiochem, 407635) adjusted to 3 ng/ml with 1% CMC was injected into the air pouch of the mouse using a syringe with a 22G needle, and well mixed. After 24 hours, orbital blood drawing was conducted using a heparin-treated Terumo hematocrit capillary tube (TERUMO Corporation, VC-H075H). The collected blood was centrifuged at 3,000 rpm for 10 minutes to obtain a plasma fraction. To inactivate endogenous alkaline phosphatases, the plasma sample was treated in a hot water bath at 65°C for 10 minutes and then the PLAP activity derived from E9 cells in the plasma was measured. More specifically, 40 μl of PLAP buffer (0.28 M $Na_2CO_3$-$NaHCO_3$ pH 10.0, 8 mM $MgSO_4$) was added to a black plate for ELISA (Sumitomo Bakelite Co., Ltd., No. MS-8496K), and 10 μl of the plasma sample was added thereto. Then, 50 μl of Lumi-Phos 530 (Lumigen, Inc.) was added, and mixed. Subsequently, while shielding light, the mixture was incubated at room temperature for 1 hour, followed by measurement of intensity of chemiluminescence using a plate reader (Perkin Elmer, ARVO). As a result, a remarkable increase of PLAP activity secreted in blood upon IL4 stimulation was detected (Fig. 19).

The above-mentioned results revealed that the transcriptional activity in transplanted cells in a nonhuman animal model can be measured by transplanting secretory protein-expression vector-transfected cells into the nonhuman animal and measuring the amount of the secretory protein in the nonhuman animal.
Further, the above-mentioned results indicated that a carrier can be used in the transplantation.
Furthermore, the above-mentioned results indicated that the transcription regulatory sequence is not limited to a particular sequence.
Also, the above-mentioned results indicated that measurement of the transcriptional activity is possible not only in tumor cells but also in immortalized cells.

Example 16: Test of inhibitory action in mouse air pouch model introduced with E9 cells

To study whether a compound that affects transcriptional activity in transplanted cells in a nonhuman animal model can be screened, experiments were performed using 2-(6-[3-(4-fluorophenyl)-1H-4-pyrazolyl]imidazo[1,2-a]pyridin-3-yl)-1,3-thiazole trihydrochloride, a compound known to inhibit transcriptional activity through STAT6 activation by IL4 stimulation in vitro (WO02/088107).

5% cytodex3 (Amersham Bioscience, 17-0485-01) solution and 2% CMC solution (Daiichi Kogyo Seiyaku Co., Ltd.) were prepared. More specifically, 5 g of cytodex3 was suspended in 100 ml of PBS (SIGMA, R8537) and sterilized by autoclaving. On the other hand, the 2% CMC solution was prepared by adding 2 g of CMC in 100 ml of PBS while stirring with a stirrer bar and followed by sterilization by autoclaving.
Then, E9 cells were subjected to trypsin-EDTA treatment to be removed from the wall of the vessel and the cells were suspended in DMEM to make $1.0 \times 10^6$ cells/ml. 0.2 g of cytodex3 was mixed with $1.0 \times 10^7$ cells/ml of E9 cells. More specifically, 4 ml of 5% cytodex3 solution was mixed with 10 ml of $1.0 \times 10^6$ cells/ml of cell suspension. Then, the cell/cytodex3 suspension was inoculated on a non-tissue culture plate (IWAKI, SH90-15) and cultured in a $CO_2$ incubator. On the next day, the cells were recovered and centrifuged to remove supernatant. DMEM was added to the cells to be $2.0 \times 10^6$ cells/ml, and further the equal amount of 2% CMC solution was added to adjust the cell concentration to be $1.0 \times 10^6$ cells/ml and the final concentration of CMC to be 1%.

Under anesthesia with diethyl ether (WAKO, 055-01155), 3 ml of air was introduced under the skin on the back of a CDF1 mouse (Charles River Japan) with a 5-ml syringe with a 27G needle to make an air pouch. Then, 2 ml of the cell suspension was injected into the air pouch with a syringe with a 22G needle. More specifically, $2.0 \times 10^6$ cells per mouse were injected.
Then, 2-(6-[3-(4-fluorophenyl)-1H-4-pyrazolyl]-imidazo-[1,2-a]-pyridin-3-yl)-1,3-thiazole trihydrochloride (prepared according to the production method described in WO02/088107) was suspended in an aqueous 0.5% methylcellulose (WAKO PURE CHEMICAL INDUSTRIES, LTD.) solution using an agate mortar. The test compound solution was orally administered at a dose of 20 mg/kg. On the other hand, aqueous 0.5% methylcellulose solution was administered to the control group.
After 1 hour from the administration, 1 ml of IL4 (Calbiochem, 407635) adjusted to 3 ng/ml with 1% CMC was injected into the air pouch of the mouse with a syringe with a 22G needle and well mixed.
After 24 hours, orbital blood drawing was conducted using a heparin-treated Terumo hematocrit capillary tube (TERUMO Corporation, VC-H075H). The collected blood was centrifuged at 3,000 rpm for 10 minutes to obtain a plasma fraction. To inactivate endogenous alkaline phosphatase, the plasma sample was treated in a hot water bath at 65°C for 10 minutes and then PLAP activity derived from E9 cells in the plasma was measured. More specifically, 40 μl of PLAP buffer (0.28 M $Na_2CO_3$-$NaHCO_3$ pH 10.0, 8 mM $MgSO_4$) was added to a black plate for ELISA (Sumitomo Bakelite Co., Ltd., No. MS-8496K), and 10 μl of the plasma sample was added thereto. Then, 50 μl of Lumi-Phos 530 (Lumigen, Inc.) was added, and mixed. Subsequently, while shielding light, the mixture was incubated at room temperature for 1 hour, followed by measurement of intensity of chemiluminescence using a plate reader (Perkin Elmer, ARVO). As a result, the test compound suppressed 94.1 % of the PLAP activity activated by the IL4 stimulation as compared with the control group (Fig. 20).

Therefore, it was revealed that a compound that affects the transcriptional activity in transplanted cells in a nonhuman animal model can be screened by administrating a compound to a nonhuman animal model that produces a secretory protein obtained by transplanting secretory protein-expression vector-transfected cells into a nonhuman animal; measuring the amount of the secretory protein in the biological fluid, and selecting the compound that changes the amount of the secretory protein.

Example 17: Preparation of SV40-PLAP reporter plasmid

A polynucleotide consisting of SV40 promoter was inserted into the KpnI/HindIII site of the PLAP basic vector plasmid to obtain SV40-PLAP reporter plasmid. More specifically, first, pSEAP control reporter plasmid (Clontech) was cleaved with BglII and MluI, and then the cleaved ends were blunted by a blunting reaction (TAKARA BIO Co., Ltd., Cat. 6025). Then, the plasmid was subjected to self-ligation by a ligase reaction. The plasmid was transfected into *E. coli* by a conventional method to transform the *E. coli.* Plasmid was recovered from several *E.* coli transformants and the sequence was confirmed by ABI prism DNA sequencing kit (Applied Biosystems). Then, the plasmid was cleaved with KpnI and HindIII to obtain SV40 promoter fragment. The SV40 promoter fragment was inserted by a ligase reaction into the PLAP basic vector plasmid which had been cleaved with KpnI and HindIII in advance. The plasmid was transfected into *E. coli* by a conventional method to transform the *E. coli.* Plasmid was recovered from several E. *coli* transformants and the sequence was confirmed by ABI prism DNA sequencing kit (Applied Biosystems), thereby SV40-PLAP reporter plasmid was obtained (Fig. 21).

Example 18: Preparation of cells into which SV40-PLAP reporter plasmid has been stably transfected

The SV40-PLAP reporter plasmid obtained in Example 12 was transfected into human gastric cancer cell line MKN-45 cells using Effectone Reagent (QIAGEN, Cat. No. 301427) to clone stably transfected cells. More specifically, MKN-45 cells (JCRB Cell Bank, JCRB 0254) subcultured in RPMI medium were recovered and suspended in the RPMI medium to be $10 \times 10^4$ cells/ml. Then, the cell suspension was inoculated at a volume of 2 ml/well on a 6-well cell culture plate (Becton Dickinson Labware, 35-3046) and cultured in a $CO_2$ incubator. On the next day, 200 μl of EC buffer and 16 μl of Enhancer were added to 2 μg of the SV40-PLAP reporter plasmid and incubated at room temperature for 5 minutes.
Then 20 μl of Effectone Reagent was added to the DNA solution, which was further incubated at room temperature for 5 minutes. 964 μl of the RPMI medium was added to the DNA solution and mixed, and the mixture was added to the culture supernatant of the cells at a volume of 964 μl/well, and incubated in a $CO_2$ incubator. On the next day, the culture supernatant of the cells was removed by suction and 2 ml of a fresh RPMI medium was added. On the next day, the cells were subjected to trypsin-EDTA treatment to be removed from the wall of the vessel and suspended in RPMI medium containing 600 μg/ml G-418 (Geneticin: Invitrogen). The suspension was inoculated in a 15-cm-diameter cell culture Petri dish (Becton Dickinson Labware, 35-3025), and the cultivation was continued in the $CO_2$ incubator. The RPMI medium containing 600 μg/ml of G-418 was exchanged every four days. On day 14 from the cultivation with addition of G-418, the cells were subjected to trypsin-EDTA treatment to be removed from the wall of the vessel and cloned by a limiting dilution technique according to a conventional method, thereby MKN-45/SV40-PLAP(M1) was obtained.

Example 19: In vivo measurement of transcriptional activity of SV40-PLAP reporter plasmid-transfected cells (subcutaneous transplantation)

MKN-45/SV40-PLAP (M1) subcultured in RPMI medium containing 600 μg/ml G-418 (Example 13) was subjected to trypsin-EDTA treatment to be removed from the wall of the vessel and suspended in the RPMI medium to be $5 \times 10^7$ cells/ml. 100 μl of the cell suspension ($5 \times 10^6$ cells/mouse) was subcutaneously transplanted to a BALB/c nude mouse (female, 8 weeks, purchased from Charles River Japan Inc.) by using a 1-ml syringe (with a 26G needle) for tuberculin. Orbital blood drawing was conducted using a heparin-coated hematocrit tube (manufactured by Drummond) on consecutive days including the day of transplantation. The hematocrit tube was closed on one end thereof with a putty for its exclusive use (manufactured by Terumo Corporation). The obtained blood was centrifuged at 10,000 rpm for 2 minutes using a centrifuge for hematocrit tubes to separate a plasma fraction. The plasma was diluted 10-fold with physiological saline to prepare 10-fold diluted plasma, which was stored under refrigeration at -20°C until assay of its PLAP activity. Measurement of the tumor volume of a tumor composed of MKN-45/SV40-PLAP(M1) and assay of plasma PLAP activity were performed by the method described in Example 4.
As a result, the blood PLAP activity was 1,000 units or less before the transplantation of MKN-45/SV40-PLAP (M1), but it increased with increasing tumor volume (Fig. 22). The time-dependent change of the blood PLAP activity was almost parallel to the time-dependent change of the tumor volume. Therefore, it was revealed that the number of tumor cells can be estimated by measuring the blood PLAP activity in a nonhuman animal model.
The above-mentioned results revealed that in a nonhuman animal model that produces a secretory protein obtained by transplanting secretory protein-expression vector-transfected cells into a nonhuman animal, the number of transplanted cells and the tumor volume can be determined by measuring the amount of the secretory protein in the biological fluid and using the amount of the secretory protein as an index.

INDUSTRIAL APPLICABILITY

According to the present invention, a method of noninvasively, simply, and accurately measuring transcriptional activity of a transcription regulatory sequence in transplanted cells in a nonhuman animal model has been established and quantitative evaluation has been made possible.
The present invention has made it possible to measure time-dependent change of transcriptional activity that has been difficult to be measured by cumbersome, less quantitative evaluation conventional methods.
Further, according to the present invention, a method of noninvasively, simply, and accurately screening a compound that affects transcriptional activity of a transcription regulatory sequence in a nonhuman animal model has been established and quantitative evaluation has been made possible.
Furthermore, a method of noninvasively, simply, and accurately measuring the number of transplanted cells and tumor volume and a method of screening a compound that affects the number of transplanted cells or tumor volume have been established, by measuring an amount of secretory protein in a biological fluid of a nonhuman animal model obtained by transplanting cells that have been transfected with secretory protein-expression vector containing a constitutive transcription regulatory sequence as a transcription regulatory sequence into a nonhuman animal model, and thus quantitative evaluation has been made possible.
The present invention has made it possible to measure a time-dependent change of tumor volume in an orthotopic transplantation model transplanted with tumor cells, which has been difficult to be evaluated by conventional evaluation methods.

## Claims

1. A method of measuring transcriptional activity in cells transplanted into a nonhuman animal model, comprising:

   measuring an amount of a secretory protein in a nonhuman animal model that produces the secretory protein, the nonhuman animal model being obtained by transplanting cells that have been transfected therein an expression vector comprising a transcription regulatory sequence and a polynucleotide coding for the secretory protein operably linked to the transcription regulatory sequence, into a nonhuman animal; and
   measuring transcriptional activity through the transcription regulatory sequence based on the amount of the secretory protein.

2. The method according to claim 1, wherein the transcription regulatory sequence comprises a transcription regulatory factor-binding sequence.

3. The method according to claim 2, wherein the transcription regulatory factor-binding sequence is at least one sequence selected from the group consisting of SEQ ID No: 1, SEQ ID No: 2, SEQ ID No: 3, SEQ ID No: 4, SEQ ID No: 5, SEQ ID No: 6, SEQ ID No: 7, and SEQ ID No: 8.

4. The method according to any one of claims 1 to 3, wherein the secretory protein is a secretory enzyme.

5. The method according to claim 4, wherein the secretory enzyme is a secretory alkaline phosphatase.

6. The method according to claim 5, wherein the secretory alkaline phosphatase is a heat-resistant secretory alkaline phosphatase.

7. The method according to claim 5, wherein the secretory alkaline phosphatase is a secretory placenta-derived alkaline phosphatase.

8. The method according to claim 7, wherein the secretory placenta-derived alkaline phosphatase is a protein consisting of an amino sequence of SEQ ID No: 11.

9. The method according to any one of claims 1 to 8, wherein an amount of the secretory protein in blood is measured.

10. The method according to claim 9, wherein the amount of the secretory protein in blood is measured by measuring an enzymatic activity.

11. The method according to claim 10, wherein the enzymatic activity is alkaline phosphatase activity.

12. The method according to any one of claims 1 to 11, wherein the cells are tumor cells or immortalized cells.

13. A method of screening a compound that affects transcriptional activity, comprising the steps of:

   (a) administering a compound to a nonhuman animal model that produces a secretory protein, the nonhuman animal model being obtained by transplanting cells that have been transfected therein an expression vector comprising a polynucleotide coding for the secretory protein, into a nonhuman animal; and

(b) measuring transcriptional activity in the transplanted cells in the nonhuman animal model administered with the compound, by the method according to any one of claims 1 to 12.

**14.** A method of screening a compound that affects transcriptional activity, comprising the steps of:

(a) transplanting cells that have been transfected therein an expression vector comprising a polynucleotide coding for a secretory protein, into a nonhuman animal administered with a compound; and
(b) measuring transcriptional activity in the transplanted cells in the nonhuman animal model, by the method according to any one of claims 1 to 12.

**15.** A method of measuring the number of transplanted cells in a nonhuman animal model, comprising:

measuring an amount of a secretory protein in a nonhuman animal model that produces the secretory protein, the nonhuman animal model being obtained by transplanting cells that have been transfected therein an expression vector comprising a transcription regulatory sequence and a polynucleotide coding for a secretory protein operably linked to the transcription regulatory sequence, into a nonhuman animal; and
measuring the number of the transplanted cells based on the amount of the secretory protein.

**16.** The method according to claims 15, wherein the secretory protein is a secretory enzyme.

**17.** The method according to claim 16, wherein the secretory enzyme is a secretory alkaline phosphatase.

**18.** The method according to claim 17, wherein the secretory alkaline phosphatase is a heat-resistant secretory alkaline phosphatase.

**19.** The method according to claim 17, wherein the secretory alkaline phosphatase is a secretory placenta-derived alkaline phosphatase.

**20.** The method according to claim 19, wherein the secretory placenta-derived alkaline phosphatase is a protein consisting of an amino sequence represented by SEQ ID No: 11.

**21.** The method according to any one of claims 15 to 20, wherein an amount of the secretory protein in blood is measured.

**22.** The method according to claim 21, wherein the amount of the secretory protein in blood is measured by measuring an enzymatic activity.

**23.** The method according to claim 22, wherein the enzymatic activity is alkaline phosphatase activity.

**24.** The method according to any one of claims 15 to 23, wherein the cells are tumor cells or immortalized cells.

**25.** The method according to any one of claims 15 to 24, wherein the transcription regulatory sequence comprises a constitutive transcription regulatory sequence.

**26.** The method according to claim 25, wherein the constitutive transcription regulatory sequence is at least one sequence selected from the group consisting of SV40 promoter, CMV promoter, thymidine kinase promoter, ubiquitin C promoter, elongation factor 1 alpha (EF1a) promoter, β-actin promoter, glyceraldehyde-3-phosphate dehydrogenase promoter, phosphoglycerokinase promoter, β2-microglobulin promoter, and β-glucuronidase promoter.

**27.** The method according to claim 25, wherein the constitutive transcription regulatory sequence is SV40 promoter.

**28.** The method according to claim 25, wherein the constitutive transcription regulatory sequence is a sequence represented by SEQ ID No: 9.

**29.** A method of screening a compound that affects transcriptional activity, comprising the steps of:

(a) administering a compound to a nonhuman animal model that produces a secretory protein, the nonhuman animal model being obtained by transplanting cells that have been transfected therein an expression vector comprising a polynucleotide coding for the secretory protein, into a nonhuman animal; and

(b) measuring the number of the transplanted cells in the nonhuman animal model administered with the compound, by the method according to any one of claims 15 to 28.

**30.** A method of screening a compound that affects the number of transplanted cells, comprising the steps of:

(a) transplanting cells that have been transfected therein an expression vector comprising a polynucleotide coding for a secretory protein, into a nonhuman animal administered with a compound; and
(b) measuring the number of the transplanted cells in the nonhuman animal by the method according to any one of claims 15 to 28.

**31.** A method of measuring tumor volume in a nonhuman animal model, comprising:

measuring an amount of a secretory protein in a nonhuman animal model that develops a tumor and produces the secretory protein in the tumor, the nonhuman animal model being obtained by transplanting cells that have been transfected therein an expression vector comprising a transcription regulatory sequence and a polynucleotide coding for a secretory protein operably linked to the transcription regulatory sequence, into a nonhuman animal; and
measuring tumor volume based on the amount of the secretory protein.

**32.** The method according to claim 31, wherein the secretory protein is a secretory enzyme.

**33.** The method according to claim 32, wherein the secretory enzyme is a secretory alkaline phosphatase.

**34.** The method according to claim 33, wherein the secretory alkaline phosphatase is a heat-resistant secretory alkaline phosphatase.

**35.** The method according to claim 33, wherein the secretory alkaline phosphatase is a secretory placenta-derived alkaline phosphatase.

**36.** The method according to claim 35, wherein the secretory placenta-derived alkaline phosphatase is a protein consisting of an amino sequence represented by SEQ ID No: 11.

**37.** The method according to any one of claims 31 to 36, wherein an amount of the secretory protein in blood is measured.

**38.** The method according to claim 37, wherein the amount of the secretory protein in blood is measured by measuring an enzymatic activity.

**39.** The method according to claim 38, wherein the enzymatic activity is alkaline phosphatase activity.

**40.** The method according to any one of claims 31 to 39, wherein the cell is a tumor cell or an immortalized cell.

**41.** The method according to any one of claims 31 to 40, wherein the transcription regulatory sequence comprises a constitutive transcription regulatory sequence.

**42.** The method according to claim 41, wherein the constitutive transcription regulatory sequence is at least one sequence selected from the group consisting of SV40 promoter, CMV promoter, thymidine kinase promoter, ubiquitin C promoter, elongation factor 1 alpha (EF1a) promoter, β-actin promoter, glyceraldehyde-3-phosphate dehydrogenase promoter, phosphoglycerokinase promoter, β2-microglobulin promoter, and β-glucuronidase promoter.

**43.** The method according to claim 41, wherein the constitutive transcription regulatory sequence is an SV40 promoter.

**44.** The method according to claim 41, wherein the constitutive transcription regulatory sequence is a sequence represented by SEQ ID No: 9.

**45.** A method of screening a compound that affects tumor volume, comprising the steps of:

(a) administering a compound to a nonhuman animal model that develops a tumor and produces a secretory protein in the tumor, the nonhuman animal model being obtained by transplanting cells that have been transfected therein an expression vector comprising a polynucleotide coding for the secretory protein, into a nonhuman animal; and
(b) measuring tumor volume in the nonhuman animal model administered with the compound, by the method according to any one of claims 31 to 44.

**46.** An expression vector comprising a polynucleotide coding for a secretory protein, for use in the method according to any one of claims 1 to 45.

**47.** A cell that has been transfected therein an expression vector comprising a polynucleotide coding for a secretory protein, for use in the method according to any one of claims 1 to 45.

**48.** A nonhuman animal that produces a secretory protein, obtained by transplanting cells that have been transfected therein an expression vector that comprises a polynucleotide coding for a secretory protein, into a nonhuman animal, for use in the method according to any one of claims 1 to 45.

**49.** A measuring kit, comprising an expression vector that comprises a polynucleotide coding for a secretory protein, for use in the method according to any one of claims 1 to 45.

**50.** A measuring kit, comprising cells that have been transfected therein an expression vector that comprises a polynucleotide coding for a secretory protein, for use in the method according to any one of claims 1 to 45.

**51.** A measuring kit, comprising a nonhuman animal that produces a secretory protein, obtained by transplanting cells that have been transfected therein an expression vector that contains a polynucleotide coding for the secretory protein, into a nonhuman animal, for use in the method according to any one of claims 1 to 45.

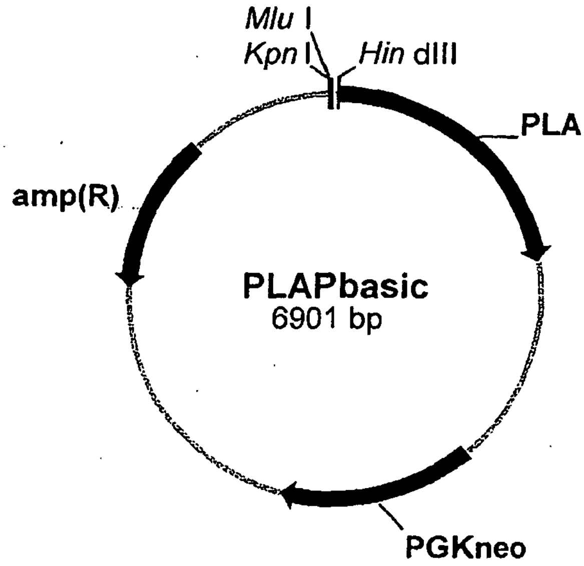
Mlu I
Kpn I
Hin dIII
PLAP
amp(R)
PLAPbasic
6901 bp
PGKneo

Fig. 1

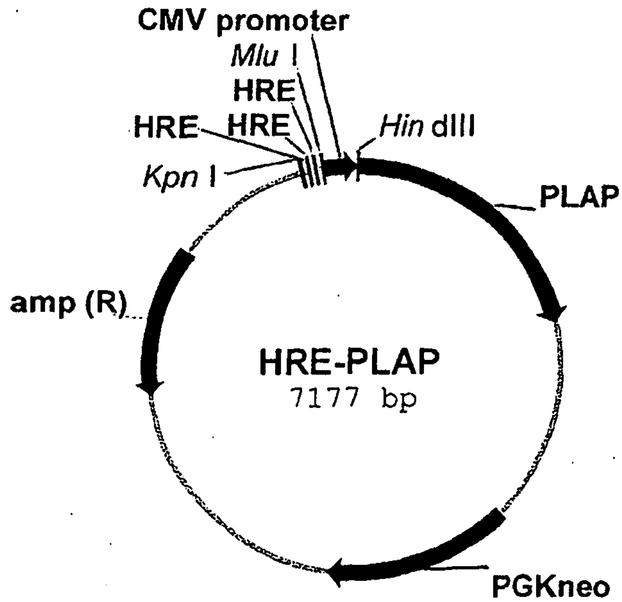
CMV promoter
Mlu I
HRE
HRE
HRE
Hin dIII
Kpn I
PLAP
amp (R)
HRE-PLAP
7177 bp
PGKneo

Fig. 2

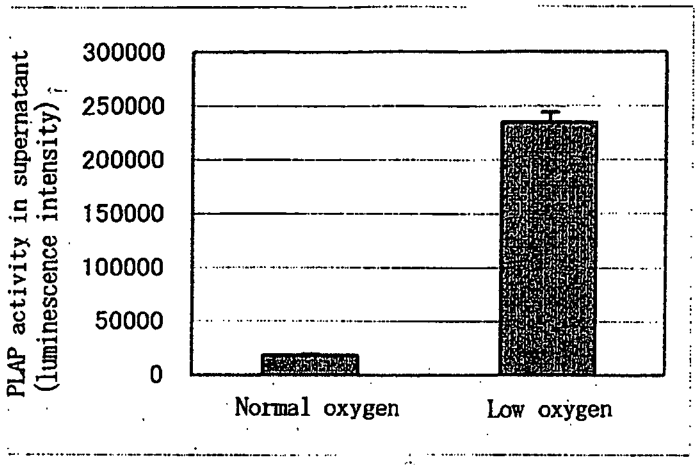
PLAP activity in supernatant (luminescence intensity)
300000
250000
200000
150000
100000
50000
0
Normal oxygen
Low oxygen

Fig. 3

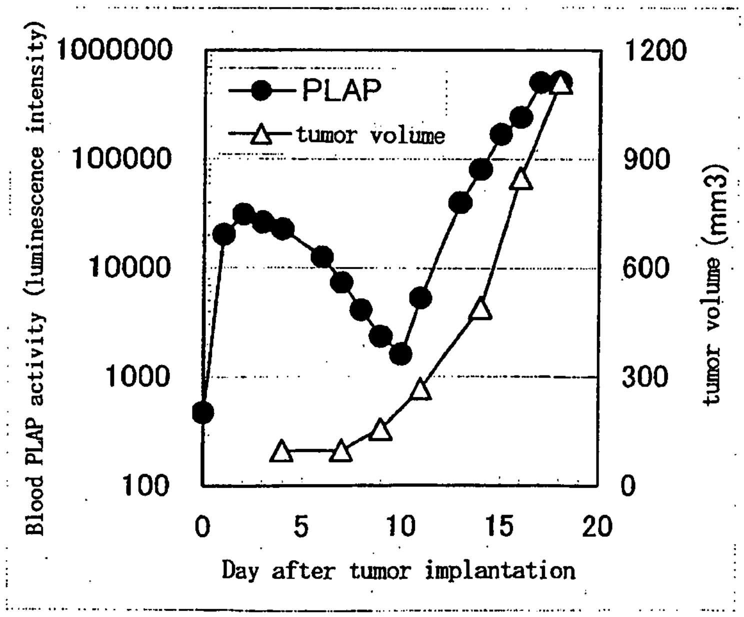
Blood PLAP activity (luminescence intensity)
1000000
100000
10000
1000
100
PLAP
tumor volume
1200
900
600
300
0
tumor volume (mm3)
0
5
10
15
20
Day after tumor implantation

Fig. 4

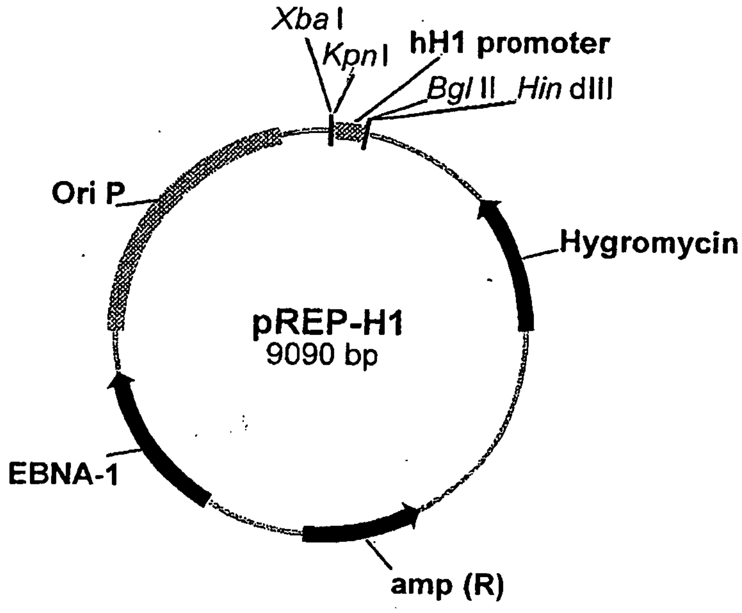
Xba I
Kpn I
hH1 promoter
Bgl II
Hin dIII
Ori P
Hygromycin
pREP-H1
9090 bp
EBNA-1
amp (R)

Fig. 5

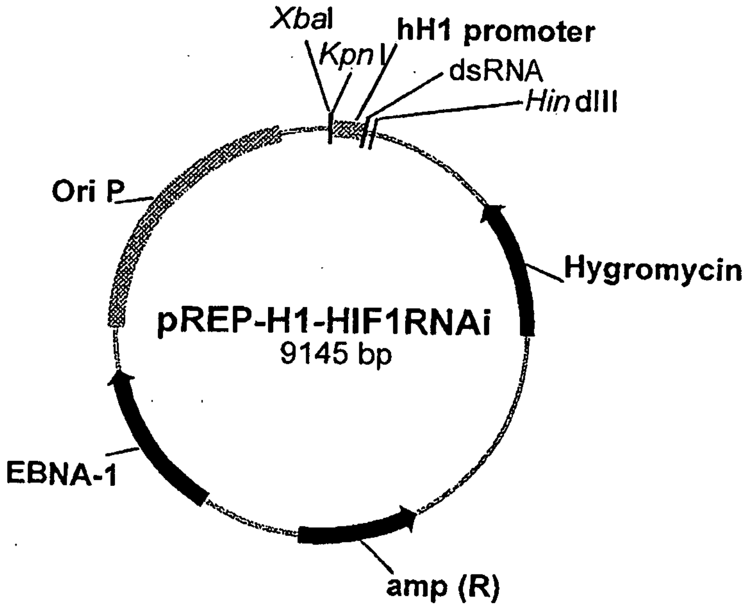
XbaI
Kpn I
hH1 promoter
dsRNA
Hin dIII
Ori P
Hygromycin
pREP-H1-HIF1RNAi
9145 bp
EBNA-1
amp (R)

Fig. 6

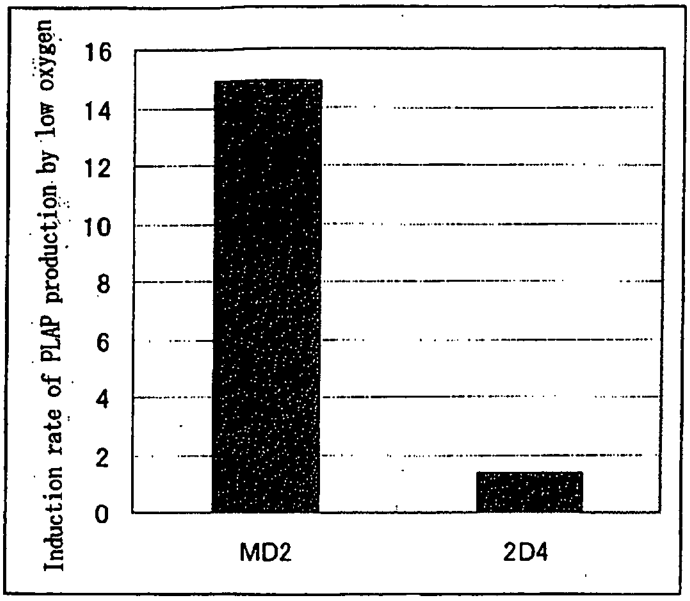
Induction rate of PLAP production by low oxygen
16
14
12
10
8
6
4
2
0
MD2
2D4

Fig. 7

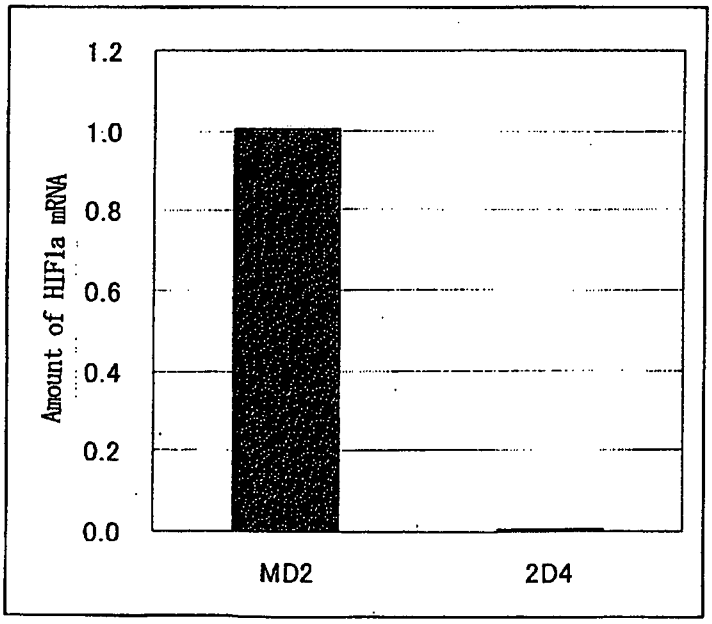
Amount of HIF1a mRNA
1.2
1.0
0.8
0.6
0.4
0.2
0.0
MD2
2D4

Fig. 8

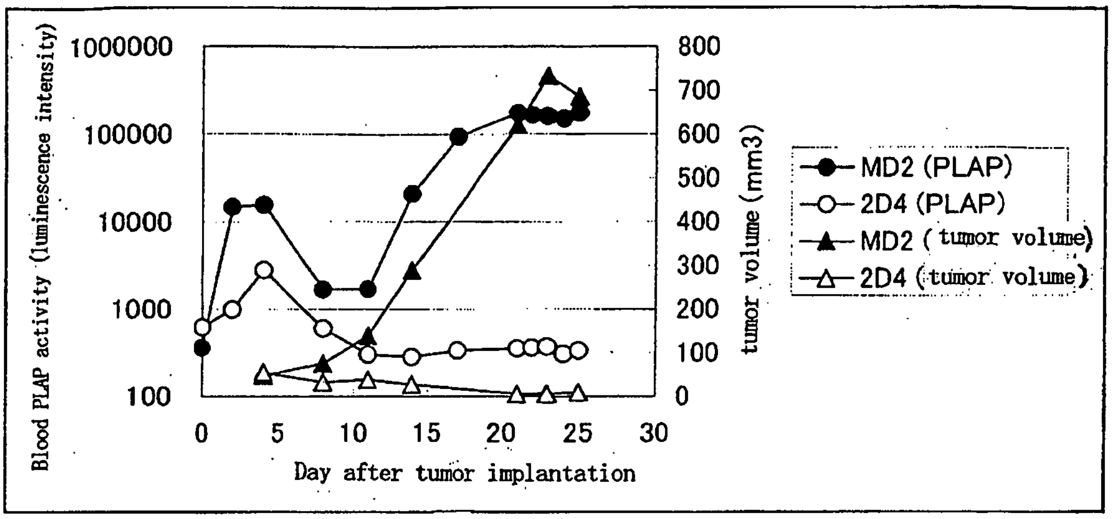
Blood PLAP activity (luminescence intensity)
1000000
100000
10000
1000
100
800
700
600
500
400
300
200
100
0
tumor volume (mm3)
0
5
10
15
20
25
30
Day after tumor implantation
MD2 (PLAP)
2D4 (PLAP)
MD2 (tumor volume)
2D4 (tumor volume)

Fig. 9

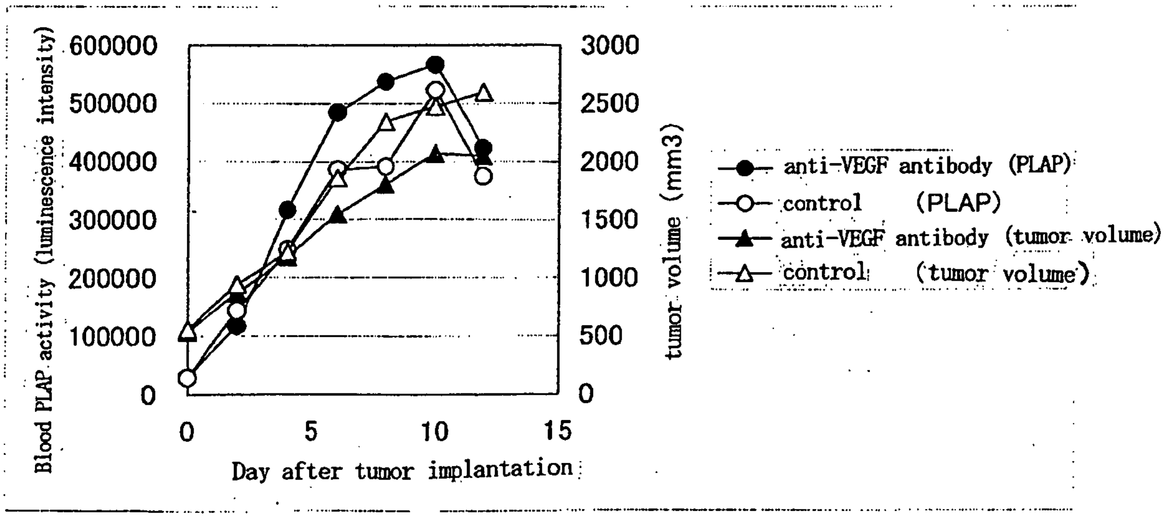
Blood PLAP activity (luminescence intensity)
600000
500000
400000
300000
200000
100000
0
3000
2500
2000
1500
1000
500
0
tumor volume (mm3)
0
5
10
15
Day after tumor implantation
anti-VEGF antibody (PLAP)
control (PLAP)
anti-VEGF antibody (tumor volume)
control (tumor volume)

Fig. 10

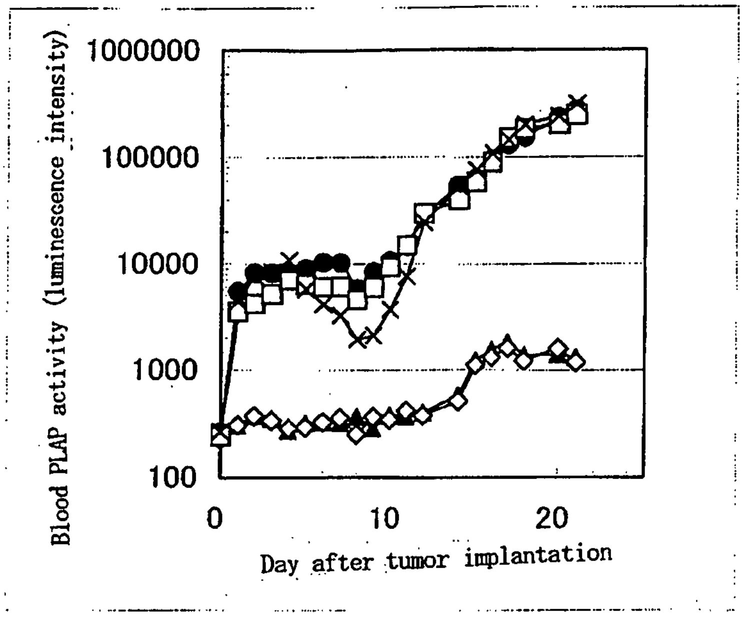
Blood PLAP activity (luminescence intensity)
1000000
100000
10000
1000
100
0
10
20
Day after tumor implantation

Fig. 11

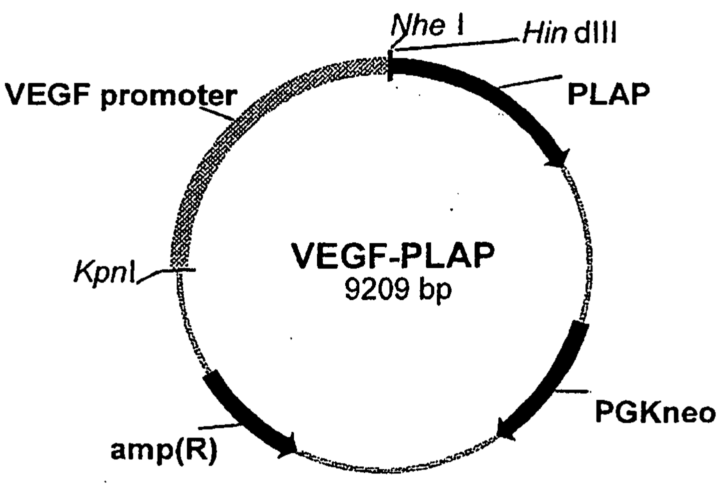
Nhe I
Hin dIII
VEGF promoter
PLAP
VEGF-PLAP
9209 bp
KpnI
PGKneo
amp(R)

Fig. 12

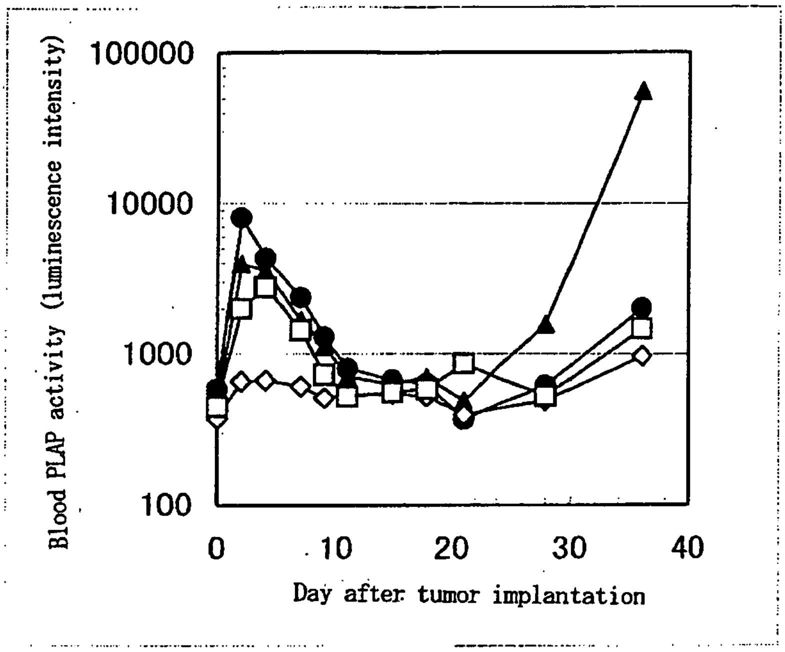
Blood PLAP activity (luminescence intensity)
100000
10000
1000
100
0
10
20
30
40
Day after tumor implantation

Fig. 13

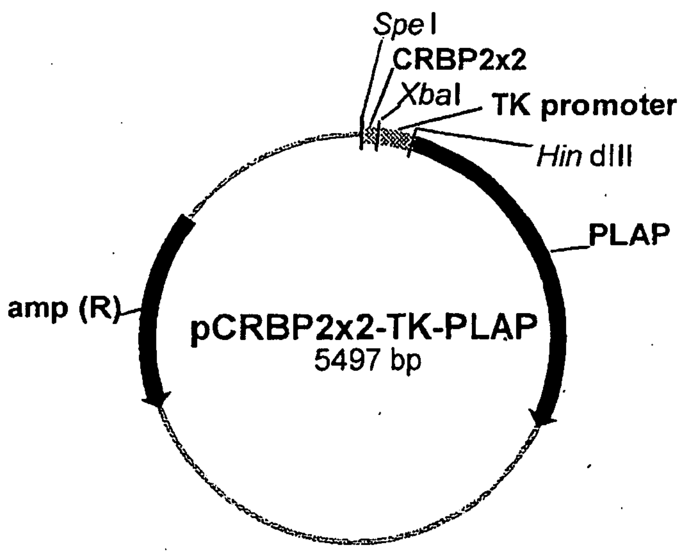
SpeI
CRBP2x2
XbaI
TK promoter
HindIII
PLAP
amp (R)
pCRBP2x2-TK-PLAP
5497 bp

Fig. 14

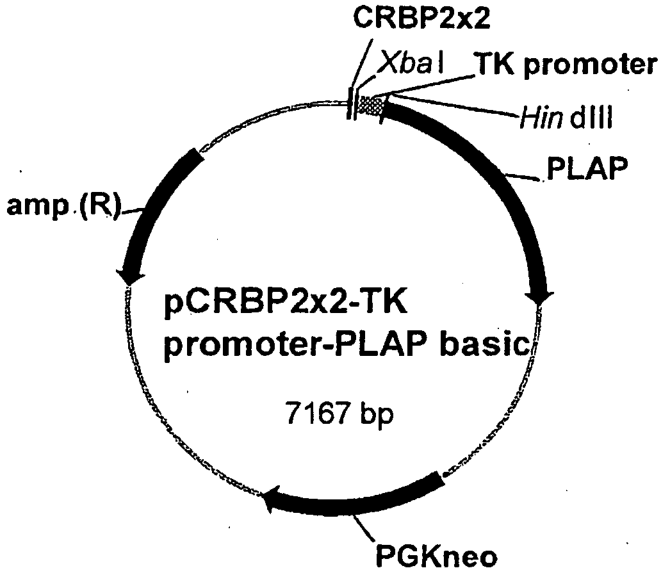
CRBP2x2
XbaI
TK promoter
Hin dIII
PLAP
amp (R)
pCRBP2x2-TK promoter-PLAP basic
7167 bp
PGKneo

Fig. 15

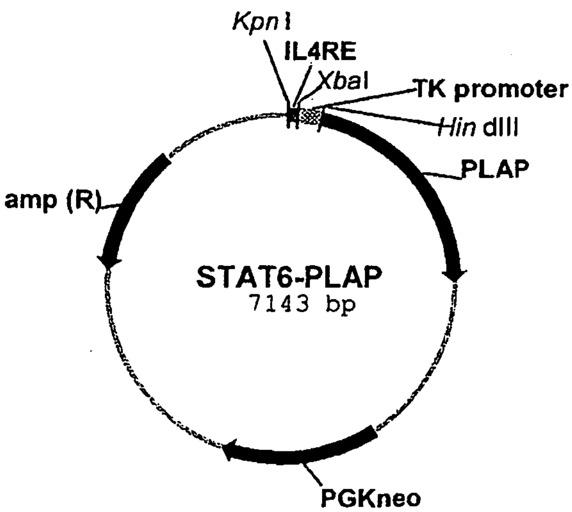
KpnI
IL4RE
XbaI
TK promoter
Hin dIII
PLAP
amp (R)
STAT6-PLAP
7143 bp
PGKneo

Fig. 16

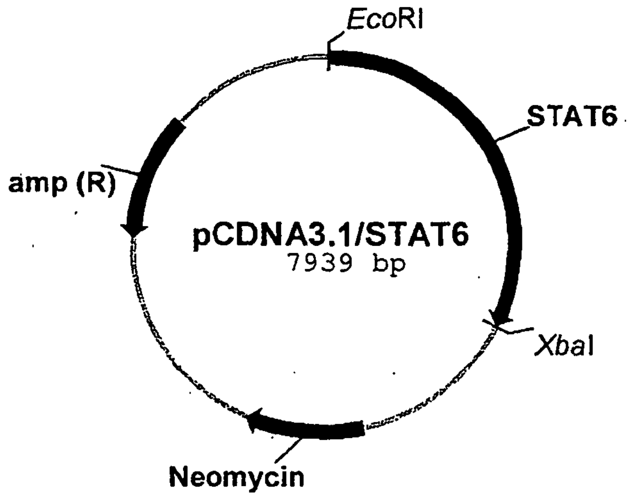
EcoRI
STAT6
amp (R)
pCDNA3.1/STAT6
7939 bp
XbaI
Neomycin

Fig. 17

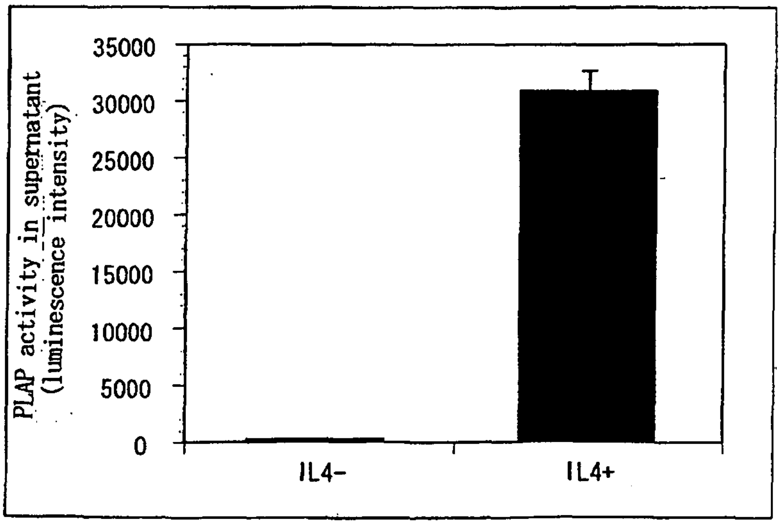
PLAP activity in supernatant (luminescence intensity)
35000
30000
25000
20000
15000
10000
5000
0
IL4-
IL4+

Fig. 18

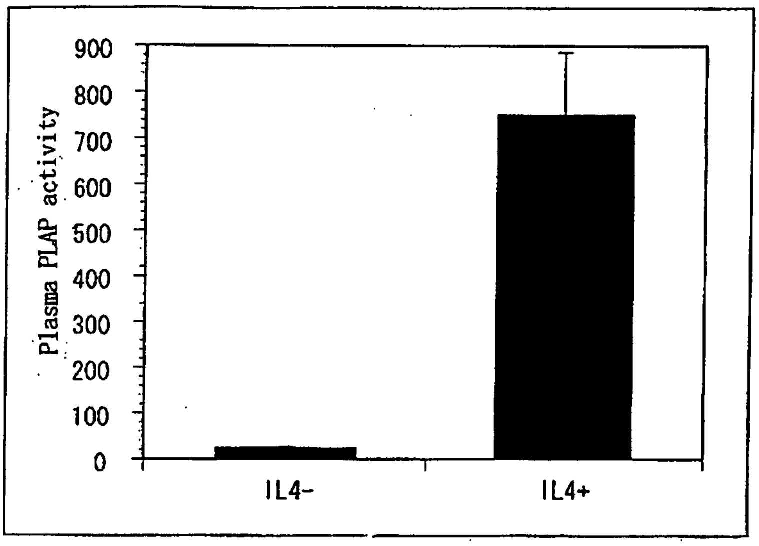
Plasma PLAP activity
900
800
700
600
500
400
300
200
100
0
IL4-
IL4+

Fig. 19

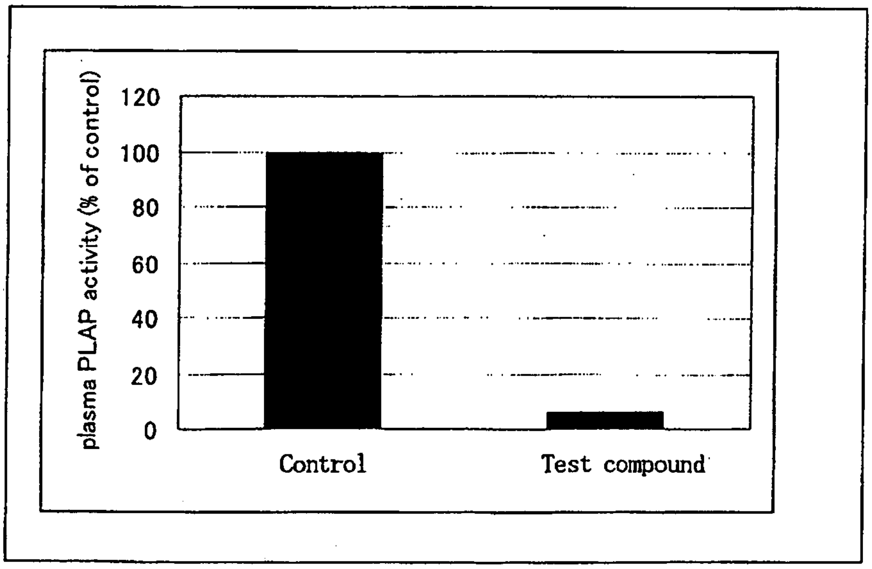
plasma PLAP activity (% of control)
120
100
80
60
40
20
0
Control
Test compound

Fig. 20

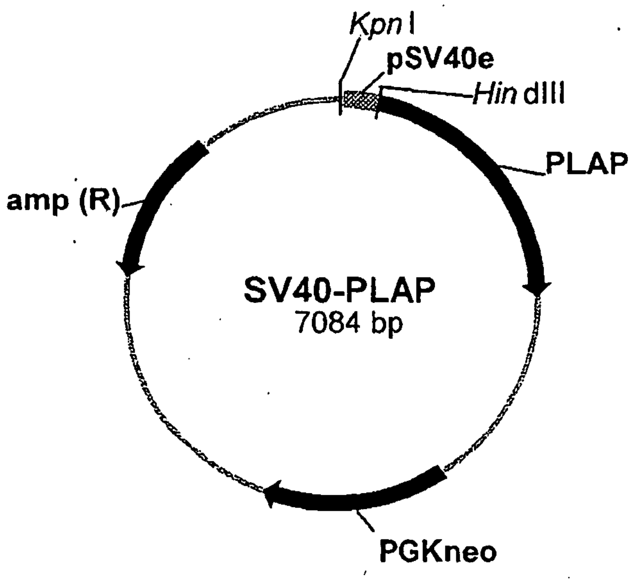
Kpn I
pSV40e
Hin dIII
PLAP
amp (R)
SV40-PLAP
7084 bp
PGKneo

Fig. 21

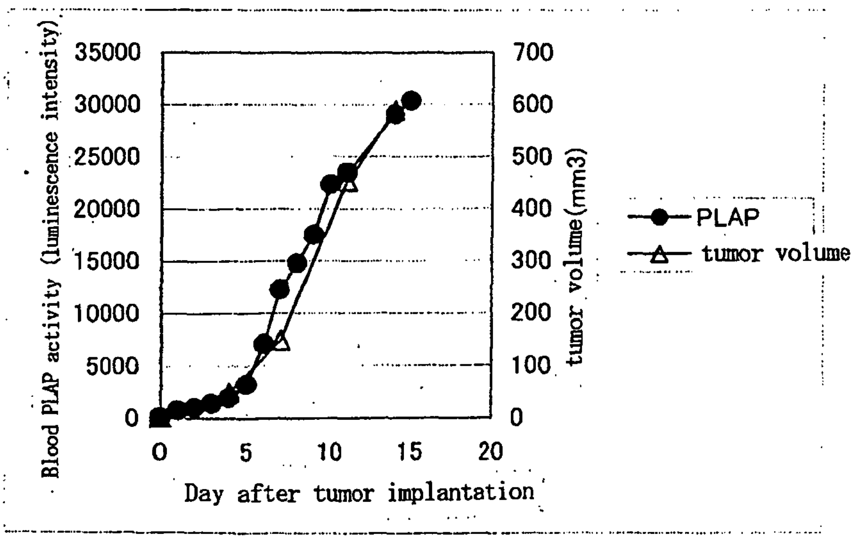
Blood PLAP activity (luminescence intensity)
35000
30000
25000
20000
15000
10000
5000
0
700
600
500
400
300
200
100
0
tumor volume(mm3)
0
5
10
15
20
Day after tumor implantation
PLAP
tumor volume

Fig. 22

**INTERNATIONAL SEARCH REPORT**

International application No. PCT/JP2005/005257

A. CLASSIFICATION OF SUBJECT MATTER
Int.Cl[7] C12N15/65, 15/55, A01K67/027, C12N5/10, C12Q1/42, G01N33/15, 33/50, 33/68

According to International Patent Classification (IPC) or to both national classification and IPC

B. FIELDS SEARCHED

Minimum documentation searched (classification system followed by classification symbols)
Int.Cl[7] C12N15/52-15/66, A01K67/027, C12N5/10, C12Q1/42, G01N33/15, 33/50, 33/68

Documentation searched other than minimum documentation to the extent that such documents are included in the fields searched

Electronic data base consulted during the international search (name of data base and, where practicable, search terms used)
JSTPlus, PUBMED

C. DOCUMENTS CONSIDERED TO BE RELEVANT

| Category* | Citation of document, with indication, where appropriate, of the relevant passages | Relevant to claim No. |
|---|---|---|
| X<br>A | JOOSTEN C.E. et al., Production of a sialylated N-linked glycoprotein in insect cells: role of glycosidases and effect of harvest time on glycosylation, Biotechnol Prog. (2003), Vol.19, No.1, pages 193 to 201 | 46-47<br>1-45,48-51 |
| X<br>A | BAO R. et al., Activation of cancer-specific gene expression by the survivin promoter, J Natl Cancer Inst. (2002), Vol.94, No.7, pages 522 to 528 | 46-47<br>1-45,48-51 |
| X<br>A | LEE J.C. et al., A reporter-based assay for identifying hepatitis C virus inhibitors based on subgenomic replicon cells, J Virol Methods., 01 March, 2004 (01.03.04), Vol.116, No.1, pages 27 to 33 | 46-47<br>1-45,48-51 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
21 June, 2005 (21.06.05)

Date of mailing of the international search report
12 July, 2005 (05.07.05)

Name and mailing address of the ISA/
Japanese Patent Office

Facsimile No.

Authorized officer

Telephone No.

Form PCT/ISA/210 (second sheet) (January 2004)

INTERNATIONAL SEARCH REPORT

International application No. PCT/JP2005/005257

C (Continuation). DOCUMENTS CONSIDERED TO BE RELEVANT

| Category* | Citation of document, with indication, where appropriate, of the relevant passages | Relevant to claim No. |
|---|---|---|
| A | YANG M. et al., Dual-color fluorescence imaging distinguishes tumor cells from induced host angiogenic vessels and stromal cells, Proc.Natl.Acad.Sci.USA. (2003), Vol.100, No.24, pages 14259 to 14262 | 1-51 |
| A | SHINJI S. et al., External whole-body image of EGFP gene expression, J.Nippon Med.Sch. (2003), Vol.70, No.6, pages 462 to 463 | 1-51 |
| A | BOUVET M. et al., Real-time optical imaging of primary tumor growth and multiple metastatic events in a pancreastic cancer orthotopic model, Cancer Res. (2002), Vol.62, No.5, pages 1534 to 1540 | 1-51 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02088107 A


### Non-patent literature cited in the description


- Molecular Cloning, A Laboratory Manual. Cold Spring Harbor Press, 2001
- **KUCHARCZUK.** *Development,* 1999, vol. 126 (9), 1957-1965
- **SHOEMAKER.** *The 7th International Symposium on Cancer Chemotherapy,* 2002
- **YAMAGATA.** *Journal of experimental & clinical cancer research,* 2000, vol. 19 (2), 211-217
- **YANG.** *Cancer Research,* 1999, vol. 59 (4), 781-786
- **HOPP et al.** *Bio/Technol.,* 1988, vol. 6, 1204-10
- Molecular Cloning, A Laboratory Manual. Cold Spring Harbor Press, 1989
- Current Protocols in Molecular Biology. John Wiley & Sons, 1987
- **KAUFMAN et al.** *Mol. Cell. Biol.,* 1989, vol. 9, 946
- **NIWA et al.** *Gene,* 1991, vol. 108, 193-200
- **SEED ; ARUFFO.** *Proc. Natl. Acad. Sci. USA,* 1987, vol. 84, 3365-9
- **MIZUSHIMA et al.** *Nucleic Acids Res.,* 1990, vol. 18, 5322
- **KIM et al.** *Gene,* 1990, vol. 91, 217-23
- **TAKEBE et al.** *Mol. Cell. Biol.,* 1988, vol. 8, 466
- **MULLIGAN et al.** *Nature,* 1979, vol. 277, 108
- Genetic Engineering. Academic Press, 1982, vol. 3, 83-141
- **GHEYSEN ; FIERS.** *J. Mol. Appl. Genet.,* 1982, vol. 1, 385-94
- **CULLEN.** *Methods Enzymol.,* 1987, vol. 152, 684-704
- **KIMURA.** *The Journal of Biological Chemistry,* 2001, vol. 276, 2292-2298
- **RICHARD MORIGGL.** *Molecular and Cellular Biology,* 1997, vol. 17, 3663-3678
- **GINSBERG.** *Genes & development,* 1994, vol. 8 (22), 2665-2679
- **FAWELL.** *Cell,* 1990, vol. 60 (6), 953-962
- **ORKIN.** *Cell,* 1990, vol. 63 (4), 665-72
- **WASYLYK.** *Molecular and Cellular Biology,* 1989, vol. 9 (5), 2247-2250
- **LEVINE.** *Nature,* 1991, vol. 351 (6326), 453-6
- **STEFFEN.** *Nucleic Acids Research,* 1992, vol. 20 (1), 3-26
- **MARTIN SEIDEL et al.** *Proc. Natl. Acad. Sci,* 1995, vol. 92, 3041-3045
- **CHIRWIN et al.** *Biochemistry,* 1979, vol. 18, 5294-9
- **CHOMCZYNSKI ; SACCHI.** *Anal. Biochem.,* 1987, vol. 162, 156-9
- **FROHMAN et al.** *Proc. Natl. Acad. Sci. USA,* 1988, vol. 85, 8998-9002
- **BELYAVSKY et al.** *Nucleic Acids Res.,* 1989, vol. 17, 2919-32
- **MARUYAMA ; SUGANO.** *Gene,* 1994, vol. 138, 171-4
- **SUZUKI.** *Gene,* 1997, vol. 200, 149-56
- **SANGER et al.** *Proc. Natl. Acad. Sci. USA,* 1977, vol. 74, 5463
- **CHU et al.** *Nucleic Acids Res.,* 1987, vol. 15, 1311-26
- **DERIJARD.** *Cell,* 1994, vol. 7, 1025-37
- **LAMB.** *Nature Genetics,* 1993, vol. 5, 22-30
- **RABINDRAN et al.** *Science,* 1993, vol. 259, 230-4
- **CHEN ; OKAYAMA.** *Mol. Cell. Biol.,* 1987, vol. 7, 2745-52
- **LOPATA et al.** *Nucleic Acids Res.,* 1984, vol. 12, 5707-17
- **SUSSMAN ; MILMAN.** *Mol. Cell. Biol.,* 1985, vol. 4, 1642-3
- **HOPP et al.** *Bio/Tehcnol.,* 1988, vol. 6, 1204-10
- **ASANO et al.** *Jpn J Cancer Res.,* January 1999, vol. 90 (1), 93-100
- **MARTIN SEIDEL et al.** *Proc. Natl. Acad. Sci. USA,* 1995, vol. 92, 3041-3045
- **FIRE.** *Nature,* 1998, vol. 391, 806-811
- **MARK et al.** *Proc. Natl. Acad. Sci. USA,* 1984, vol. 81, 5662-6
- **ZOLLER ; SMITH.** *Nucleic Acids Res.,* 1982, vol. 10, 6487-500
- **WANG et al.** *Science,* 1984, vol. 224, 1431-3

- **DALBADIE-MCFARLAND et al.** *Proc. Natl. Acad. Sci. USA,* 1982, vol. 79, 6409-13
- **HASHIMOTO-GOTO et al.** *Gene,* 1995, vol. 152, 271-5
- **KUNKEL.** *Proc. Natl. Acad. Sci. USA,* 1985, vol. 82, 488-92
- **KRAMER ; FRITZ.** *Method. Enzymol.,* 1987, vol. 154, 350-67
- **KUNKEL.** *Method. Enzymol.,* 1988, vol. 85, 2763-6
- **GOTO.** *Molecular Pharmacology,* 1996, vol. 49, 860-873
- **FORSYTHE.** *Molecular and Cellular Biology,* 1996, vol. 16 (9), 4604-4613
- **HARRIS.** *Nature Reviews Cancer,* 2002, vol. 2, 38-47
- **JIANG.** *Am. J. Physiol,* 1996, vol. 271, C1172-C1180
- **BLAGOSKLONNY.** *Cancer Cell,* 2004, vol. 5, 13-17
- **MIZUI.** *The Journal of antibiotics,* 2004, vol. 57, 188-196
- **JINZHAO HOU.** *Science,* 1994, vol. 265 (16), 1701-1706
- **RICHARD MORIGGL et al.** *Molecular and Cellular Biology,* 1997, vol. 17, 3663-3678
- **HELEN KOTANIDES et al.** *The Journal of Biological Chemistry,* 1996, vol. 271 (41), 25555-25561
- *MOLECULAR PHARMACOLOGY,* 1996, vol. 49, 860-873

SEQUENCE LISTING

<110> Eisai Co., Ltd.

<120> USING NONHUMAN ANIMAL MODEL, METHOD OF MEASURING TRANSCRIPTIONAL ACTIVITY, METHOD OF MEASURING CELL NUMBER AND METHOD OF MEASURING TUMOR VOLUME

<130> 0459WO-C4305

<150> JP 2004-84810
<151> 2004-03-23

<160> 44

<170> PatentIn version 3.1

<210> 1
<211> 24
<212> DNA
<213> Homo sapiens

<400> 1
catacgtggg ctccaacagg tcct 24

<210> 2
<211> 24
<212> DNA
<213> Homo sapiens

<400> 2
cctacgtgct gtctcacaca gcct 24

<210> 3
<211> 24
<212> DNA
<213> Homo sapiens

<400> 3
cgcacgtggc cccggacacg cagc 24

<210> 4
<211> 24
<212> DNA
<213> Homo sapiens

<400> 4
cacacgtggg ttcccgcacg tccg 24

<210> 5
<211> 8
<212> DNA
<213> Homo sapiens

<220>
<221> misc_feature
<222> (1)..(8)
<223> IL4RE

<400> 5
ttnnnnaa 8

<210> 6
<211> 9
<212> DNA
<213> Homo sapiens

<220>
<221> misc_feature
<222> (1)..(9)
<223> IL4RE

<400> 6
ttnnnnnaa 9

<210> 7
<211> 10
<212> DNA
<213> Homo sapiens

<220>
<221> misc_feature
<222> (1)..(10)
<223> IL4RE

<400> 7
ttnnnnnnaa 10

<210> 8
<211> 10
<212> DNA
<213> Homo sapiens

<400> 8
ttcccaagaa 10

<210> 9
<211> 197
<212> DNA
<213> Simian virus 40

<400> 9
tgcatctcaa ttagtcagca accatagtcc cgcccctaac tccgcccatc ccgcccctaa 60
ctccgcccag ttccgcccat tctccgcccc atggctgact aattttttt atttatgcag 120
aggccgaggc cgcctcggcc tctgagctat tccagaagta gtgaggaggc ttttttggag 180
gcctaggctt ttgcaaa 197

<210> 10
<211> 1521
<212> DNA
<213> Homo sapiens

<220>
<221> CDS
<222> (1)..(1518)
<223>

<400> 10
atg ctg ctg ctg ctg ctg ctg ctg ggc ctg agg cta cag ctc tcc ctg 48
Met Leu Leu Leu Leu Leu Leu Leu Gly Leu Arg Leu Gln Leu Ser Leu
1 5 10 15
ggc atc atc cca gtt gag gag gag aac ccg gac ttc tgg aac cgc gag 96
Gly Ile Ile Pro Val Glu Glu Glu Asn Pro Asp Phe Trp Asn Arg Glu
20 25 30
gca gcc gag gcc ctg ggt gcc gcc aag aag ctg cag cct gca cag aca 144
Ala Ala Glu Ala Leu Gly Ala Ala Lys Lys Leu Gln Pro Ala Gln Thr
35 40 45
gcc gcc aag aac ctc atc atc ttc ctg ggc gat ggg atg ggg gtg tct 192
Ala Ala Lys Asn Leu Ile Ile Phe Leu Gly Asp Gly Met Gly Val Ser
50 55 60
acg gtg aca gct gcc agg atc cta aaa ggg cag aag aag gac aaa ctg 240
Thr Val Thr Ala Ala Arg Ile Leu Lys Gly Gln Lys Lys Asp Lys Leu

```
65                  70                  75                  80
ggg cct gag ata ccc ctg gcc atg gac cgc ttc cca tat gtg gct ctg     288
Gly Pro Glu Ile Pro Leu Ala Met Asp Arg Phe Pro Tyr Val Ala Leu
                85                  90                  95
tcc aag aca tac aat gta gac aaa cat gtg cca gac agt gga gcc aca     336
Ser Lys Thr Tyr Asn Val Asp Lys His Val Pro Asp Ser Gly Ala Thr
            100                 105                 110
gcc acg gcc tac ctg tgc ggg gtc aag ggc aac ttc cag acc att ggc     384
Ala Thr Ala Tyr Leu Cys Gly Val Lys Gly Asn Phe Gln Thr Ile Gly
        115                 120                 125
ttg agt gca gcc gcc cgc ttt aac cag tgc aac acg aca cgc ggc aac     432
Leu Ser Ala Ala Ala Arg Phe Asn Gln Cys Asn Thr Thr Arg Gly Asn
    130                 135                 140
gag gtc atc tcc gtg atg aat cgg gcc aag aaa gca ggg aag tca gtg     480
Glu Val Ile Ser Val Met Asn Arg Ala Lys Lys Ala Gly Lys Ser Val
145                 150                 155                 160
gga gtg gta acc acc aca cga gtg cag cac gcc tcg cca gcc ggc acc     528
Gly Val Val Thr Thr Thr Arg Val Gln His Ala Ser Pro Ala Gly Thr
                165                 170                 175
tac gcc cac acg gtg aac cgc aac tgg tac tcg gac gcc gac gtg cct     576
Tyr Ala His Thr Val Asn Arg Asn Trp Tyr Ser Asp Ala Asp Val Pro
            180                 185                 190
gcc tcg gcc cgc cag gag ggg tgc cag gac atc gct acg cag ctc atc     624
Ala Ser Ala Arg Gln Glu Gly Cys Gln Asp Ile Ala Thr Gln Leu Ile
        195                 200                 205
tcc aac atg gac att gac gtg atc cta ggt gga ggc cga aag tac atg     672
Ser Asn Met Asp Ile Asp Val Ile Leu Gly Gly Gly Arg Lys Tyr Met
    210                 215                 220
ttt ccc atg gga acc cca gac cct gag tac cca gat gac tac agc caa     720
Phe Pro Met Gly Thr Pro Asp Pro Glu Tyr Pro Asp Asp Tyr Ser Gln
225                 230                 235                 240
ggt ggg acc agg ctg gac ggg aag aat ctg gtg cag gaa tgg ctg gcg     768
Gly Gly Thr Arg Leu Asp Gly Lys Asn Leu Val Gln Glu Trp Leu Ala
                245                 250                 255
aag cgc cag ggt gcc cgg tat gtg tgg aac cgc act gag ctc atg cag     816
Lys Arg Gln Gly Ala Arg Tyr Val Trp Asn Arg Thr Glu Leu Met Gln
            260                 265                 270
gct tcc ctg gac ccg tct gtg acc cat ctc atg ggt ctc ttt gag cct     864
Ala Ser Leu Asp Pro Ser Val Thr His Leu Met Gly Leu Phe Glu Pro
        275                 280                 285
gga gac atg aaa tac gag atc cac cga gac tcc aca ctg gac ccc tcc     912
Gly Asp Met Lys Tyr Glu Ile His Arg Asp Ser Thr Leu Asp Pro Ser
    290                 295                 300
ctg atg gag atg aca gag gct gcc ctg cgc ctg ctg agc agg aac ccc     960
Leu Met Glu Met Thr Glu Ala Ala Leu Arg Leu Leu Ser Arg Asn Pro
305                 310                 315                 320
cgc ggc ttc ttc ctc ttc gtg gag ggt ggt cgc atc gac cat ggt cat    1008
Arg Gly Phe Phe Leu Phe Val Glu Gly Gly Arg Ile Asp His Gly His
```

325 330 335
cat gaa agc agg gct tac cgg gca ctg act gag acg atc atg ttc gac 1056
His Glu Ser Arg Ala Tyr Arg Ala Leu Thr Glu Thr Ile Met Phe Asp
340 345 350
gac gcc att gag agg gcg ggc cag ctc acc agc gag gag gac acg ctg 1104
Asp Ala Ile Glu Arg Ala Gly Gln Leu Thr Ser Glu Glu Asp Thr Leu
355 360 365
agc ctc gtc act gcc gac cac tcc cac gtc ttc tcc ttc gga ggc tac 1152
Ser Leu Val Thr Ala Asp His Ser His Val Phe Ser Phe Gly Gly Tyr
370 375 380
ccc ctg cga ggg agc tcc atc ttc ggg ctg gcc cct ggc aag gcc cgg 1200
Pro Leu Arg Gly Ser Ser Ile Phe Gly Leu Ala Pro Gly Lys Ala Arg
385 390 395 400
gat cgt aag gcc tac aca gtg cta ctg tat ggc aat ggc cca ggg tat 1248
Asp Arg Lys Ala Tyr Thr Val Leu Leu Tyr Gly Asn Gly Pro Gly Tyr
405 410 415
gtc cta aag gat gga gct aga cca gat gtc aca gag tca gag tct gga 1296
Val Leu Lys Asp Gly Ala Arg Pro Asp Val Thr Glu Ser Glu Ser Gly
420 425 430
tct cca gag tac cgt cag caa tcg gcc gta ccg tta gat gaa gag act 1344
Ser Pro Glu Tyr Arg Gln Gln Ser Ala Val Pro Leu Asp Glu Glu Thr
435 440 445
cac gca ggg gaa gac gtc gct gtc ttt gca aga ggt ccc cag gca cat 1392
His Ala Gly Glu Asp Val Ala Val Phe Ala Arg Gly Pro Gln Ala His
450 455 460
ctc gtg cat ggc gta cag gaa cag act ttc atc gct cat gta atg gca 1440
Leu Val His Gly Val Gln Glu Gln Thr Phe Ile Ala His Val Met Ala
465 470 475 480
ttc gca gca tgt ttg gag cca tat acc gct tgc gat tta gct cca cca 1488
Phe Ala Ala Cys Leu Glu Pro Tyr Thr Ala Cys Asp Leu Ala Pro Pro
485 490 495
gca ggt acg aca gac gct gcc cat cca ggt taa 1521
Ala Gly Thr Thr Asp Ala Ala His Pro Gly
500 505

<210> 11
<211> 506
<212> PRT
<213> Homo sapiens

<400> 11
Met Leu Leu Leu Leu Leu Leu Leu Gly Leu Arg Leu Gln Leu Ser Leu
1 5 10 15
Gly Ile Ile Pro Val Glu Glu Glu Asn Pro Asp Phe Trp Asn Arg Glu
20 25 30
Ala Ala Glu Ala Leu Gly Ala Ala Lys Lys Leu Gln Pro Ala Gln Thr
35 40 45

Ala Ala Lys Asn Leu Ile Ile Phe Leu Gly Asp Gly Met Gly Val Ser
50 55 60
Thr Val Thr Ala Ala Arg Ile Leu Lys Gly Gln Lys Lys Asp Lys Leu
65 70 75 80
Gly Pro Glu Ile Pro Leu Ala Met Asp Arg Phe Pro Tyr Val Ala Leu
85 90 95
Ser Lys Thr Tyr Asn Val Asp Lys His Val Pro Asp Ser Gly Ala Thr
100 105 110
Ala Thr Ala Tyr Leu Cys Gly Val Lys Gly Asn Phe Gln Thr Ile Gly
115 120 125
Leu Ser Ala Ala Ala Arg Phe Asn Gln Cys Asn Thr Thr Arg Gly Asn
130 135 140
Glu Val Ile Ser Val Met Asn Arg Ala Lys Lys Ala Gly Lys Ser Val
145 150 155 160
Gly Val Val Thr Thr Thr Arg Val Gln His Ala Ser Pro Ala Gly Thr
165 170 175
Tyr Ala His Thr Val Asn Arg Asn Trp Tyr Ser Asp Ala Asp Val Pro
180 185 190
Ala Ser Ala Arg Gln Glu Gly Cys Gln Asp Ile Ala Thr Gln Leu Ile
195 200 205
Ser Asn Met Asp Ile Asp Val Ile Leu Gly Gly Gly Arg Lys Tyr Met
210 215 220
Phe Pro Met Gly Thr Pro Asp Pro Glu Tyr Pro Asp Asp Tyr Ser Gln
225 230 235 240
Gly Gly Thr Arg Leu Asp Gly Lys Asn Leu Val Gln Glu Trp Leu Ala
245 250 255
Lys Arg Gln Gly Ala Arg Tyr Val Trp Asn Arg Thr Glu Leu Met Gln
260 265 270
Ala Ser Leu Asp Pro Ser Val Thr His Leu Met Gly Leu Phe Glu Pro
275 280 285
Gly Asp Met Lys Tyr Glu Ile His Arg Asp Ser Thr Leu Asp Pro Ser
290 295 300
Leu Met Glu Met Thr Glu Ala Ala Leu Arg Leu Leu Ser Arg Asn Pro
305 310 315 320
Arg Gly Phe Phe Leu Phe Val Glu Gly Gly Arg Ile Asp His Gly His
325 330 335
His Glu Ser Arg Ala Tyr Arg Ala Leu Thr Glu Thr Ile Met Phe Asp
340 345 350
Asp Ala Ile Glu Arg Ala Gly Gln Leu Thr Ser Glu Glu Asp Thr Leu
355 360 365
Ser Leu Val Thr Ala Asp His Ser His Val Phe Ser Phe Gly Gly Tyr
370 375 380
Pro Leu Arg Gly Ser Ser Ile Phe Gly Leu Ala Pro Gly Lys Ala Arg
385 390 395 400
Asp Arg Lys Ala Tyr Thr Val Leu Leu Tyr Gly Asn Gly Pro Gly Tyr
405 410 415
Val Leu Lys Asp Gly Ala Arg Pro Asp Val Thr Glu Ser Glu Ser Gly
420 425 430

```
Ser Pro Glu Tyr Arg Gln Gln Ser Ala Val Pro Leu Asp Glu Glu Thr
        435                 440                 445
His Ala Gly Glu Asp Val Ala Val Phe Ala Arg Gly Pro Gln Ala His
    450                 455                 460
Leu Val His Gly Val Gln Glu Gln Thr Phe Ile Ala His Val Met Ala
465                 470                 475                 480
Phe Ala Ala Cys Leu Glu Pro Tyr Thr Ala Cys Asp Leu Ala Pro Pro
                485                 490                 495
Ala Gly Thr Thr Asp Ala Ala His Pro Gly
            500                 505


<210>  12
<211>  1521
<212>  DNA
<213>  Homo sapiens

<220>
<221>  CDS
<222>  (1)..(1518)
<223>

<400>  12
atg ctg ctg ctg ctg ctg ctg ctg ggc ctg agg cta cag ctc tcc ctg      48
Met Leu Leu Leu Leu Leu Leu Leu Gly Leu Arg Leu Gln Leu Ser Leu
1               5                   10                  15
ggc atc atc cca gtt gag gag gag aac ccg gac ttc tgg aac cgc gag      96
Gly Ile Ile Pro Val Glu Glu Glu Asn Pro Asp Phe Trp Asn Arg Glu
            20                  25                  30
gca gcc gag gcc ctg ggt gcc gcc aag aag ctg cag cct gca cag aca     144
Ala Ala Glu Ala Leu Gly Ala Ala Lys Lys Leu Gln Pro Ala Gln Thr
        35                  40                  45
gcc gcc aag aac ctc atc atc ttc ctg ggc gat ggg atg ggg gtg tct     192
Ala Ala Lys Asn Leu Ile Ile Phe Leu Gly Asp Gly Met Gly Val Ser
    50                  55                  60
acg gtg aca gct gcc agg atc cta aaa ggg cag aag aag gac aaa ctg     240
Thr Val Thr Ala Ala Arg Ile Leu Lys Gly Gln Lys Lys Asp Lys Leu
65                  70                  75                  80
ggg cct gag ata ccc ctg gcc atg gac cgc ttc cca tat gtg gct ctg     288
Gly Pro Glu Ile Pro Leu Ala Met Asp Arg Phe Pro Tyr Val Ala Leu
                85                  90                  95
tcc aag aca tac aat gta gac aaa cat gtg cca gac agt gga gcc aca     336
Ser Lys Thr Tyr Asn Val Asp Lys His Val Pro Asp Ser Gly Ala Thr
            100                 105                 110
gcc acg gcc tac ctg tgc ggg gtc aag ggc aac ttc cag acc att ggc     384
Ala Thr Ala Tyr Leu Cys Gly Val Lys Gly Asn Phe Gln Thr Ile Gly
        115                 120                 125
ttg agt gca gcc gcc cgc ttt aac cag tgc aac acg aca cgc ggc aac     432
```

Leu Ser Ala Ala Ala Arg Phe Asn Gln Cys Asn Thr Thr Arg Gly Asn
130 135 140
gag gtc atc tcc gtg atg aat cgg gcc aag aaa gca ggg aag tca gtg 480
Glu Val Ile Ser Val Met Asn Arg Ala Lys Lys Ala Gly Lys Ser Val
145 150 155 160
gga gtg gta acc acc aca cga gtg cag cac gcc tcg cca gcc ggc acc 528
Gly Val Val Thr Thr Thr Arg Val Gln His Ala Ser Pro Ala Gly Thr
165 170 175
tac gcc cac acg gtg aac cgc aac tgg tac tcg gac gcc gac gtg cct 576
Tyr Ala His Thr Val Asn Arg Asn Trp Tyr Ser Asp Ala Asp Val Pro
180 185 190
gcc tcg gcc cgc cag gag ggg tgc cag gac atc gct acg cag ctc atc 624
Ala Ser Ala Arg Gln Glu Gly Cys Gln Asp Ile Ala Thr Gln Leu Ile
195 200 205
tcc aac atg gac att gac gtg atc cta ggt gga ggc cga aag tac atg 672
Ser Asn Met Asp Ile Asp Val Ile Leu Gly Gly Gly Arg Lys Tyr Met
210 215 220
ttt cgc atg gga acc cca gac cct gag tac cca gat gac tac agc caa 720
Phe Arg Met Gly Thr Pro Asp Pro Glu Tyr Pro Asp Asp Tyr Ser Gln
225 230 235 240
ggt ggg acc agg ctg gac ggg aag aat ctg gtg cag gaa tgg ctg gcg 768
Gly Gly Thr Arg Leu Asp Gly Lys Asn Leu Val Gln Glu Trp Leu Ala
245 250 255
aag cgc cag ggt gcc cgg tat gtg tgg aac cgc act gag ctc atg cag 816
Lys Arg Gln Gly Ala Arg Tyr Val Trp Asn Arg Thr Glu Leu Met Gln
260 265 270
gct tcc ctg gac ccg tct gtg acc cat ctc atg ggt ctc ttt gag cct 864
Ala Ser Leu Asp Pro Ser Val Thr His Leu Met Gly Leu Phe Glu Pro
275 280 285
gga gac atg aaa tac gag atc cac cga gac tcc aca ctg gac ccc tcc 912
Gly Asp Met Lys Tyr Glu Ile His Arg Asp Ser Thr Leu Asp Pro Ser
290 295 300
ctg atg gag atg aca gag gct gcc ctg cgc ctg ctg agc agg aac ccc 960
Leu Met Glu Met Thr Glu Ala Ala Leu Arg Leu Leu Ser Arg Asn Pro
305 310 315 320
cgc ggc ttc ttc ctc ttc gtg gag ggt ggt cgc atc gac cat ggt cat 1008
Arg Gly Phe Phe Leu Phe Val Glu Gly Gly Arg Ile Asp His Gly His
325 330 335
cat gaa agc agg gct tac cgg gca ctg act gag acg atc atg ttc gac 1056
His Glu Ser Arg Ala Tyr Arg Ala Leu Thr Glu Thr Ile Met Phe Asp
340 345 350
gac gcc att gag agg gcg ggc cag ctc acc agc gag gag gac acg ctg 1104
Asp Ala Ile Glu Arg Ala Gly Gln Leu Thr Ser Glu Glu Asp Thr Leu
355 360 365
agc ctc gtc act gcc gac cac tcc cac gtc ttc tcc ttc gga ggc tac 1152
Ser Leu Val Thr Ala Asp His Ser His Val Phe Ser Phe Gly Gly Tyr
370 375 380
ccc ctg cga ggg agc tcc atc ttc ggg ctg gcc cct ggc aag gcc cgg 1200

Pro Leu Arg Gly Ser Ser Ile Phe Gly Leu Ala Pro Gly Lys Ala Arg
385 390 395 400
gac agg aag gcc tac acg gtc ctc cta tac gga aac ggt cca ggc tat 1248
Asp Arg Lys Ala Tyr Thr Val Leu Leu Tyr Gly Asn Gly Pro Gly Tyr
405 410 415
gtg ctc aag gac ggc gcc cgg ccg gat gtt acc gag agc gag agc ggg 1296
Val Leu Lys Asp Gly Ala Arg Pro Asp Val Thr Glu Ser Glu Ser Gly
420 425 430
agc ccc gag tat cgg cag cag tca gca gtg ccc ctg gac gaa gag acc 1344
Ser Pro Glu Tyr Arg Gln Gln Ser Ala Val Pro Leu Asp Glu Glu Thr
435 440 445
cac gca ggc gag gac gtg gcg gtg ttc gcg cgc ggc ccg cag gcg cac 1392
His Ala Gly Glu Asp Val Ala Val Phe Ala Arg Gly Pro Gln Ala His
450 455 460
ctg gtt cac ggc gtg cag gag cag acc ttc ata gcg cac gtc atg gcc 1440
Leu Val His Gly Val Gln Glu Gln Thr Phe Ile Ala His Val Met Ala
465 470 475 480
ttc gcc gcc tgc ctg gag ccc tac acc gcc tgc gac ctg gcg ccc ccc 1488
Phe Ala Ala Cys Leu Glu Pro Tyr Thr Ala Cys Asp Leu Ala Pro Pro
485 490 495
gcc ggc acc acc gac gcc gcg cac ccg ggt taa 1521
Ala Gly Thr Thr Asp Ala Ala His Pro Gly
500 505

<210> 13
<211> 506
<212> PRT
<213> Homo sapiens

<400> 13
Met Leu Leu Leu Leu Leu Leu Leu Gly Leu Arg Leu Gln Leu Ser Leu
1 5 10 15
Gly Ile Ile Pro Val Glu Glu Glu Asn Pro Asp Phe Trp Asn Arg Glu
20 25 30
Ala Ala Glu Ala Leu Gly Ala Ala Lys Lys Leu Gln Pro Ala Gln Thr
35 40 45
Ala Ala Lys Asn Leu Ile Ile Phe Leu Gly Asp Gly Met Gly Val Ser
50 55 60
Thr Val Thr Ala Ala Arg Ile Leu Lys Gly Gln Lys Lys Asp Lys Leu
65 70 75 80
Gly Pro Glu Ile Pro Leu Ala Met Asp Arg Phe Pro Tyr Val Ala Leu
85 90 95
Ser Lys Thr Tyr Asn Val Asp Lys His Val Pro Asp Ser Gly Ala Thr
100 105 110
Ala Thr Ala Tyr Leu Cys Gly Val Lys Gly Asn Phe Gln Thr Ile Gly
115 120 125
Leu Ser Ala Ala Ala Arg Phe Asn Gln Cys Asn Thr Thr Arg Gly Asn

130 135 140
Glu Val Ile Ser Val Met Asn Arg Ala Lys Lys Ala Gly Lys Ser Val
145 150 155 160
Gly Val Val Thr Thr Thr Arg Val Gln His Ala Ser Pro Ala Gly Thr
165 170 175
Tyr Ala His Thr Val Asn Arg Asn Trp Tyr Ser Asp Ala Asp Val Pro
180 185 190
Ala Ser Ala Arg Gln Glu Gly Cys Gln Asp Ile Ala Thr Gln Leu Ile
195 200 205
Ser Asn Met Asp Ile Asp Val Ile Leu Gly Gly Gly Arg Lys Tyr Met
210 215 220
Phe Arg Met Gly Thr Pro Asp Pro Glu Tyr Pro Asp Asp Tyr Ser Gln
225 230 235 240
Gly Gly Thr Arg Leu Asp Gly Lys Asn Leu Val Gln Glu Trp Leu Ala
245 250 255
Lys Arg Gln Gly Ala Arg Tyr Val Trp Asn Arg Thr Glu Leu Met Gln
260 265 270
Ala Ser Leu Asp Pro Ser Val Thr His Leu Met Gly Leu Phe Glu Pro
275 280 285
Gly Asp Met Lys Tyr Glu Ile His Arg Asp Ser Thr Leu Asp Pro Ser
290 295 300
Leu Met Glu Met Thr Glu Ala Ala Leu Arg Leu Leu Ser Arg Asn Pro
305 310 315 320
Arg Gly Phe Phe Leu Phe Val Glu Gly Gly Arg Ile Asp His Gly His
325 330 335
His Glu Ser Arg Ala Tyr Arg Ala Leu Thr Glu Thr Ile Met Phe Asp
340 345 350
Asp Ala Ile Glu Arg Ala Gly Gln Leu Thr Ser Glu Glu Asp Thr Leu
355 360 365
Ser Leu Val Thr Ala Asp His Ser His Val Phe Ser Phe Gly Gly Tyr
370 375 380
Pro Leu Arg Gly Ser Ser Ile Phe Gly Leu Ala Pro Gly Lys Ala Arg
385 390 395 400
Asp Arg Lys Ala Tyr Thr Val Leu Leu Tyr Gly Asn Gly Pro Gly Tyr
405 410 415
Val Leu Lys Asp Gly Ala Arg Pro Asp Val Thr Glu Ser Glu Ser Gly
420 425 430
Ser Pro Glu Tyr Arg Gln Gln Ser Ala Val Pro Leu Asp Glu Glu Thr
435 440 445
His Ala Gly Glu Asp Val Ala Val Phe Ala Arg Gly Pro Gln Ala His
450 455 460
Leu Val His Gly Val Gln Glu Gln Thr Phe Ile Ala His Val Met Ala
465 470 475 480
Phe Ala Ala Cys Leu Glu Pro Tyr Thr Ala Cys Asp Leu Ala Pro Pro
485 490 495
Ala Gly Thr Thr Asp Ala Ala His Pro Gly
500 505

<210> 14
<211> 1560
<212> DNA
<213> Homo sapiens

<220>
<221> CDS
<222> (1)..(1557)
<223>

<400> 14
atg ctg ctg ctg ctg ctg ctg ctg ggc ctg agg cta cag ctc tcc ctg 48
Met Leu Leu Leu Leu Leu Leu Leu Gly Leu Arg Leu Gln Leu Ser Leu
1 5 10 15
ggc atc atc cca gtt gag gag gag aac ccg gac ttc tgg aac cgc gag 96
Gly Ile Ile Pro Val Glu Glu Glu Asn Pro Asp Phe Trp Asn Arg Glu
20 25 30
gca gcc gag gcc ctg ggt gcc gcc aag aag ctg cag cct gca cag aca 144
Ala Ala Glu Ala Leu Gly Ala Ala Lys Lys Leu Gln Pro Ala Gln Thr
35 40 45
gcc gcc aag aac ctc atc atc ttc ctg ggc gat ggg atg ggg gtg tct 192
Ala Ala Lys Asn Leu Ile Ile Phe Leu Gly Asp Gly Met Gly Val Ser
50 55 60
acg gtg aca gct gcc agg atc cta aaa ggg cag aag aag gac aaa ctg 240
Thr Val Thr Ala Ala Arg Ile Leu Lys Gly Gln Lys Lys Asp Lys Leu
65 70 75 80
ggg cct gag ata ccc ctg gcc atg gac cgc ttc cca tat gtg gct ctg 288
Gly Pro Glu Ile Pro Leu Ala Met Asp Arg Phe Pro Tyr Val Ala Leu
85 90 95
tcc aag aca tac aat gta gac aaa cat gtg cca gac agt gga gcc aca 336
Ser Lys Thr Tyr Asn Val Asp Lys His Val Pro Asp Ser Gly Ala Thr
100 105 110
gcc acg gcc tac ctg tgc ggg gtc aag ggc aac ttc cag acc att ggc 384
Ala Thr Ala Tyr Leu Cys Gly Val Lys Gly Asn Phe Gln Thr Ile Gly
115 120 125
ttg agt gca gcc gcc cgc ttt aac cag tgc aac acg aca cgc ggc aac 432
Leu Ser Ala Ala Ala Arg Phe Asn Gln Cys Asn Thr Thr Arg Gly Asn
130 135 140
gag gtc atc tcc gtg atg aat cgg gcc aag aaa gca ggg aag tca gtg 480
Glu Val Ile Ser Val Met Asn Arg Ala Lys Lys Ala Gly Lys Ser Val
145 150 155 160
gga gtg gta acc acc aca cga gtg cag cac gcc tcg cca gcc ggc acc 528
Gly Val Val Thr Thr Thr Arg Val Gln His Ala Ser Pro Ala Gly Thr
165 170 175
tac gcc cac acg gtg aac cgc aac tgg tac tcg gac gcc gac gtg cct 576
Tyr Ala His Thr Val Asn Arg Asn Trp Tyr Ser Asp Ala Asp Val Pro
180 185 190
gcc tcg gcc cgc cag gag ggg tgc cag gac atc gct acg cag ctc atc 624

```
Ala Ser Ala Arg Gln Glu Gly Cys Gln Asp Ile Ala Thr Gln Leu Ile
        195                 200                 205
tcc aac atg gac att gac gtg atc cta ggt gga ggc cga aag tac atg      672
Ser Asn Met Asp Ile Asp Val Ile Leu Gly Gly Gly Arg Lys Tyr Met
    210                 215                 220
ttt cgc atg gga acc cca gac cct gag tac cca gat gac tac agc caa      720
Phe Arg Met Gly Thr Pro Asp Pro Glu Tyr Pro Asp Asp Tyr Ser Gln
225                 230                 235                 240
ggt ggg acc agg ctg gac ggg aag aat ctg gtg cag gaa tgg ctg gcg      768
Gly Gly Thr Arg Leu Asp Gly Lys Asn Leu Val Gln Glu Trp Leu Ala
                245                 250                 255
aag cgc cag ggt gcc cgg tat gtg tgg aac cgc act gag ctc atg cag      816
Lys Arg Gln Gly Ala Arg Tyr Val Trp Asn Arg Thr Glu Leu Met Gln
            260                 265                 270
gct tcc ctg gac ccg tct gtg acc cat ctc atg ggt ctc ttt gag cct      864
Ala Ser Leu Asp Pro Ser Val Thr His Leu Met Gly Leu Phe Glu Pro
        275                 280                 285
gga gac atg aaa tac gag atc cac cga gac tcc aca ctg gac ccc tcc      912
Gly Asp Met Lys Tyr Glu Ile His Arg Asp Ser Thr Leu Asp Pro Ser
    290                 295                 300
ctg atg gag atg aca gag gct gcc ctg cgc ctg ctg agc agg aac ccc      960
Leu Met Glu Met Thr Glu Ala Ala Leu Arg Leu Leu Ser Arg Asn Pro
305                 310                 315                 320
cgc ggc ttc ttc ctc ttc gtg gag ggt ggt cgc atc gac cat ggt cat     1008
Arg Gly Phe Phe Leu Phe Val Glu Gly Gly Arg Ile Asp His Gly His
                325                 330                 335
cat gaa agc agg gct tac cgg gca ctg act gag acg atc atg ttc gac     1056
His Glu Ser Arg Ala Tyr Arg Ala Leu Thr Glu Thr Ile Met Phe Asp
            340                 345                 350
gac gcc att gag agg gcg ggc cag ctc acc agc gag gag gac acg ctg     1104
Asp Ala Ile Glu Arg Ala Gly Gln Leu Thr Ser Glu Glu Asp Thr Leu
        355                 360                 365
agc ctc gtc act gcc gac cac tcc cac gtc ttc tcc ttc gga ggc tac     1152
Ser Leu Val Thr Ala Asp His Ser His Val Phe Ser Phe Gly Gly Tyr
    370                 375                 380
ccc ctg cga ggg agc tcc atc ttc ggg ctg gcc cct ggc aag gcc cgg     1200
Pro Leu Arg Gly Ser Ser Ile Phe Gly Leu Ala Pro Gly Lys Ala Arg
385                 390                 395                 400
gac agg aag gcc tac acg gtc ctc cta tac gga aac ggt cca ggc tat     1248
Asp Arg Lys Ala Tyr Thr Val Leu Leu Tyr Gly Asn Gly Pro Gly Tyr
                405                 410                 415
gtg ctc aag gac ggc gcc cgg ccg gat gtt acc gag agc gag agc ggg     1296
Val Leu Lys Asp Gly Ala Arg Pro Asp Val Thr Glu Ser Glu Ser Gly
            420                 425                 430
agc ccc gag tat cgg cag cag tca gca gtg ccc ctg gac gaa gag acc     1344
Ser Pro Glu Tyr Arg Gln Gln Ser Ala Val Pro Leu Asp Glu Glu Thr
        435                 440                 445
cac gca ggc gag gac gtg gcg gtg ttc gcg cgc ggc ccg cag gcg cac     1392
```

His Ala Gly Glu Asp Val Ala Val Phe Ala Arg Gly Pro Gln Ala His
450 455 460
ctg gtt cac ggc gtg cag gag cag acc ttc ata gcg cac gtc atg gcc 1440
Leu Val His Gly Val Gln Glu Gln Thr Phe Ile Ala His Val Met Ala
465 470 475 480
ttc gcc gcc tgc ctg gag ccc tac acc gcc tgc gac ctg gcg ccc ccc 1488
Phe Ala Ala Cys Leu Glu Pro Tyr Thr Ala Cys Asp Leu Ala Pro Pro
485 490 495
gcc ggc acc acc gac gcc gcg cac ccg ggt tac tct aga gtc ggg gcg 1536
Ala Gly Thr Thr Asp Ala Ala His Pro Gly Tyr Ser Arg Val Gly Ala
500 505 510
gcc ggc cgc ttc gag cag aca tga 1560
Ala Gly Arg Phe Glu Gln Thr
515

<210> 15
<211> 519
<212> PRT
<213> Homo sapiens

<400> 15
Met Leu Leu Leu Leu Leu Leu Leu Gly Leu Arg Leu Gln Leu Ser Leu
1 5 10 15
Gly Ile Ile Pro Val Glu Glu Glu Asn Pro Asp Phe Trp Asn Arg Glu
20 25 30
Ala Ala Glu Ala Leu Gly Ala Ala Lys Lys Leu Gln Pro Ala Gln Thr
35 40 45
Ala Ala Lys Asn Leu Ile Ile Phe Leu Gly Asp Gly Met Gly Val Ser
50 55 60
Thr Val Thr Ala Ala Arg Ile Leu Lys Gly Gln Lys Lys Asp Lys Leu
65 70 75 80
Gly Pro Glu Ile Pro Leu Ala Met Asp Arg Phe Pro Tyr Val Ala Leu
85 90 95
Ser Lys Thr Tyr Asn Val Asp Lys His Val Pro Asp Ser Gly Ala Thr
100 105 110
Ala Thr Ala Tyr Leu Cys Gly Val Lys Gly Asn Phe Gln Thr Ile Gly
115 120 125
Leu Ser Ala Ala Ala Arg Phe Asn Gln Cys Asn Thr Thr Arg Gly Asn
130 135 140
Glu Val Ile Ser Val Met Asn Arg Ala Lys Lys Ala Gly Lys Ser Val
145 150 155 160
Gly Val Val Thr Thr Thr Arg Val Gln His Ala Ser Pro Ala Gly Thr
165 170 175
Tyr Ala His Thr Val Asn Arg Asn Trp Tyr Ser Asp Ala Asp Val Pro
180 185 190
Ala Ser Ala Arg Gln Glu Gly Cys Gln Asp Ile Ala Thr Gln Leu Ile
195 200 205

Ser Asn Met Asp Ile Asp Val Ile Leu Gly Gly Gly Arg Lys Tyr Met
210 215 220
Phe Arg Met Gly Thr Pro Asp Pro Glu Tyr Pro Asp Asp Tyr Ser Gln
225 230 235 240
Gly Gly Thr Arg Leu Asp Gly Lys Asn Leu Val Gln Glu Trp Leu Ala
245 250 255
Lys Arg Gln Gly Ala Arg Tyr Val Trp Asn Arg Thr Glu Leu Met Gln
260 265 270
Ala Ser Leu Asp Pro Ser Val Thr His Leu Met Gly Leu Phe Glu Pro
275 280 285
Gly Asp Met Lys Tyr Glu Ile His Arg Asp Ser Thr Leu Asp Pro Ser
290 295 300
Leu Met Glu Met Thr Glu Ala Ala Leu Arg Leu Leu Ser Arg Asn Pro
305 310 315 320
Arg Gly Phe Phe Leu Phe Val Glu Gly Gly Arg Ile Asp His Gly His
325 330 335
His Glu Ser Arg Ala Tyr Arg Ala Leu Thr Glu Thr Ile Met Phe Asp
340 345 350
Asp Ala Ile Glu Arg Ala Gly Gln Leu Thr Ser Glu Glu Asp Thr Leu
355 360 365
Ser Leu Val Thr Ala Asp His Ser His Val Phe Ser Phe Gly Gly Tyr
370 375 380
Pro Leu Arg Gly Ser Ser Ile Phe Gly Leu Ala Pro Gly Lys Ala Arg
385 390 395 400
Asp Arg Lys Ala Tyr Thr Val Leu Leu Tyr Gly Asn Gly Pro Gly Tyr
405 410 415
Val Leu Lys Asp Gly Ala Arg Pro Asp Val Thr Glu Ser Glu Ser Gly
420 425 430
Ser Pro Glu Tyr Arg Gln Gln Ser Ala Val Pro Leu Asp Glu Glu Thr
435 440 445
His Ala Gly Glu Asp Val Ala Val Phe Ala Arg Gly Pro Gln Ala His
450 455 460
Leu Val His Gly Val Gln Glu Gln Thr Phe Ile Ala His Val Met Ala
465 470 475 480
Phe Ala Ala Cys Leu Glu Pro Tyr Thr Ala Cys Asp Leu Ala Pro Pro
485 490 495
Ala Gly Thr Thr Asp Ala Ala His Pro Gly Tyr Ser Arg Val Gly Ala
500 505 510
Ala Gly Arg Phe Glu Gln Thr
515

<210> 16
<211> 26
<212> DNA
<213> Artificial Sequence

<220>

<223> primer

<400> 16
ccagaattcc tgcctcgcca ctgtcc 26

<210> 17
<211> 22
<212> DNA
<213> Artificial Sequence

<220>
<223> primer

<400> 17
ttaggatcct ggcagctgtc ac 22

<210> 18
<211> 22
<212> DNA
<213> Artificial Sequence

<220>
<223> primer

<400> 18
gtgacagctg ccaggatcct aa 22

<210> 19
<211> 22
<212> DNA
<213> Artificial Sequence

<220>
<223> primer

<400> 19
aggaccgtgt aggcctccct gt 22

<210> 20
<211> 98
<212> DNA
<213> Artificial Sequence

<220>
<223> oligo DNA

<400> 20
aagcccggga tcgtaaggcc tacacagtgc tactgtatgg caatggccca gggtatgtcc 60
taaaggatgg agctagacca gatgtcacag agtcagag 98

<210> 21
<211> 98
<212> DNA
<213> Artificial Sequence

<220>
<223> oligo DNA

<400> 21
aaagacagcg acgtcttccc ctgcgtgagt ctcttcatct aacggtacgg ccgattgctg 60
acggtactct ggagatccag actctgactc tgtgacat 98

<210> 22
<211> 98
<212> DNA
<213> Artificial Sequence

<220>
<223> oligo DNA

<400> 22
ggaagacgtc gctgtctttg caagaggtcc ccaggcacat ctcgtgcatg gcgtacagga 60
acagactttc atcgctcatg taatggcatt cgcagcat 98

<210> 23
<211> 100
<212> DNA
<213> Artificial Sequence

<220>
<223> oligo DNA

<400> 23
tccagatctg ggttaacctg gatgggcagc gtctgtcgta cctgctggtg gagctaaatc 60
gcaagcggta tatggctcca aacatgctgc gaatgccatt 100

<210> 24
<211> 17
<212> DNA

<213> Artificial Sequence

<220>
<223> oligo DNA

<400> 24
aattcaagct taccatg 17

<210> 25
<211> 9
<212> DNA
<213> Artificial Sequence

<220>
<223> oligo DNA

<400> 25
gtaagcttg 9

<210> 26
<211> 33
<212> DNA
<213> Artificial Sequence

<220>
<223> oligo DNA

<400> 26
cgagctctta cgcgtgctag cccgggctcg aga 33

<210> 27
<211> 41
<212> DNA
<213> Artificial Sequence

<220>
<223> oligo DNA

<400> 27
agcttctcga gcccgggcta gcacgcgtaa gagctcggta c 41

<210> 28
<211> 39
<212> DNA

<213> Artificial Sequence

<220>
<223> oligo DNA

<400> 28
cacagtgcat acgtgggctc caacaggtcc tcttcgtac 39

<210> 29
<211> 39
<212> DNA
<213> Artificial Sequence

<220>
<223> oligo DNA

<400> 29
gaagaggacc tgttggagcc cacgtatgca ctgtggtac 39

<210> 30
<211> 27
<212> DNA
<213> Artificial Sequence

<220>
<223> primer

<400> 30
ggcggtacgc gtgtacggtg ggaggtc 27

<210> 31
<211> 22
<212> DNA
<213> Artificial Sequence

<220>
<223> primer

<400> 31
taccaagctt aagtttaaac gc 22

<210> 32
<211> 26
<212> DNA

<213> Artificial Sequence

<220>
<223> primer

<400> 32
acagaattcg aacgctgacg tcatca 26

<210> 33
<211> 43
<212> DNA
<213> Artificial Sequence

<220>
<223> primer

<400> 33
gaaagcttgg tagatctgtg gtctcataca gaacttataa gat 43

<210> 34
<211> 22
<212> DNA
<213> Artificial Sequence

<220>
<223> oligo DNA

<400> 34
ctagaggtac cagctgctag cg 22

<210> 35
<211> 22
<212> DNA
<213> Artificial Sequence

<220>
<223> oligo DNA

<400> 35
aattcgctag cagctggtac ct 22

<210> 36
<211> 19
<212> RNA

<213> Homo sapiens

<400> 36
gauaaguucu gaacgucga 19

<210> 37
<211> 64
<212> DNA
<213> Artificial Sequence

<220>
<223> oligo DNA

<400> 37
gatccccgat aagttctgaa cgtcgattca agagatcgac gttcagaact tatctttttg 60
gaaa 64

<210> 38
<211> 64
<212> DNA
<213> Artificial Sequence

<220>
<223> oligo DNA

<400> 38
agcttttcca aaaagataag ttctgaacgt cgatctcttg aatcgacgtt cagaacttat 60
cggg 64

<210> 39
<211> 35
<212> DNA
<213> Artificial Sequence

<220>
<223> oligo DNA

<400> 39
ctagtcaggt cacaggtcac aggtcacagt tcaat 35

<210> 40
<211> 35
<212> DNA
<213> Artificial Sequence

<220>
<223> oligo DNA

<400> 40
ctagattgaa ctgtgacctg tgacctgtga cctga 35

<210> 41
<211> 78
<212> DNA
<213> Artificial Sequence

<220>
<223> oligo DNA

<400> 41
agcggtacct cgacttccca agaacagaat cgacttccca agaacagaat cgacttccca 60
agaacagaat ctagagct 78

<210> 42
<211> 78
<212> DNA
<213> Artificial Sequence

<220>
<223> oligo DNA

<400> 42
agctctagat tctgttcttg ggaagtcgat tctgttcttg ggaagtcgat tctgttcttg 60
ggaagtcgag gtaccgct 78

<210> 43
<211> 33
<212> DNA
<213> Artificial Sequence

<220>
<223> primer

<400> 43
cggaattcat gtctctgtgg ggtctggtct cca 33

<210> 44
<211> 33

<212> DNA
<213> Artificial Sequence

<220>
<223> primer

<400> 44
gctctagatc accaactggg gttggcccttt agg 33